# EUROPEAN PATENT SPECIFICATION

(11) **EP 1 844 064 B1**
(45) Date of publication and mention of the grant of the patent: **30.01.2013**
(21) Application number: 06734133.9
(22) Date of filing: 31.01.2006
(51) Int. Cl.: C07K 14/325, C12N 15/00

(54) **AXMI-018, AXMI-020, AND AXMI-021, A FAMILY OF DELTA-ENDOTOXIN GENES AND METHODS FOR THEIR USE**
AXMI-018, AXMI-020 UND AXMI-021, DELTA-ENDOTOXIN-GENFAMILIE UND VERWENDUNGSVERFAHREN DAFÜR
AXMI-018, AXMI-020, ET AXMI-021, FAMILLE DE GENES DE DELTA-ENDOTOXINE ET LEURS METHODES D'UTILISATION

(30) Priority: 31.01.2005 US 648578 P
(43) Date of publication of application: 17.10.2007
(73) Proprietor: Athenix Corporation, Research Triangle Park, NC 27709 (US)
(72) Inventor: CAROZZI, Nadine, Raleigh, North Carolina 27615 (US); HARGISS, Tracy, Kernersville, North Carolina 27284 (US); KOZIEL, Michael, G., Raleigh, North Carolina 27613 (US); DUCK, Nicholas, B., Apex, North Carolina 27502 (US)
(74) Representative: Power, David
(86) International application number: PCT/US2006/003434
(87) International publication number: WO 2006/083891

(56) References cited:
- US-A- 5 589 382
- DATABASE EMBL [Online] 30 July 2002 (2002-07-30), "Bacillus thuringiensis serovar roskildiensis gene for Cry21Ba1, complete cds." XP002396569 retrieved from EBI accession no. EM_PRO:AB088406 Database accession no. AB088406 cited in the application
- DE MAAGD R A ET AL: "How Bacillus thuringiensis has evolved specific toxins to colonize the insect world" TRENDS IN GENETICS, ELSEVIER, AMSTERDAM, NL, vol. 17, no. 4, 1 April 2001 (2001-04-01), pages 193-199, XP004231927 ISSN: 0168-9525

### SEQUENCE LISTING

<110> Nadine Carozzi
   Tracy Hargiss
   Michael G. Koziel
   Nicholas B. Duck
<120> AXMI-018, AXMI-020, and AXMI-021, A
   Family of Delta-Endotoxin Genes and Methods for Their Use
<130> 45600/307109
<150> 60/648,578 <151> 2005-1-31
<160> 15
<170> FastSEQ for Windows Version 4.0
<210> 1
   <211> 3675
   <212> DNA
   <213> Bacillus thuringiensis
<220>
   <221> CDS
   <222> (1)...(3675)
<400> 1
   atg aca caa aat cat tca ttc tct gat aat aca tcc tca tcg acg ggt    48
   Met Thr Gln Asn His Ser Phe Ser Asp Asn Thr Ser Ser Ser Thr Gly
    1               5                  10                  15
   gta tct act tta gaa tca tct tta att cct tac aat gtg tac gcg aca    96
   Val Ser Thr Leu Glu Ser Ser Leu Ile Pro Tyr Asn Val Tyr Ala Thr
               20                  25                  30
   gat cag ttt aac tct aat aaa aat tgg gaa gat gca ctg aaa aaa tta    144
   Asp Gln Phe Asn Ser Asn Lys Asn Trp Glu Asp Ala Leu Lys Lys Leu
           35                  40                  45
   tta gaa aaa ttt tat tcc ggt gat tta aca cag gat gct att gat att    192
   Leu Glu Lys Phe Tyr Ser Gly Asp Leu Thr Gln Asp Ala Ile Asp Ile
       50                  55                  60
   ttt ctt ggt gac agc ggc ttt gat tac tta tct tta gta aat gtt att    240
   Phe Leu Gly Asp Ser Gly Phe Asp Tyr Leu Ser Leu Val Asn Val Ile
   65                  70                  75                  80
   ttt tct att gca gga tct ttt att cct tat gtg ggt gct ctt gtc cct    288
   Phe Ser Ile Ala Gly Ser Phe Ile Pro Tyr Val Gly Ala Leu Val Pro
                   85                  90                  95
   atc att aat ctt ctt ttt gga tca gag agc aaa cca gat gta ttt gaa    336
   Ile Ile Asn Leu Leu Phe Gly Ser Glu Ser Lys Pro Asp Val Phe Glu
               100                 105                 110
   caa atg aga gca cga att gaa gca tta att cat aag gaa tta tct gca    384
   Gln Met Arg Ala Arg Ile Glu Ala Leu Ile His Lys Glu Leu Ser Ala
             115                 120                 125
   gac cat gtg caa aca tta aaa gca gaa att aag gga ctt aaa gat acg      432
   Asp His Val Gln Thr Leu Lys Ala Glu Ile Lys Gly Leu Lys Asp Thr
       130                 135                 140
   gga gat cta tat caa aaa gat gta aat gct gtt gca gga aga aca aat      480
   Gly Asp Leu Tyr Gln Lys Asp Val Asn Ala Val Ala Gly Arg Thr Asn
   145                 150                 155                 160
   gga cct acc cct cca tca ttt gat agc aat aca gat gct tta aaa gca      528
   Gly Pro Thr Pro Pro Ser Phe Asp Ser Asn Thr Asp Ala Leu Lys Ala
                   165                 170                 175
   gaa ctt cga agt caa atc aca gct aca aac act cta ttt gtg caa cga      576
   Glu Leu Arg Ser Gln Ile Thr Ala Thr Asn Thr Leu Phe Val Gln Arg
               180                 185                 190
   atg cct caa ttt gct ata gag gga tat gaa gag att act cta cct tta      624
   Met Pro Gln Phe Ala Ile Glu Gly Tyr Glu Glu Ile Thr Leu Pro Leu
           195                 200                 205
   cac act atc gct gca agt atg cat ctt ata ttc tta aaa gat gtt tgt      672
   His Thr Ile Ala Ala Ser Met His Leu Ile Phe Leu Lys Asp Val Cys
       210                 215                 220
   gaa cat ggt gct gaa tgg gga att gct aat act aca tta aca aat tat      720
   Glu His Gly Ala Glu Trp Gly Ile Ala Asn Thr Thr Leu Thr Asn Tyr
   225                 230                 235                 240
   caa ggt caa tta caa gat tgt att aga gag tat tca aat aaa gct tat      768
   Gln Gly Gln Leu Gln Asp Cys Ile Arg Glu Tyr Ser Asn Lys Ala Tyr
                   245                 250                 255
   tcg atg ttc aat att ggt tta cag agg gca aaa aat aat gga aac aat      816
   Ser Met Phe Asn Ile Gly Leu Gln Arg Ala Lys Asn Asn Gly Asn Asn
               260                 265                 270
   atg tgg aat aac gta aat aac tat atc cgc aca atg aaa tta aat gct      864
   Met Trp Asn Asn Val Asn Asn Tyr Ile Arg Thr Met Lys Leu Asn Ala
           275                 280                 285
   tta gat act gtt gct caa tgg cct att ctg gat aaa gta aca tac cca      912
   Leu Asp Thr Val Ala Gln Trp Pro Ile Leu Asp Lys Val Thr Tyr Pro
       290                 295                 300
   tta gat aca aca tta caa caa aca cgc ggt ata ttt tca gat cta tca      960
   Leu Asp Thr Thr Leu Gln Gln Thr Arg Gly Ile Phe Ser Asp Leu Ser
   305                 310                 315                 320
   ggt agg ggg ggg aca caa tct aat tat aga tat gat tat gat gct gtt
   1008
   Gly Arg Gly Gly Thr Gln Ser Asn Tyr Arg Tyr Asp Tyr Asp Ala Val
                   325                 330                 335
   caa ggt tat gct cct cct ttt gtc gga ttt gat acc aaa cta aat gtt
   1056
   Gln Gly Tyr Ala Pro Pro Phe Val Gly Phe Asp Thr Lys Leu Asn Val
               340                 345                 350
   gta aac gat ttt ggt tat aaa gat tta acc gca att cag aca ttt aca
   1104
   Val Asn Asp Phe Gly Tyr Lys Asp Leu Thr Ala Ile Gln Thr Phe Thr
   355 360 365
   ggt gat cga att gat tca att tgg caa tca ttt aag tat aat tca gga
   1152
   Gly Asp Arg Ile Asp Ser Ile Trp Gln Ser Phe Lys Tyr Asn Ser Gly
   370 375 380
   gag cct ttt ctc acg aac tta ggg aat ggt aaa ccc gga aac aac ccc
   1200
   Glu Pro Phe Leu Thr Asn Leu Gly Asn Gly Lys Pro Gly Asn Asn Pro
   385 390 395 400
   gtg att cca aat agc aga gat aat ccg att att tcc gca aaa gga tct
   1248
   Val Ile Pro Asn Ser Arg Asp Asn Pro Ile Ile Ser Ala Lys Gly Ser
   405 410 415
   aga cca tct gca aac tat gtt ggg atg aat ttc caa cga gca aat aaa
   1296
   Arg Pro Ser Ala Asn Tyr Val Gly Met Asn Phe Gln Arg Ala Asn Lys
   420 425 430
   act gta gtt tca aat gga tat gta att cct aat gac aat tat aca gta
   1344
   Thr Val Val Ser Asn Gly Tyr Val Ile Pro Asn Asp Asn Tyr Thr Val
   435 440 445
   ccc gct ggg cat aaa ctt gga tgg att tca gcc ctg cat gat gaa ttg
   1392
   Pro Ala Gly His Lys Leu Gly Trp Ile Ser Ala Leu His Asp Glu Leu
   450 455 460
   gat aat gca aat aat gcg gat cta gtt gta tcg gtt tgg gtg aaa aat
   1440
   Asp Asn Ala Asn Asn Ala Asp Leu Val Val Ser Val Trp Val Lys Asn
   465 470 475 480
   gat atc ttc cag gaa aat att atc ggt tcc ata aaa aca gtt act act
   1488
   Asp Ile Phe Gln Glu Asn Ile Ile Gly Ser Ile Lys Thr Val Thr Thr
   485 490 495
   gat gat gga acc aca gaa aat aga caa caa att ata ggg atc ccg gca
   1536
   Asp Asp Gly Thr Thr Glu Asn Arg Gln Gln Ile Ile Gly Ile Pro Ala
   500 505 510
   gat aaa cat atg aca aga agt aca aag cga atg gaa ctg gaa ttt atc
   1584
   Asp Lys His Met Thr Arg Ser Thr Lys Arg Met Glu Leu Glu Phe Ile
   515 520 525
   aat ggt aca aat ggg tca atg agc tta tct agt act aat gat caa ttg
   1632
   Asn Gly Thr Asn Gly Ser Met Ser Leu Ser Ser Thr Asn Asp Gln Leu
   530 535 540
   tat tat acg att aat cct ata gtt agc cag aga tat caa att cgg tat
   1680
   Tyr Tyr Thr Ile Asn Pro Ile Val Ser Gln Arg Tyr Gln Ile Arg Tyr
   545                 550                 555                 560
   cgc gta gca aca act tca gca gaa tct tta gac cta tgg atc gat ggt
   1728
   Arg Val Ala Thr Thr Ser Ala Glu Ser Leu Asp Leu Trp Ile Asp Gly
                   565                 570                 575
   tat aaa cgc gga aca acc ccg tta cca aat aca agt agc aca tca acg
   1776
   Tyr Lys Arg Gly Thr Thr Pro Leu Pro Asn Thr Ser Ser Thr Ser Thr
               580                 585                 590
   caa aca caa aaa gtg ata att caa ggg tta caa gga aaa tat caa tta
   1824
   Gln Thr Gln Lys Val Ile Ile Gln Gly Leu Gln Gly Lys Tyr Gln Leu
           595                 600                 605
   att aat gga cca act ctt gat ttg aca gca ggt tcc cat act ttt ggt
   1872
   Ile Asn Gly Pro Thr Leu Asp Leu Thr Ala Gly Ser His Thr Phe Gly
       610                 615                 620
   att atg tta aca gca aat gct tct caa aat gta ttt att gat cgc att
   1920
   Ile Met Leu Thr Ala Asn Ala Ser Gln Asn Val Phe Ile Asp Arg Ile
   625                 630                 635                 640
   gaa ttt gtt cct ata gct aca aca gaa cct gtc aca ata ccc aat aca
   1968
   Glu Phe Val Pro Ile Ala Thr Thr Glu Pro Val Thr Ile Pro Asn Thr
                   645                 650                 655
   cct att aaa act tat aca aat cca cca aat cct caa caa gta ctt tgg
   2016
   Pro Ile Lys Thr Tyr Thr Asn Pro Pro Asn Pro Gln Gln Val Leu Trp
               660                 665                 670
   act gct cag cca ggt att ttg ggt gat ata gta aat tta tct ggc tat
   2064
   Thr Ala Gln Pro Gly Ile Leu Gly Asp Ile Val Asn Leu Ser Gly Tyr
           675                 680                 685
   act aat ggt gca aat gga tat tat acc ggt gtt atg cct gct att cgc
   2112
   Thr Asn Gly Ala Asn Gly Tyr Tyr Thr Gly Val Met Pro Ala Ile Arg
       690                 695                 700
   att caa ttt ttc cga aac aat caa tta gtg gat cac tat gat act tcc
   2160
   Ile Gln Phe Phe Arg Asn Asn Gln Leu Val Asp His Tyr Asp Thr Ser
   705                 710                 715                 720
   gaa ggc aga tac cct cat aat gct gat ttt aat atg tct aac tat aaa
   2208
   Glu Gly Arg Tyr Pro His Asn Ala Asp Phe Asn Met Ser Asn Tyr Lys
                   725                 730                 735
   gta act ggt gga ttt gat aaa att gtt tta att cca ata cat caa tat
   2256
   Val Thr Gly Gly Phe Asp Lys Ile Val Leu Ile Pro Ile His Gln Tyr
               740                 745                 750
   tac act gaa cct gta gaa ggt cag ata agt ggt acc ata aca cta ata
   2304
   Tyr Thr Glu Pro Val Glu Gly Gln Ile Ser Gly Thr Ile Thr Leu Ile
           755                 760                 765
   aag att caa aac aaa ttc atg aca gaa gaa gac tta acc aaa gta acc
   2352
   Lys Ile Gln Asn Lys Phe Met Thr Glu Glu Asp Leu Thr Lys Val Thr
       770                 775                 780
   cag gaa gtg aat gcg tta ttt ata aca gat acg caa tta gct tcg acc
   2400
   Gln Glu Val Asn Ala Leu Phe Ile Thr Asp Thr Gln Leu Ala Ser Thr
   785                 790                 795                 800
   gtg acg gat tat tgg att gat caa gtt tac ctg aaa gtc aat gct tta
   2448
   Val Thr Asp Tyr Trp Ile Asp Gln Val Tyr Leu Lys Val Asn Ala Leu
                   805                 810                 815
   tca gat gat ttg ttt gga aca gaa aaa gaa agg ctg cgc caa cgt atg
   2496
   Ser Asp Asp Leu Phe Gly Thr Glu Lys Glu Arg Leu Arg Gln Arg Met
               820                 825                 830
   gct cgg gct aag caa cta aat aat aca aaa aat ata tta gtg ggt ggc
   2544
   Ala Arg Ala Lys Gln Leu Asn Asn Thr Lys Asn Ile Leu Val Gly Gly
           835                 840                 845
   tca ttt caa acc gta aca cat tgg caa ctt agt tca ggt gta gca ctc
   2592
   Ser Phe Gln Thr Val Thr His Trp Gln Leu Ser Ser Gly Val Ala Leu
       850                 855                 860
   cta gct gat aat cca tta ttt gcg gga aca tat gta tca tta cct cct
   2640
   Leu Ala Asp Asn Pro Leu Phe Ala Gly Thr Tyr Val Ser Leu Pro Pro
   865                 870                 875                 880
   tcc act tat cct gat aca aaa cct tct tat gtg tat caa aaa gtg gat
   2688
   Ser Thr Tyr Pro Asp Thr Lys Pro Ser Tyr Val Tyr Gln Lys Val Asp
                   885                 890                 895
   gaa agt aaa cta aaa cca tat acg cgc tat atc gta aga ggt ttt att
   2736
   Glu Ser Lys Leu Lys Pro Tyr Thr Arg Tyr Ile Val Arg Gly Phe Ile
               900                 905                 910
   gga gaa gca gaa gac tta gca ctc atg gtt tct cga tat ggg aaa gaa
   2784
   Gly Glu Ala Glu Asp Leu Ala Leu Met Val Ser Arg Tyr Gly Lys Glu
           915                 920                 925
   att gat aca gct ctt acg gtt cct tat caa gaa gcg tta cca tta tca
   2832
   Ile Asp Thr Ala Leu Thr Val Pro Tyr Gln Glu Ala Leu Pro Leu Ser
   930 935 940
   ccg gat agt tca tcg aat tgt tgt gga cca gtt gct tgt ccg cca tgt
   2880
   Pro Asp Ser Ser Ser Asn Cys Cys Gly Pro Val Ala Cys Pro Pro Cys
   945 950 955 960
   gaa gga cat aat tat gat gca cat caa ttt tcc tat acc att gat gta
   2928
   Glu Gly His Asn Tyr Asp Ala His Gln Phe Ser Tyr Thr Ile Asp Val
   965 970 975
   ggg gct tta caa cta gaa agc aat cta ggc att gaa att ggc ttc aaa
   2976
   Gly Ala Leu Gln Leu Glu Ser Asn Leu Gly Ile Glu Ile Gly Phe Lys
   980 985 990
   att act agc cca acg ggg ttt gca caa ata agc aac ctt gaa att gta
   3024
   Ile Thr Ser Pro Thr Gly Phe Ala Gln Ile Ser Asn Leu Glu Ile Val
   995 1000 1005
   gaa gac cgt tct tta aca gaa gcg gag aca atc aaa gta caa caa cgc
   3072
   Glu Asp Arg Ser Leu Thr Glu Ala Glu Thr Ile Lys Val Gln Gln Arg
   1010 1015 1020
   gaa aaa caa tgg cta cgt ctg tct caa aaa caa caa tca caa tta caa
   3120
   Glu Lys Gln Trp Leu Arg Leu Ser Gln Lys Gln Gln Ser Gln Leu Gln
   1025 1030 1035 1040
   aaa cag tat gat caa acg atg caa tat ttc gct act tta tat aca aca
   3168
   Lys Gln Tyr Asp Gln Thr Met Gln Tyr Phe Ala Thr Leu Tyr Thr Thr
   1045 1050 1055
   tca gac caa acg gag ctt aaa aat act gtg caa tat aca gat att gca
   3216
   Ser Asp Gln Thr Glu Leu Lys Asn Thr Val Gln Tyr Thr Asp Ile Ala
   1060 1065 1070
   aac gtt caa gtt ata aca ttc ccg tct act atg cag tgg ttt atc cct
   3264
   Asn Val Gln Val Ile Thr Phe Pro Ser Thr Met Gln Trp Phe Ile Pro
   1075 1080 1085
   caa tta tca aga aca tcg tct cct atg ata gag gag tta gta cgt aca
   3312
   Gln Leu Ser Arg Thr Ser Ser Pro Met Ile Glu Glu Leu Val Arg Thr
   1090 1095 1100
   aaa gaa aaa gct ttg caa tta tat cca acc aat gtc ata caa aac gga
   3360
   Lys Glu Lys Ala Leu Gln Leu Tyr Pro Thr Asn Val Ile Gln Asn Gly
   1105 1110 1115 1120
   aat ttc tct tcc ggt tta tct act tgg cat gtg ata gaa aat aca aac
   3408
   Asn Phe Ser Ser Gly Leu Ser Thr Trp His Val Ile Glu Asn Thr Asn
                   1125                1130                1135
   gta cgt ata gag ttc att aat ggt ata tct gta tta cat gtg cct tct
   3456
   Val Arg Ile Glu Phe Ile Asn Gly Ile Ser Val Leu His Val Pro Ser
               1140                1145                1150
   tgg gat gaa act gta tca caa acg att aca tta ccg cca cac caa gaa
   3504
   Trp Asp Glu Thr Val Ser Gln Thr Ile Thr Leu Pro Pro His Gln Glu
           1155                1160                1165
   aat atc tta tat caa tta cgc gta act gca aaa gga aat ggt agt gtt
   3552
   Asn Ile Leu Tyr Gln Leu Arg Val Thr Ala Lys Gly Asn Gly Ser Val
       1170                1175                1180
   atc ctt cag cat aat ggc gaa caa gaa aga cta tat ttc gat caa aat
   3600
   Ile Leu Gln His Asn Gly Glu Gln Glu Arg Leu Tyr Phe Asp Gln Asn
   1185                1190                1195                1200
   aat tat ctg cag aat tcc agc aca ctg gcg gcc gtt act agt gga tcc
   3648
   Asn Tyr Leu Gln Asn Ser Ser Thr Leu Ala Ala Val Thr Ser Gly Ser
                   1205                1210                1215
   gag ctc ggt acc aag ctt gat gca tag
   3675
   Glu Leu Gly Thr Lys Leu Asp Ala  *
               1220
<210> 2
   <211> 1224
   <212> PRT
   <213> Bacillus thuringiensis
<400> 2
   Met Thr Gln Asn His Ser Phe Ser Asp Asn Thr Ser Ser Ser Thr Gly
    1               5                  10                  15
   Val Ser Thr Leu Glu Ser Ser Leu Ile Pro Tyr Asn Val Tyr Ala Thr
               20                  25                  30
   Asp Gln Phe Asn Ser Asn Lys Asn Trp Glu Asp Ala Leu Lys Lys Leu
           35                  40                  45
   Leu Glu Lys Phe Tyr Ser Gly Asp Leu Thr Gln Asp Ala Ile Asp Ile
       50                  55                  60
   Phe Leu Gly Asp Ser Gly Phe Asp Tyr Leu Ser Leu Val Asn Val Ile
   65                  70                  75                  80
   Phe Ser Ile Ala Gly Ser Phe Ile Pro Tyr Val Gly Ala Leu Val Pro
                   85                  90                  95
   Ile Ile Asn Leu Leu Phe Gly Ser Glu Ser Lys Pro Asp Val Phe Glu
               100                 105                 110
   Gln Met Arg Ala Arg Ile Glu Ala Leu Ile His Lys Glu Leu Ser Ala
           115                 120                 125
   Asp His Val Gln Thr Leu Lys Ala Glu Ile Lys Gly Leu Lys Asp Thr
       130                 135                 140
   Gly Asp Leu Tyr Gln Lys Asp Val Asn Ala Val Ala Gly Arg Thr Asn
   145                 150                 155                 160
   Gly Pro Thr Pro Pro Ser Phe Asp Ser Asn Thr Asp Ala Leu Lys Ala
                   165                 170                 175
   Glu Leu Arg Ser Gln Ile Thr Ala Thr Asn Thr Leu Phe Val Gln Arg
               180                 185                 190
   Met Pro Gln Phe Ala Ile Glu Gly Tyr Glu Glu Ile Thr Leu Pro Leu
           195                 200                 205
   His Thr Ile Ala Ala Ser Met His Leu Ile Phe Leu Lys Asp Val Cys
       210                 215                 220
   Glu His Gly Ala Glu Trp Gly Ile Ala Asn Thr Thr Leu Thr Asn Tyr
   225                 230                 235                 240
   Gln Gly Gln Leu Gln Asp Cys Ile Arg Glu Tyr Ser Asn Lys Ala Tyr
                   245                 250                 255
   Ser Met Phe Asn Ile Gly Leu Gln Arg Ala Lys Asn Asn Gly Asn Asn
               260                 265                 270
   Met Trp Asn Asn Val Asn Asn Tyr Ile Arg Thr Met Lys Leu Asn Ala
           275                 280                 285
   Leu Asp Thr Val Ala Gln Trp Pro Ile Leu Asp Lys Val Thr Tyr Pro
       290                 295                 300
   Leu Asp Thr Thr Leu Gln Gln Thr Arg Gly Ile Phe Ser Asp Leu Ser
   305                 310                 315                 320
   Gly Arg Gly Gly Thr Gln Ser Asn Tyr Arg Tyr Asp Tyr Asp Ala Val
                   325                 330                 335
   Gln Gly Tyr Ala Pro Pro Phe Val Gly Phe Asp Thr Lys Leu Asn Val
               340                 345                 350
   Val Asn Asp Phe Gly Tyr Lys Asp Leu Thr Ala Ile Gln Thr Phe Thr
           355                 360                 365
   Gly Asp Arg Ile Asp Ser Ile Trp Gln Ser Phe Lys Tyr Asn Ser Gly
       370                 375                 380
   Glu Pro Phe Leu Thr Asn Leu Gly Asn Gly Lys Pro Gly Asn Asn Pro
   385                 390                 395                 400
   Val Ile Pro Asn Ser Arg Asp Asn Pro Ile Ile Ser Ala Lys Gly Ser
                   405                 410                 415
   Arg Pro Ser Ala Asn Tyr Val Gly Met Asn Phe Gln Arg Ala Asn Lys
               420                 425                 430
   Thr Val Val Ser Asn Gly Tyr Val Ile Pro Asn Asp Asn Tyr Thr Val
           435                 440                 445
   Pro Ala Gly His Lys Leu Gly Trp Ile Ser Ala Leu His Asp Glu Leu
       450                 455                 460
   Asp Asn Ala Asn Asn Ala Asp Leu Val Val Ser Val Trp Val Lys Asn
   465                 470                 475                 480
   Asp Ile Phe Gln Glu Asn Ile Ile Gly Ser Ile Lys Thr Val Thr Thr
                   485                 490                 495
   Asp Asp Gly Thr Thr Glu Asn Arg Gln Gln Ile Ile Gly Ile Pro Ala
               500                 505                 510
   Asp Lys His Met Thr Arg Ser Thr Lys Arg Met Glu Leu Glu Phe Ile
           515                 520                 525
   Asn Gly Thr Asn Gly Ser Met Ser Leu Ser Ser Thr Asn Asp Gln Leu
       530                 535                 540
   Tyr Tyr Thr Ile Asn Pro Ile Val Ser Gln Arg Tyr Gln Ile Arg Tyr
   545                 550                 555                 560
   Arg Val Ala Thr Thr Ser Ala Glu Ser Leu Asp Leu Trp Ile Asp Gly
                   565                 570                 575
   Tyr Lys Arg Gly Thr Thr Pro Leu Pro Asn Thr Ser Ser Thr Ser Thr
               580                 585                 590
   Gln Thr Gln Lys Val Ile Ile Gln Gly Leu Gln Gly Lys Tyr Gln Leu
           595                 600                 605
   Ile Asn Gly Pro Thr Leu Asp Leu Thr Ala Gly Ser His Thr Phe Gly
       610                 615                 620
   Ile Met Leu Thr Ala Asn Ala Ser Gln Asn Val Phe Ile Asp Arg Ile
   625 630 635 640
   Glu Phe Val Pro Ile Ala Thr Thr Glu Pro Val Thr Ile Pro Asn Thr
   645 650 655
   Pro Ile Lys Thr Tyr Thr Asn Pro Pro Asn Pro Gln Gln Val Leu Trp
   660 665 670
   Thr Ala Gln Pro Gly Ile Leu Gly Asp Ile Val Asn Leu Ser Gly Tyr
   675 680 685
   Thr Asn Gly Ala Asn Gly Tyr Tyr Thr Gly Val Met Pro Ala Ile Arg
   690 695 700
   Ile Gln Phe Phe Arg Asn Asn Gln Leu Val Asp His Tyr Asp Thr Ser
   705 710 715 720
   Glu Gly Arg Tyr Pro His Asn Ala Asp Phe Asn Met Ser Asn Tyr Lys
   725 730 735
   Val Thr Gly Gly Phe Asp Lys Ile Val Leu Ile Pro Ile His Gln Tyr
   740 745 750
   Tyr Thr Glu Pro Val Glu Gly Gln Ile Ser Gly Thr Ile Thr Leu Ile
   755 760 765
   Lys Ile Gln Asn Lys Phe Met Thr Glu Glu Asp Leu Thr Lys Val Thr
   770 775 780
   Gln Glu Val Asn Ala Leu Phe Ile Thr Asp Thr Gln Leu Ala Ser Thr
   785 790 795 800
   Val Thr Asp Tyr Trp Ile Asp Gln Val Tyr Leu Lys Val Asn Ala Leu
   805 810 815
   Ser Asp Asp Leu Phe Gly Thr Glu Lys Glu Arg Leu Arg Gln Arg Met
   820 825 830
   Ala Arg Ala Lys Gln Leu Asn Asn Thr Lys Asn Ile Leu Val Gly Gly
   835 840 845
   Ser Phe Gln Thr Val Thr His Trp Gln Leu Ser Ser Gly Val Ala Leu
   850 855 860
   Leu Ala Asp Asn Pro Leu Phe Ala Gly Thr Tyr Val Ser Leu Pro Pro
   865 870 875 880
   Ser Thr Tyr Pro Asp Thr Lys Pro Ser Tyr Val Tyr Gln Lys Val Asp
   885 890 895
   Glu Ser Lys Leu Lys Pro Tyr Thr Arg Tyr Ile Val Arg Gly Phe Ile
   900 905 910
   Gly Glu Ala Glu Asp Leu Ala Leu Met Val Ser Arg Tyr Gly Lys Glu
   915 920 925
   Ile Asp Thr Ala Leu Thr Val Pro Tyr Gln Glu Ala Leu Pro Leu Ser
   930 935 940
   Pro Asp Ser Ser Ser Asn Cys Cys Gly Pro Val Ala Cys Pro Pro Cys
   945 950 955 960
   Glu Gly His Asn Tyr Asp Ala His Gln Phe Ser Tyr Thr Ile Asp Val
   965 970 975
   Gly Ala Leu Gln Leu Glu Ser Asn Leu Gly Ile Glu Ile Gly Phe Lys
   980 985 990
   Ile Thr Ser Pro Thr Gly Phe Ala Gln Ile Ser Asn Leu Glu Ile Val
   995 1000 1005
   Glu Asp Arg Ser Leu Thr Glu Ala Glu Thr Ile Lys Val Gln Gln Arg
   1010 1015 1020
   Glu Lys Gln Trp Leu Arg Leu Ser Gln Lys Gln Gln Ser Gln Leu Gln
   1025 1030 1035 1040
   Lys Gln Tyr Asp Gln Thr Met Gln Tyr Phe Ala Thr Leu Tyr Thr Thr
   1045 1050 1055
   Ser Asp Gln Thr Glu Leu Lys Asn Thr Val Gln Tyr Thr Asp Ile Ala
   1060 1065 1070
   Asn Val Gln Val Ile Thr Phe Pro Ser Thr Met Gln Trp Phe Ile Pro
   1075 1080 1085
   Gln Leu Ser Arg Thr Ser Ser Pro Met Ile Glu Glu Leu Val Arg Thr
   1090 1095 1100
   Lys Glu Lys Ala Leu Gln Leu Tyr Pro Thr Asn Val Ile Gln Asn Gly
   1105                1110                1115                1120
   Asn Phe Ser Ser Gly Leu Ser Thr Trp His Val Ile Glu Asn Thr Asn
                   1125                1130                1135
   Val Arg Ile Glu Phe Ile Asn Gly Ile Ser Val Leu His Val Pro Ser
               1140                1145                1150
   Trp Asp Glu Thr Val Ser Gln Thr Ile Thr Leu Pro Pro His Gln Glu
           1155                1160                1165
   Asn Ile Leu Tyr Gln Leu Arg Val Thr Ala Lys Gly Asn Gly Ser Val
       1170                1175                1180
   Ile Leu Gln His Asn Gly Glu Gln Glu Arg Leu Tyr Phe Asp Gln Asn
   1185                1190                1195                1200
   Asn Tyr Leu Gln Asn Ser Ser Thr Leu Ala Ala Val Thr Ser Gly Ser
                   1205                1210                1215
   Glu Leu Gly Thr Lys Leu Asp Ala
               1220
<210> 3
   <211> 3708
   <212> DNA
   <213> Bacillus thuringiensis
<220>
   <221> CDS
   <222> (1)...(3708)
<400> 3
   atg aca caa aat cat tca ttc tct gat aat aca tcc tca tcg acg ggt    48
   Met Thr Gln Asn His Ser Phe Ser Asp Asn Thr Ser Ser Ser Thr Gly
    1               5                  10                  15
   gta tct act tta gaa tca tct tta att cct tac aat gtg tac gcg aca    96
   Val Ser Thr Leu Glu Ser Ser Leu Ile Pro Tyr Asn Val Tyr Ala Thr
               20                  25                  30
   gat cag ttt aac tct aat aaa aat tgg gaa gat gca ctg aaa aaa tta   144
   Asp Gln Phe Asn Ser Asn Lys Asn Trp Glu Asp Ala Leu Lys Lys Leu
           35                  40                  45
   tta gaa aaa ttt tat tcc ggt gat tta aca cag gat gct att gat att   192
   Leu Glu Lys Phe Tyr Ser Gly Asp Leu Thr Gln Asp Ala Ile Asp Ile
       50                  55                  60
   ttt ctt ggt gac agc ggc ttt gat tac tta tct tta gta aat gtt att   240
   Phe Leu Gly Asp Ser Gly Phe Asp Tyr Leu Ser Leu Val Asn Val Ile
   65                  70                  75                  80
   ttt tct att gca gga tct ttt att cct tat gtg ggt gct ctt gtc cct   288
   Phe Ser Ile Ala Gly Ser Phe Ile Pro Tyr Val Gly Ala Leu Val Pro
                   85                  90                  95
   atc att aat ctt ctt ttt gga tca gag agc aaa cca gat gta ttt gaa   336
   Ile Ile Asn Leu Leu Phe Gly Ser Glu Ser Lys Pro Asp Val Phe Glu
               100                 105                 110
   caa atg aga gca cga att gaa gca tta att cat aag gaa tta tct gca   384
   Gln Met Arg Ala Arg Ile Glu Ala Leu Ile His Lys Glu Leu Ser Ala
           115                 120                 125
   gac cat gtg caa aca tta aaa gca gaa att aag gga ctt aaa gat acg     432
   Asp His Val Gln Thr Leu Lys Ala Glu Ile Lys Gly Leu Lys Asp Thr
       130                 135                 140
   gga gat cta tat caa aaa gat gta aat gct gtt gca gga aga aca aat     480
   Gly Asp Leu Tyr Gln Lys Asp Val Asn Ala Val Ala Gly Arg Thr Asn
   145                 150                 155                 160
   gga cct acc cct cca tca ttt gat agc aat aca gat gct tta aaa gca     528
   Gly Pro Thr Pro Pro Ser Phe Asp Ser Asn Thr Asp Ala Leu Lys Ala
                   165                 170                 175
   gaa ctt cga agt caa atc aca gct aca aac act cta ttt gtg caa cga     576
   Glu Leu Arg Ser Gln Ile Thr Ala Thr Asn Thr Leu Phe Val Gln Arg
               180                 185                 190
   atg cct caa ttt gct ata gag gga tat gaa gag att act cta cct tta     624
   Met Pro Gln Phe Ala Ile Glu Gly Tyr Glu Glu Ile Thr Leu Pro Leu
           195                 200                 205
   cac act atc gct gca agt atg cat ctt ata ttc tta aaa gat gtt tgt     672
   His Thr Ile Ala Ala Ser Met His Leu Ile Phe Leu Lys Asp Val Cys
       210                 215                 220
   gaa cat ggt gct gaa tgg gga att gct aat act aca tta aca aat tat     720
   Glu His Gly Ala Glu Trp Gly Ile Ala Asn Thr Thr Leu Thr Asn Tyr
   225                 230                 235                 240
   caa ggt caa tta caa gat tgt att aga gag tat tca aat aaa gct tat     768
   Gln Gly Gln Leu Gln Asp Cys Ile Arg Glu Tyr Ser Asn Lys Ala Tyr
                   245                 250                 255
   tcg atg ttc aat att ggt tta cag agg gca aaa aat aat gga aac aat     816
   Ser Met Phe Asn Ile Gly Leu Gln Arg Ala Lys Asn Asn Gly Asn Asn
               260                 265                 270
   atg tgg aat aac gta aat aac tat atc cgc aca atg aaa tta aat gct     864
   Met Trp Asn Asn Val Asn Asn Tyr Ile Arg Thr Met Lys Leu Asn Ala
           275                 280                 285
   tta gat act gtt gct caa tgg cct att ctg gat aaa gta aca tac cca     912
   Leu Asp Thr Val Ala Gln Trp Pro Ile Leu Asp Lys Val Thr Tyr Pro
       290                 295                 300
   tta gat aca aca tta caa caa aca cgc ggt ata ttt tca gat cta tca     960
   Leu Asp Thr Thr Leu Gln Gln Thr Arg Gly Ile Phe Ser Asp Leu Ser
   305                 310                 315                 320
   ggt agg ggg ggg aca caa tct aat tat aga tat gat tat gat gct gtt
   1008
   Gly Arg Gly Gly Thr Gln Ser Asn Tyr Arg Tyr Asp Tyr Asp Ala Val
                   325                 330                 335
   caa ggt tat gct cct tct ttt gtc gga ttt gat acc gaa cta aat gtt
   1056
   Gln Gly Tyr Ala Pro Ser Phe Val Gly Phe Asp Thr Glu Leu Asn Val
               340                 345                 350
   gta aac gat ttt ggt tat aaa gat tta acc gca att cag aca ttt aca
   1104
   Val Asn Asp Phe Gly Tyr Lys Asp Leu Thr Ala Ile Gln Thr Phe Thr
   355 360 365
   ggt gat cga att gat tca att tgg caa tca ttt aag tat aat tca gga
   1152
   Gly Asp Arg Ile Asp Ser Ile Trp Gln Ser Phe Lys Tyr Asn Ser Gly
   370 375 380
   gag cct ttt ctc acg aac tta ggg aat ggt aaa cgc gga aac aac ccc
   1200
   Glu Pro Phe Leu Thr Asn Leu Gly Asn Gly Lys Arg Gly Asn Asn Pro
   385 390 395 400
   gtg att cca aat agc aga gat aat ccg att att tcc gca aaa gga tct
   1248
   Val Ile Pro Asn Ser Arg Asp Asn Pro Ile Ile Ser Ala Lys Gly Ser
   405 410 415
   aga cca tct gca aac tat gtt ggg atg aat ttc caa cga gca gat aaa
   1296
   Arg Pro Ser Ala Asn Tyr Val Gly Met Asn Phe Gln Arg Ala Asp Lys
   420 425 430
   act gta gtt cca aat gga tat gta att cct aat gac aat tat aca gta
   1344
   Thr Val Val Pro Asn Gly Tyr Val Ile Pro Asn Asp Asn Tyr Thr Val
   435 440 445
   ccc gct ggg cat aaa ctt gga tgg att tca gcc ctg cat gat gaa tta
   1392
   Pro Ala Gly His Lys Leu Gly Trp Ile Ser Ala Leu His Asp Glu Leu
   450 455 460
   gat aat gca aat aat gcg gat cta gtt gta tcg gtt tgg gtg aaa aat
   1440
   Asp Asn Ala Asn Asn Ala Asp Leu Val Val Ser Val Trp Val Lys Asn
   465 470 475 480
   gat atc ttc cag gaa aat att atc ggt tcc ata aaa aca gtt act act
   1488
   Asp Ile Phe Gln Glu Asn Ile Ile Gly Ser Ile Lys Thr Val Thr Thr
   485 490 495
   gat gat gga acc aca gaa aat aga caa caa att ata ggg atc ccg gca
   1536
   Asp Asp Gly Thr Thr Glu Asn Arg Gln Gln Ile Ile Gly Ile Pro Ala
   500 505 510
   gat aaa cat atg aca aga agt aca aag cga atg gaa ctg gaa ttt atc
   1584
   Asp Lys His Met Thr Arg Ser Thr Lys Arg Met Glu Leu Glu Phe Ile
   515 520 525
   aat ggt aca aat ggg tca atg agc tta tct agt act aat gat caa ttg
   1632
   Asn Gly Thr Asn Gly Ser Met Ser Leu Ser Ser Thr Asn Asp Gln Leu
   530 535 540
   tat tat acg att aat cct ata gtt agc cag aga tat caa att cgg tat
   1680
   Tyr Tyr Thr Ile Asn Pro Ile Val Ser Gln Arg Tyr Gln Ile Arg Tyr
   545                 550                 555                 560
   cgc gta gca aca act tca gca gaa tct tta gac cta tgg atc gat ggt
   1728
   Arg Val Ala Thr Thr Ser Ala Glu Ser Leu Asp Leu Trp Ile Asp Gly
                   565                 570                 575
   tat aaa cgc gga aca acc ccg tta cca aat aca agt agc aca tca acg
   1776
   Tyr Lys Arg Gly Thr Thr Pro Leu Pro Asn Thr Ser Ser Thr Ser Thr
               580                 585                 590
   caa aca caa aaa gtg ata att caa ggg tta caa gga aaa tat caa tta
   1824
   Gln Thr Gln Lys Val Ile Ile Gln Gly Leu Gln Gly Lys Tyr Gln Leu
           595                 600                 605
   att aat gga cca att ctt gat ttg aca gca ggt tcc cat act ttt ggt
   1872
   Ile Asn Gly Pro Ile Leu Asp Leu Thr Ala Gly Ser His Thr Phe Gly
       610                 615                 620
   att gcg tta aca gca act cct tct caa aat gta ttt att gat cgg att
   1920
   Ile Ala Leu Thr Ala Thr Pro Ser Gln Asn Val Phe Ile Asp Arg Ile
   625                 630                 635                 640
   gaa ttt gtt cct ata ggg tca cct tgc cag aat ata ttt cct gct ggt
   1968
   Glu Phe Val Pro Ile Gly Ser Pro Cys Gln Asn Ile Phe Pro Ala Gly
                   645                 650                 655
   cca ttt aca gta gat aat gga aga aaa aca gtt tgg act tcc tcg aca
   2016
   Pro Phe Thr Val Asp Asn Gly Arg Lys Thr Val Trp Thr Ser Ser Thr
               660                 665                 670
   gga aca gcc ttt tca gta gaa aat att caa gga ttt gtg gga atg aga
   2064
   Gly Thr Ala Phe Ser Val Glu Asn Ile Gln Gly Phe Val Gly Met Arg
           675                 680                 685
   aat ttt aat tgg cgt att gaa ttt tta caa aaa ggg gtt act tta tct
   2112
   Asn Phe Asn Trp Arg Ile Glu Phe Leu Gln Lys Gly Val Thr Leu Ser
       690                 695                 700
   caa tat acc ata cca att acc ggg gct tca ttt gat cat tat tct ttt
   2160
   Gln Tyr Thr Ile Pro Ile Thr Gly Ala Ser Phe Asp His Tyr Ser Phe
   705                 710                 715                 720
   ggc cct ttt tct aaa gac ata cct gaa gga ttt gat acg att caa atc
   2208
   Gly Pro Phe Ser Lys Asp Ile Pro Glu Gly Phe Asp Thr Ile Gln Ile
                   725                 730                 735
   gta tct ccc gat ttt ccg ata gtt ata acg cct att gat gga aaa gtc
   2256
   Val Ser Pro Asp Phe Pro Ile Val Ile Thr Pro Ile Asp Gly Lys Val
   740 745 750
   tgt ttt gac aca agt agt caa aaa tct ttt aca acc gaa gcg gat tta
   2304
   Cys Phe Asp Thr Ser Ser Gln Lys Ser Phe Thr Thr Glu Ala Asp Leu
   755 760 765
   gcc aaa gta aca gcc gta gtc aat gcc tta ttt ata aca gat acg caa
   2352
   Ala Lys Val Thr Ala Val Val Asn Ala Leu Phe Ile Thr Asp Thr Gln
   770 775 780
   tta gct tcg acc gtg acg gat tat tgg att gat caa gtt tac ctg aaa
   2400
   Leu Ala Ser Thr Val Thr Asp Tyr Trp Ile Asp Gln Val Tyr Leu Lys
   785 790 795 800
   gtc aat gct tta tca gat gat ttg ttt gga aca gaa aaa gaa agg ctg
   2448
   Val Asn Ala Leu Ser Asp Asp Leu Phe Gly Thr Glu Lys Glu Arg Leu
   805 810 815
   cgc caa cgt atg gct cgg gct aag caa cta aat aat aca aaa aat ata
   2496
   Arg Gln Arg Met Ala Arg Ala Lys Gln Leu Asn Asn Thr Lys Asn Ile
   820 825 830
   tta gtg ggt ggc tca ttc caa acc cta aca aat tgg caa ctt agt tca
   2544
   Leu Val Gly Gly Ser Phe Gln Thr Leu Thr Asn Trp Gln Leu Ser Ser
   835 840 845
   ggt gta gca ctc cta gct gat aat cca tta ttt gcg gga aca tat gta
   2592
   Gly Val Ala Leu Leu Ala Asp Asn Pro Leu Phe Ala Gly Thr Tyr Val
   850 855 860
   tca tta cct cca tcc act tat cct gat aca aaa cct tct tat gtg tat
   2640
   Ser Leu Pro Pro Ser Thr Tyr Pro Asp Thr Lys Pro Ser Tyr Val Tyr
   865 870 875 880
   caa aaa gtg gat gaa agt aaa cta aaa cca tat acg cgc tat atc gta
   2688
   Gln Lys Val Asp Glu Ser Lys Leu Lys Pro Tyr Thr Arg Tyr Ile Val
   885 890 895
   aga ggt ttt att gga gaa gca gaa gac tta gca ctc atg gtt tct cga
   2736
   Arg Gly Phe Ile Gly Glu Ala Glu Asp Leu Ala Leu Met Val Ser Arg
   900 905 910
   tat ggg aaa gaa att gat aca gct ttt acg gtt cct tat caa gaa gcg
   2784
   Tyr Gly Lys Glu Ile Asp Thr Ala Phe Thr Val Pro Tyr Gln Glu Ala
   915 920 925
   tta cca tta tca ccg gat agt tca tcg aat tgt tgt gga cca gtt gct
   2832
   Leu Pro Leu Ser Pro Asp Ser Ser Ser Asn Cys Cys Gly Pro Val Ala
   930 935 940
   tgt ccg cca tgt gaa gga cat aat tat gat gca cat caa ttt tcc tat
   2880
   Cys Pro Pro Cys Glu Gly His Asn Tyr Asp Ala His Gln Phe Ser Tyr
   945 950 955 960
   acc att gat gta ggg gct tta caa cta gaa agc aat cta ggc att gaa
   2928
   Thr Ile Asp Val Gly Ala Leu Gln Leu Glu Ser Asn Leu Gly Ile Glu
   965 970 975
   att ggc ttc aaa att act agc cca acg ggg ttt gca caa ata agc aac
   2976
   Ile Gly Phe Lys Ile Thr Ser Pro Thr Gly Phe Ala Gln Ile Ser Asn
   980 985 990
   ctt gaa att gta gaa gac cgt tct tta aca gaa gcg gag aca atc aaa
   3024
   Leu Glu Ile Val Glu Asp Arg Ser Leu Thr Glu Ala Glu Thr Ile Lys
   995 1000 1005
   gta caa caa cgc gaa aaa caa tgg cta cgt ctg tct caa aaa caa caa
   3072
   Val Gln Gln Arg Glu Lys Gln Trp Leu Arg Leu Ser Gln Lys Gln Gln
   1010 1015 1020
   tca caa tta caa aaa cag tat gat caa acg atg caa tat ttc gct act
   3120
   Ser Gln Leu Gln Lys Gln Tyr Asp Gln Thr Met Gln Tyr Phe Ala Thr
   1025 1030 1035 1040
   tta tat aca aca tca gac caa acg gag ctt aaa aat act gtg caa tat
   3168
   Leu Tyr Thr Thr Ser Asp Gln Thr Glu Leu Lys Asn Thr Val Gln Tyr
   1045 1050 1055
   aca gat att gca aac gtt caa gtt ata aca ttc ccg tct act atg cag
   3216
   Thr Asp Ile Ala Asn Val Gln Val Ile Thr Phe Pro Ser Thr Met Gln
   1060 1065 1070
   tgg ttt atc cct caa tta cga aga aca tcg tct cct atg ata gag gag
   3264
   Trp Phe Ile Pro Gln Leu Arg Arg Thr Ser Ser Pro Met Ile Glu Glu
   1075 1080 1085
   tta gta cgt aca aaa gaa aaa gct ttg caa tta tat cca acc aat gtc
   3312
   Leu Val Arg Thr Lys Glu Lys Ala Leu Gln Leu Tyr Pro Thr Asn Val
   1090 1095 1100
   ata caa aac gga aat ttc tct tcc ggt tta tct act tgg cat gtg ata
   3360
   Ile Gln Asn Gly Asn Phe Ser Ser Gly Leu Ser Thr Trp His Val Ile
   1105 1110 1115 1120
   gaa aat aca aac gta cgt ata gag ttc att aat ggt ata tct gta tta
   3408
   Glu Asn Thr Asn Val Arg Ile Glu Phe Ile Asn Gly Ile Ser Val Leu
                   1125                1130                1135
   cat gtg cct tct tgg gat gaa act gta tca caa acg att aca tta ccg
   3456
   His Val Pro Ser Trp Asp Glu Thr Val Ser Gln Thr Ile Thr Leu Pro
               1140                1145                1150
   cca cac caa gaa aat atc tta tat caa tta cgc gta act gca aaa gga
   3504
   Pro His Gln Glu Asn Ile Leu Tyr Gln Leu Arg Val Thr Ala Lys Gly
           1155                1160                1165
   aat ggt agt gtt atc ctt cag cat aat ggc gaa caa gaa aga cta tat
   3552
   Asn Gly Ser Val Ile Leu Gln His Asn Gly Glu Gln Glu Arg Leu Tyr
       1170                1175                1180
   ttc gat caa aat aat tca aca gga aat act ttt gta aca aaa gaa att
   3600
   Phe Asp Gln Asn Asn Ser Thr Gly Asn Thr Phe Val Thr Lys Glu Ile
   1185                1190                1195                1200
   tcc ttt tat cca aca gct tca act tta tca ctt cag atc caa tct gaa
   3648
   Ser Phe Tyr Pro Thr Ala Ser Thr Leu Ser Leu Gln Ile Gln Ser Glu
                   1205                1210                1215
   gga aca gat ttt tat gta aaa aca atc gac ttg ttt gta aaa cct gta
   3696
   Gly Thr Asp Phe Tyr Val Lys Thr Ile Asp Leu Phe Val Lys Pro Val
               1220                1225                1230
   cca ttg aca taa
   3708
   Pro Leu Thr  *
           1235
<210> 4
   <211> 1235
   <212> PRT
   <213> Bacillus thuringiensis
<400> 4
   Met Thr Gln Asn His Ser Phe Ser Asp Asn Thr Ser Ser Ser Thr Gly
    1               5                  10                  15
   Val Ser Thr Leu Glu Ser Ser Leu Ile Pro Tyr Asn Val Tyr Ala Thr
               20                  25                  30
   Asp Gln Phe Asn Ser Asn Lys Asn Trp Glu Asp Ala Leu Lys Lys Leu
           35                  40                  45
   Leu Glu Lys Phe Tyr Ser Gly Asp Leu Thr Gln Asp Ala Ile Asp Ile
       50                  55                  60
   Phe Leu Gly Asp Ser Gly Phe Asp Tyr Leu Ser Leu Val Asn Val Ile
   65                  70                  75                  80
   Phe Ser Ile Ala Gly Ser Phe Ile Pro Tyr Val Gly Ala Leu Val Pro
                   85                  90                  95
   Ile Ile Asn Leu Leu Phe Gly Ser Glu Ser Lys Pro Asp Val Phe Glu
               100                 105                 110
   Gln Met Arg Ala Arg Ile Glu Ala Leu Ile His Lys Glu Leu Ser Ala
           115                 120                 125
   Asp His Val Gln Thr Leu Lys Ala Glu Ile Lys Gly Leu Lys Asp Thr
       130                 135                 140
   Gly Asp Leu Tyr Gln Lys Asp Val Asn Ala Val Ala Gly Arg Thr Asn
   145                 150                 155                 160
   Gly Pro Thr Pro Pro Ser Phe Asp Ser Asn Thr Asp Ala Leu Lys Ala
                   165                 170                 175
   Glu Leu Arg Ser Gln Ile Thr Ala Thr Asn Thr Leu Phe Val Gln Arg
               180                 185                 190
   Met Pro Gln Phe Ala Ile Glu Gly Tyr Glu Glu Ile Thr Leu Pro Leu
           195                 200                 205
   His Thr Ile Ala Ala Ser Met His Leu Ile Phe Leu Lys Asp Val Cys
       210                 215                 220
   Glu His Gly Ala Glu Trp Gly Ile Ala Asn Thr Thr Leu Thr Asn Tyr
   225                 230                 235                 240
   Gln Gly Gln Leu Gln Asp Cys Ile Arg Glu Tyr Ser Asn Lys Ala Tyr
                   245                 250                 255
   Ser Met Phe Asn Ile Gly Leu Gln Arg Ala Lys Asn Asn Gly Asn Asn
               260                 265                 270
   Met Trp Asn Asn Val Asn Asn Tyr Ile Arg Thr Met Lys Leu Asn Ala
           275                 280                 285
   Leu Asp Thr Val Ala Gln Trp Pro Ile Leu Asp Lys Val Thr Tyr Pro
       290                 295                 300
   Leu Asp Thr Thr Leu Gln Gln Thr Arg Gly Ile Phe Ser Asp Leu Ser
   305                 310                 315                 320
   Gly Arg Gly Gly Thr Gln Ser Asn Tyr Arg Tyr Asp Tyr Asp Ala Val
                   325                 330                 335
   Gln Gly Tyr Ala Pro Ser Phe Val Gly Phe Asp Thr Glu Leu Asn Val
               340                 345                 350
   Val Asn Asp Phe Gly Tyr Lys Asp Leu Thr Ala Ile Gln Thr Phe Thr
           355                 360                 365
   Gly Asp Arg Ile Asp Ser Ile Trp Gln Ser Phe Lys Tyr Asn Ser Gly
       370                 375                 380
   Glu Pro Phe Leu Thr Asn Leu Gly Asn Gly Lys Arg Gly Asn Asn Pro
   385                 390                 395                 400
   Val Ile Pro Asn Ser Arg Asp Asn Pro Ile Ile Ser Ala Lys Gly Ser
                   405                 410                 415
   Arg Pro Ser Ala Asn Tyr Val Gly Met Asn Phe Gln Arg Ala Asp Lys
               420                 425                 430
   Thr Val Val Pro Asn Gly Tyr Val Ile Pro Asn Asp Asn Tyr Thr Val
           435                 440                 445
   Pro Ala Gly His Lys Leu Gly Trp Ile Ser Ala Leu His Asp Glu Leu
       450                 455                 460
   Asp Asn Ala Asn Asn Ala Asp Leu Val Val Ser Val Trp Val Lys Asn
   465                 470                 475                 480
   Asp Ile Phe Gln Glu Asn Ile Ile Gly Ser Ile Lys Thr Val Thr Thr
                   485                 490                 495
   Asp Asp Gly Thr Thr Glu Asn Arg Gln Gln Ile Ile Gly Ile Pro Ala
               500                 505                 510
   Asp Lys His Met Thr Arg Ser Thr Lys Arg Met Glu Leu Glu Phe Ile
           515                 520                 525
   Asn Gly Thr Asn Gly Ser Met Ser Leu Ser Ser Thr Asn Asp Gln Leu
       530                 535                 540
   Tyr Tyr Thr Ile Asn Pro Ile Val Ser Gln Arg Tyr Gln Ile Arg Tyr
   545                 550                 555                 560
   Arg Val Ala Thr Thr Ser Ala Glu Ser Leu Asp Leu Trp Ile Asp Gly
                   565                 570                 575
   Tyr Lys Arg Gly Thr Thr Pro Leu Pro Asn Thr Ser Ser Thr Ser Thr
               580                 585                 590
   Gln Thr Gln Lys Val Ile Ile Gln Gly Leu Gln Gly Lys Tyr Gln Leu
           595                 600                 605
   Ile Asn Gly Pro Ile Leu Asp Leu Thr Ala Gly Ser His Thr Phe Gly
       610                 615                 620
   Ile Ala Leu Thr Ala Thr Pro Ser Gln Asn Val Phe Ile Asp Arg Ile
   625                 630                 635                 640
   Glu Phe Val Pro Ile Gly Ser Pro Cys Gln Asn Ile Phe Pro Ala Gly
                   645                 650                 655
   Pro Phe Thr Val Asp Asn Gly Arg Lys Thr Val Trp Thr Ser Ser Thr
               660                 665                 670
   Gly Thr Ala Phe Ser Val Glu Asn Ile Gln Gly Phe Val Gly Met Arg
           675                 680                 685
   Asn Phe Asn Trp Arg Ile Glu Phe Leu Gln Lys Gly Val Thr Leu Ser
       690                 695                 700
   Gln Tyr Thr Ile Pro Ile Thr Gly Ala Ser Phe Asp His Tyr Ser Phe
   705                 710                 715                 720
   Gly Pro Phe Ser Lys Asp Ile Pro Glu Gly Phe Asp Thr Ile Gln Ile
                   725                 730                 735
   Val Ser Pro Asp Phe Pro Ile Val Ile Thr Pro Ile Asp Gly Lys Val
               740                 745                 750
   Cys Phe Asp Thr Ser Ser Gln Lys Ser Phe Thr Thr Glu Ala Asp Leu
           755                 760                 765
   Ala Lys Val Thr Ala Val Val Asn Ala Leu Phe Ile Thr Asp Thr Gln
       770                 775                 780
   Leu Ala Ser Thr Val Thr Asp Tyr Trp Ile Asp Gln Val Tyr Leu Lys
   785                 790                 795                 800
   Val Asn Ala Leu Ser Asp Asp Leu Phe Gly Thr Glu Lys Glu Arg Leu
                   805                 810                 815
   Arg Gln Arg Met Ala Arg Ala Lys Gln Leu Asn Asn Thr Lys Asn Ile
               820                 825                 830
   Leu Val Gly Gly Ser Phe Gln Thr Leu Thr Asn Trp Gln Leu Ser Ser
           835                 840                 845
   Gly Val Ala Leu Leu Ala Asp Asn Pro Leu Phe Ala Gly Thr Tyr Val
       850                 855                 860
   Ser Leu Pro Pro Ser Thr Tyr Pro Asp Thr Lys Pro Ser Tyr Val Tyr
   865                 870                 875                 880
   Gln Lys Val Asp Glu Ser Lys Leu Lys Pro Tyr Thr Arg Tyr Ile Val
                   885                 890                 895
   Arg Gly Phe Ile Gly Glu Ala Glu Asp Leu Ala Leu Met Val Ser Arg
               900                 905                 910
   Tyr Gly Lys Glu Ile Asp Thr Ala Phe Thr Val Pro Tyr Gln Glu Ala
           915                 920                 925
   Leu Pro Leu Ser Pro Asp Ser Ser Ser Asn Cys Cys Gly Pro Val Ala
       930                 935                 940
   Cys Pro Pro Cys Glu Gly His Asn Tyr Asp Ala His Gln Phe Ser Tyr
   945                 950                 955                 960
   Thr Ile Asp Val Gly Ala Leu Gln Leu Glu Ser Asn Leu Gly Ile Glu
                   965                 970                 975
   Ile Gly Phe Lys Ile Thr Ser Pro Thr Gly Phe Ala Gln Ile Ser Asn
               980                 985                 990
   Leu Glu Ile Val Glu Asp Arg Ser Leu Thr Glu Ala Glu Thr Ile Lys
           995                 1000                1005
   Val Gln Gln Arg Glu Lys Gln Trp Leu Arg Leu Ser Gln Lys Gln Gln
       1010                1015                1020
   Ser Gln Leu Gln Lys Gln Tyr Asp Gln Thr Met Gln Tyr Phe Ala Thr
   1025                1030                1035                1040
   Leu Tyr Thr Thr Ser Asp Gln Thr Glu Leu Lys Asn Thr Val Gln Tyr
                   1045                1050                1055
   Thr Asp Ile Ala Asn Val Gln Val Ile Thr Phe Pro Ser Thr Met Gln
               1060                1065                1070
   Trp Phe Ile Pro Gln Leu Arg Arg Thr Ser Ser Pro Met Ile Glu Glu
           1075                1080                1085
   Leu Val Arg Thr Lys Glu Lys Ala Leu Gln Leu Tyr Pro Thr Asn Val
       1090                1095                1100
   Ile Gln Asn Gly Asn Phe Ser Ser Gly Leu Ser Thr Trp His Val Ile
   1105                1110                1115                1120
   Glu Asn Thr Asn Val Arg Ile Glu Phe Ile Asn Gly Ile Ser Val Leu
                   1125                1130                1135
   His Val Pro Ser Trp Asp Glu Thr Val Ser Gln Thr Ile Thr Leu Pro
               1140                1145                1150
   Pro His Gln Glu Asn Ile Leu Tyr Gln Leu Arg Val Thr Ala Lys Gly
           1155                1160                1165
   Asn Gly Ser Val Ile Leu Gln His Asn Gly Glu Gln Glu Arg Leu Tyr
       1170                1175                1180
   Phe Asp Gln Asn Asn Ser Thr Gly Asn Thr Phe Val Thr Lys Glu Ile
   1185                1190                1195                1200
   Ser Phe Tyr Pro Thr Ala Ser Thr Leu Ser Leu Gln Ile Gln Ser Glu
                   1205                1210                1215
   Gly Thr Asp Phe Tyr Val Lys Thr Ile Asp Leu Phe Val Lys Pro Val
               1220                1225                1230
   Pro Leu Thr
           1235
<210> 5
   <211> 2082
   <212> DNA
   <213> Bacillus thuringiensis
<220>
   <221> CDS
   <222> (1)...(2082)
<400> 5
   atg aca caa aat cat tca ttc tct gat aat aca tcc tca tcg acg ggt    48
   Met Thr Gln Asn His Ser Phe Ser Asp Asn Thr Ser Ser Ser Thr Gly
    1               5                  10                  15
   gta tct act tta gaa tca tct tta att cct tac aat gtg tac gcg aca    96
   Val Ser Thr Leu Glu Ser Ser Leu Ile Pro Tyr Asn Val Tyr Ala Thr
               20                  25                  30
   gat cag ttt aac tct aat aaa aat tgg gaa gat gca ctg aaa aaa tta    144
   Asp Gln Phe Asn Ser Asn Lys Asn Trp Glu Asp Ala Leu Lys Lys Leu
           35                  40                  45
   tta gaa aaa ttt tat tcc ggt gat tta aca cag gat gct att gat att    192
   Leu Glu Lys Phe Tyr Ser Gly Asp Leu Thr Gln Asp Ala Ile Asp Ile
       50                  55                  60
   ttt ctt ggt gac agc ggc ttt gat tac tta tct tta gta aat gtt att    240
   Phe Leu Gly Asp Ser Gly Phe Asp Tyr Leu Ser Leu Val Asn Val Ile
    65                  70                  75                  80
   ttt tct att gca gga tct ttt att cct tat gtg ggt gct ctt gtc cct    288
   Phe Ser Ile Ala Gly Ser Phe Ile Pro Tyr Val Gly Ala Leu Val Pro
                   85                  90                  95
   atc att aat ctt ctt ttt gga tca gag agc aaa cca gat gta ttt gaa    336
   Ile Ile Asn Leu Leu Phe Gly Ser Glu Ser Lys Pro Asp Val Phe Glu
               100                 105                 110
   caa atg aga gca cga att gaa gca tta att cat aag gaa tta tct gca    384
   Gln Met Arg Ala Arg Ile Glu Ala Leu Ile His Lys Glu Leu Ser Ala
           115                 120                 125
   gac cat gtg caa aca tta aaa gca gaa att aag gga ctt aaa gat acg    432
   Asp His Val Gln Thr Leu Lys Ala Glu Ile Lys Gly Leu Lys Asp Thr
       130                 135                 140
   gga gat cta tat caa aaa gat gta aat gct gtt gca gga aga aca aat    480
   Gly Asp Leu Tyr Gln Lys Asp Val Asn Ala Val Ala Gly Arg Thr Asn
   145                 150                 155                 160
   gga cct acc cct cca tca ttt gat agc aat aca gat gct tta aaa gca    528
   Gly Pro Thr Pro Pro Ser Phe Asp Ser Asn Thr Asp Ala Leu Lys Ala
                   165                 170                 175
   gaa ctt cga agt caa atc aca gct aca aac act cta ttt gtg caa cga    576
   Glu Leu Arg Ser Gln Ile Thr Ala Thr Asn Thr Leu Phe Val Gln Arg
               180                 185                 190
   atg cct caa ttt gct ata gag gga tat gaa gag att act cta cct tta    624
   Met Pro Gln Phe Ala Ile Glu Gly Tyr Glu Glu Ile Thr Leu Pro Leu
           195                 200                 205
   cac act atc gct gca agt atg cat ctt ata ttc tta aaa gat gtt tgt    672
   His Thr Ile Ala Ala Ser Met His Leu Ile Phe Leu Lys Asp Val Cys
       210                 215                 220
   gaa cat ggt gct gaa tgg gga att gct aat act aca tta aca aat tat    720
   Glu His Gly Ala Glu Trp Gly Ile Ala Asn Thr Thr Leu Thr Asn Tyr
   225                 230                 235                 240
   caa ggt caa tta caa gat tgt att aga gag tat tca aat aaa gct tat    768
   Gln Gly Gln Leu Gln Asp Cys Ile Arg Glu Tyr Ser Asn Lys Ala Tyr
                   245                 250                 255
   tcg atg ttc aat att ggt tta cag agg gca aaa aat aat gga aac aat    816
   Ser Met Phe Asn Ile Gly Leu Gln Arg Ala Lys Asn Asn Gly Asn Asn
               260                 265                 270
   atg tgg aat aac gta aat aac tat atc cgc aca atg aaa tta aat gct    864
   Met Trp Asn Asn Val Asn Asn Tyr Ile Arg Thr Met Lys Leu Asn Ala
           275                 280                 285
   tta gat act gtt gct caa tgg cct att ctg gat aaa gta aca tac cca    912
   Leu Asp Thr Val Ala Gln Trp Pro Ile Leu Asp Lys Val Thr Tyr Pro
       290                 295                 300
   tta gat aca aca tta caa caa aca cgc ggt ata ttt tca gat cta tca    960
   Leu Asp Thr Thr Leu Gln Gln Thr Arg Gly Ile Phe Ser Asp Leu Ser
   305                 310                 315                 320
   ggt agg ggg ggg aca caa tct aat tat aga tat gat tat gat gct gtt
   1008
   Gly Arg Gly Gly Thr Gln Ser Asn Tyr Arg Tyr Asp Tyr Asp Ala Val
                   325                 330                 335
   caa ggt tat gct cct cct ttt gtc gga ttt gat acc aaa cta aat gtt
   1056
   Gln Gly Tyr Ala Pro Pro Phe Val Gly Phe Asp Thr Lys Leu Asn Val
               340                 345                 350
   gta aac gat ttt ggt tat aaa gat tta acc gca att cag aca ttt aca
   1104
   Val Asn Asp Phe Gly Tyr Lys Asp Leu Thr Ala Ile Gln Thr Phe Thr
           355                 360                 365
   ggt gat cga att gat tca att tgg caa tca ttt aag tat aat tca gga
   1152
   Gly Asp Arg Ile Asp Ser Ile Trp Gln Ser Phe Lys Tyr Asn Ser Gly
       370                 375                 380
   gag cct ttt ctc acg aac tta ggg aat ggt aaa ccc gga aac aac ccc
   1200
   Glu Pro Phe Leu Thr Asn Leu Gly Asn Gly Lys Pro Gly Asn Asn Pro
   385                 390                 395                 400
   gtg att cca aat agc aga gat aat ccg att att tcc gca aaa gga tct
   1248
   Val Ile Pro Asn Ser Arg Asp Asn Pro Ile Ile Ser Ala Lys Gly Ser
                   405                 410                 415
   aga cca tct gca aac tat gtt ggg atg aat ttc caa cga gca aat aaa
   1296
   Arg Pro Ser Ala Asn Tyr Val Gly Met Asn Phe Gln Arg Ala Asn Lys
               420                 425                 430
   act gta gtt tca aat gga tat gta att cct aat gac aat tat aca gta
   1344
   Thr Val Val Ser Asn Gly Tyr Val Ile Pro Asn Asp Asn Tyr Thr Val
           435                 440                 445
   ccc gct ggg cat aaa ctt gga tgg att tca gcc ctg cat gat gaa ttg
   1392
   Pro Ala Gly His Lys Leu Gly Trp Ile Ser Ala Leu His Asp Glu Leu
       450                 455                 460
   gat aat gca aat aat gcg gat cta gtt gta tcg gtt tgg gtg aaa aat
   1440
   Asp Asn Ala Asn Asn Ala Asp Leu Val Val Ser Val Trp Val Lys Asn
   465                 470                 475                 480
   gat atc ttc cag gaa aat att atc ggt tcc ata aaa aca gtt act act
   1488
   Asp Ile Phe Gln Glu Asn Ile Ile Gly Ser Ile Lys Thr Val Thr Thr
                   485                 490                 495
   gat gat gga acc aca gaa aat aga caa caa att ata ggg atc ccg gca
   1536
   Asp Asp Gly Thr Thr Glu Asn Arg Gln Gln Ile Ile Gly Ile Pro Ala
               500                 505                 510
   gat aaa cat atg aca aga agt aca aag cga atg gaa ctg gaa ttt atc
   1584
   Asp Lys His Met Thr Arg Ser Thr Lys Arg Met Glu Leu Glu Phe Ile
           515                 520                 525
   aat ggt aca aat ggg tca atg agc tta tct agt act aat gat caa ttg
   1632
   Asn Gly Thr Asn Gly Ser Met Ser Leu Ser Ser Thr Asn Asp Gln Leu
       530                 535                 540
   tat tat acg att aat cct ata gtt agc cag aga tat caa att cgg tat
   1680
   Tyr Tyr Thr Ile Asn Pro Ile Val Ser Gln Arg Tyr Gln Ile Arg Tyr
   545                 550                 555                 560
   cgc gta gca aca act tca gca gaa tct tta gac cta tgg atc gat ggt
   1728
   Arg Val Ala Thr Thr Ser Ala Glu Ser Leu Asp Leu Trp Ile Asp Gly
                   565                 570                 575
   tat aaa cgc gga aca acc ccg tta cca aat aca agt agc aca tca acg
   1776
   Tyr Lys Arg Gly Thr Thr Pro Leu Pro Asn Thr Ser Ser Thr Ser Thr
               580                 585                 590
   caa aca caa aaa gtg ata att caa ggg tta caa gga aaa tat caa tta
   1824
   Gln Thr Gln Lys Val Ile Ile Gln Gly Leu Gln Gly Lys Tyr Gln Leu
           595                 600                 605
   att aat gga cca act ctt gat ttg aca gca ggt tcc cat act ttt ggt
   1872
   Ile Asn Gly Pro Thr Leu Asp Leu Thr Ala Gly Ser His Thr Phe Gly
       610                 615                 620
   att atg tta aca gca aat gct tct caa aat gta ttt att gat cgc att
   1920
   Ile Met Leu Thr Ala Asn Ala Ser Gln Asn Val Phe Ile Asp Arg Ile
   625                 630                 635                 640
   gaa ttt gtt cct ata gct aca aca gaa cct gtc aca ata ccc aat aca
   1968
   Glu Phe Val Pro Ile Ala Thr Thr Glu Pro Val Thr Ile Pro Asn Thr
                   645                 650                 655
   cct att aaa act tat aca aat cca cca aat cct caa caa gta ctt tgg
   2016
   Pro Ile Lys Thr Tyr Thr Asn Pro Pro Asn Pro Gln Gln Val Leu Trp
               660                 665                 670
   act gct cag cca ggt att ttg ggt gat ata gta aat tat cat atc aac
   2064
   Thr Ala Gln Pro Gly Ile Leu Gly Asp Ile Val Asn Tyr His Ile Asn
           675                 680                 685
   ctt tat aac cat tta taa
   2082
   Leu Tyr Asn His Leu  *
       690
<210> 6
   <211> 693
   <212> PRT
   <213> Bacillus thuringiensis
<400> 6
   Met Thr Gln Asn His Ser Phe Ser Asp Asn Thr Ser Ser Ser Thr Gly
   1               5                   10                  15
   Val Ser Thr Leu Glu Ser Ser Leu Ile Pro Tyr Asn Val Tyr Ala Thr
               20                  25                  30
   Asp Gln Phe Asn Ser Asn Lys Asn Trp Glu Asp Ala Leu Lys Lys Leu
           35                  40                  45
   Leu Glu Lys Phe Tyr Ser Gly Asp Leu Thr Gln Asp Ala Ile Asp Ile
       50                  55                  60
   Phe Leu Gly Asp Ser Gly Phe Asp Tyr Leu Ser Leu Val Asn Val Ile
   65                  70                  75                  80
   Phe Ser Ile Ala Gly Ser Phe Ile Pro Tyr Val Gly Ala Leu Val Pro
                   85                  90                  95
   Ile Ile Asn Leu Leu Phe Gly Ser Glu Ser Lys Pro Asp Val Phe Glu
               100                 105                 110
   Gln Met Arg Ala Arg Ile Glu Ala Leu Ile His Lys Glu Leu Ser Ala
           115                 120                 125
   Asp His Val Gln Thr Leu Lys Ala Glu Ile Lys Gly Leu Lys Asp Thr
       130                 135                 140
   Gly Asp Leu Tyr Gln Lys Asp Val Asn Ala Val Ala Gly Arg Thr Asn
   145                 150                 155                 160
   Gly Pro Thr Pro Pro Ser Phe Asp Ser Asn Thr Asp Ala Leu Lys Ala
                   165                 170                 175
   Glu Leu Arg Ser Gln Ile Thr Ala Thr Asn Thr Leu Phe Val Gln Arg
               180                 185                 190
   Met Pro Gln Phe Ala Ile Glu Gly Tyr Glu Glu Ile Thr Leu Pro Leu
           195                 200                 205
   His Thr Ile Ala Ala Ser Met His Leu Ile Phe Leu Lys Asp Val Cys
       210                 215                 220
   Glu His Gly Ala Glu Trp Gly Ile Ala Asn Thr Thr Leu Thr Asn Tyr
   225                 230                 235                 240
   Gln Gly Gln Leu Gln Asp Cys Ile Arg Glu Tyr Ser Asn Lys Ala Tyr
                   245                 250                 255
   Ser Met Phe Asn Ile Gly Leu Gln Arg Ala Lys Asn Asn Gly Asn Asn
               260                 265                 270
   Met Trp Asn Asn Val Asn Asn Tyr Ile Arg Thr Met Lys Leu Asn Ala
           275                 280                 285
   Leu Asp Thr Val Ala Gln Trp Pro Ile Leu Asp Lys Val Thr Tyr Pro
       290                 295                 300
   Leu Asp Thr Thr Leu Gln Gln Thr Arg Gly Ile Phe Ser Asp Leu Ser
   305                 310                 315                 320
   Gly Arg Gly Gly Thr Gln Ser Asn Tyr Arg Tyr Asp Tyr Asp Ala Val
                   325                 330                 335
   Gln Gly Tyr Ala Pro Pro Phe Val Gly Phe Asp Thr Lys Leu Asn Val
               340                 345                 350
   Val Asn Asp Phe Gly Tyr Lys Asp Leu Thr Ala Ile Gln Thr Phe Thr
           355                 360                 365
   Gly Asp Arg Ile Asp Ser Ile Trp Gln Ser Phe Lys Tyr Asn Ser Gly
       370                 375                 380
   Glu Pro Phe Leu Thr Asn Leu Gly Asn Gly Lys Pro Gly Asn Asn Pro
   385                 390                 395                 400
   Val Ile Pro Asn Ser Arg Asp Asn Pro Ile Ile Ser Ala Lys Gly Ser
                   405                 410                 415
   Arg Pro Ser Ala Asn Tyr Val Gly Met Asn Phe Gln Arg Ala Asn Lys
               420                 425                 430
   Thr Val Val Ser Asn Gly Tyr Val Ile Pro Asn Asp Asn Tyr Thr Val
           435                 440                 445
   Pro Ala Gly His Lys Leu Gly Trp Ile Ser Ala Leu His Asp Glu Leu
       450                 455                 460
   Asp Asn Ala Asn Asn Ala Asp Leu Val Val Ser Val Trp Val Lys Asn
   465                 470                 475                 480
   Asp Ile Phe Gln Glu Asn Ile Ile Gly Ser Ile Lys Thr Val Thr Thr
                   485                 490                 495
   Asp Asp Gly Thr Thr Glu Asn Arg Gln Gln Ile Ile Gly Ile Pro Ala
               500                 505                 510
   Asp Lys His Met Thr Arg Ser Thr Lys Arg Met Glu Leu Glu Phe Ile
           515                 520                 525
   Asn Gly Thr Asn Gly Ser Met Ser Leu Ser Ser Thr Asn Asp Gln Leu
       530                 535                 540
   Tyr Tyr Thr Ile Asn Pro Ile Val Ser Gln Arg Tyr Gln Ile Arg Tyr
   545                 550                 555                 560
   Arg Val Ala Thr Thr Ser Ala Glu Ser Leu Asp Leu Trp Ile Asp Gly
                   565                 570                 575
   Tyr Lys Arg Gly Thr Thr Pro Leu Pro Asn Thr Ser Ser Thr Ser Thr
               580                 585                 590
   Gln Thr Gln Lys Val Ile Ile Gln Gly Leu Gln Gly Lys Tyr Gln Leu
           595                 600                 605
   Ile Asn Gly Pro Thr Leu Asp Leu Thr Ala Gly Ser His Thr Phe Gly
       610                 615                 620
   Ile Met Leu Thr Ala Asn Ala Ser Gln Asn Val Phe Ile Asp Arg Ile
   625                 630                 635                 640
   Glu Phe Val Pro Ile Ala Thr Thr Glu Pro Val Thr Ile Pro Asn Thr
                   645                 650                 655
   Pro Ile Lys Thr Tyr Thr Asn Pro Pro Asn Pro Gln Gln Val Leu Trp
               660                 665                 670
   Thr Ala Gln Pro Gly Ile Leu Gly Asp Ile Val Asn Tyr His Ile Asn
           675                 680                 685
   Leu Tyr Asn His Leu
       690
<210> 7
   <211> 1178
   <212> PRT
   <213> Bacillus thuringiensis
<400> 7
<210> 8
   <211> 1228
   <212> PRT
   <213> Bacillus thuringiensis
<400> 8
   Met Thr Ser Asn Arg Lys Asn Glu Asn Glu Ile Ile Asn Ala Val Ser
   1 5 10 15
   Asn His Ser Ala Gln Met Asp Leu Leu Pro Asp Ala Arg Ile Glu Asp
   20 25 30
   Ser Leu Cys Ile Ala Glu Gly Asn Asn Ile Asp Pro Phe Val Ser Ala
   35 40 45
   Ser Thr Val Gln Thr Gly Ile Asn Ile Ala Gly Arg Ile Leu Gly Val
   50 55 60
   Leu Gly Val Pro Phe Ala Gly Gln Leu Ala Ser Phe Tyr Ser Phe Leu
   65 70 75 80
   Val Gly Glu Leu Trp Pro Arg Gly Arg Asp Gln Trp Glu Ile Phe Leu
   85 90 95
   Glu His Val Glu Gln Leu Ile Asn Gln Gln Ile Thr Glu Asn Ala Arg
   100 105 110
   Asn Thr Ala Leu Ala Arg Leu Gln Gly Leu Gly Asp Ser Phe Arg Ala
   115 120 125
   Tyr Gln Gln Ser Leu Glu Asp Trp Leu Glu Asn Arg Asp Asp Ala Arg
   130 135 140
   Thr Arg Ser Val Leu Tyr Thr Gln Tyr Ile Ala Leu Glu Leu Asp Phe
   145 150 155 160
   Leu Asn Ala Met Pro Leu Phe Ala Ile Arg Asn Gln Glu Val Pro Leu
   165 170 175
   Leu Met Val Tyr Ala Gln Ala Ala Asn Leu His Leu Leu Leu Leu Arg
   180 185 190
   Asp Ala Ser Leu Phe Gly Ser Glu Phe Gly Leu Thr Ser Gln Glu Ile
   195 200 205
   Gln Arg Tyr Tyr Glu Arg Gln Val Glu Arg Thr Arg Asp Tyr Ser Asp
   210 215 220
   Tyr Cys Val Glu Trp Tyr Asn Thr Gly Leu Asn Ser Leu Arg Gly Thr
   225 230 235 240
   Asn Ala Ala Ser Trp Val Arg Tyr Asn Gln Phe Arg Arg Asp Leu Thr
   245 250 255
   Leu Gly Val Leu Asp Leu Val Ala Leu Phe Pro Ser Tyr Asp Thr Arg
   260 265 270
   Thr Tyr Pro Ile Asn Thr Ser Ala Gln Leu Thr Arg Glu Val Tyr Thr
   275 280 285
   Asp Ala Ile Gly Ala Thr Gly Val Asn Met Ala Ser Met Asn Trp Tyr
   290 295 300
   Asn Asn Asn Ala Pro Ser Phe Ser Ala Ile Glu Ala Ala Ala Ile Arg
   305 310 315 320
   Ser Pro His Leu Leu Asp Phe Leu Glu Gln Leu Thr Ile Phe Ser Ala
   325 330 335
   Ser Ser Arg Trp Ser Asn Thr Arg His Met Thr Tyr Trp Arg Gly His
   340 345 350
   Thr Ile Gln Ser Arg Pro Ile Gly Gly Gly Leu Asn Thr Ser Thr His
   355 360 365
   Gly Ala Thr Asn Thr Ser Ile Asn Pro Val Thr Leu Arg Phe Ala Ser
   370 375 380
   Arg Asp Val Tyr Arg Thr Glu Ser Tyr Ala Gly Val Leu Leu Trp Gly
   385 390 395 400
   Ile Tyr Leu Glu Pro Ile His Gly Val Pro Thr Val Arg Phe Asn Phe
   405 410 415
   Thr Asn Pro Gln Asn Ile Ser Asp Arg Gly Thr Ala Asn Tyr Ser Gln
   420 425 430
   Pro Tyr Glu Ser Pro Gly Leu Gln Leu Lys Asp Ser Glu Thr Glu Leu
   435 440 445
   Pro Pro Glu Thr Thr Glu Arg Pro Asn Tyr Glu Ser Tyr Ser His Arg
   450 455 460
   Leu Ser His Ile Gly Ile Ile Leu Gln Ser Arg Val Asn Val Pro Val
   465 470 475 480
   Tyr Ser Trp Thr His Arg Ser Ala Asp Arg Thr Asn Thr Ile Gly Pro
   485 490 495
   Asn Arg Ile Thr Gln Ile Pro Met Val Lys Ala Ser Glu Leu Pro Gln
   500 505 510
   Gly Thr Thr Val Val Arg Gly Pro Gly Phe Thr Gly Gly Asp Ile Leu
   515 520 525
   Arg Arg Thr Asn Thr Gly Gly Phe Gly Pro Ile Arg Val Thr Val Asn
   530 535 540
   Gly Pro Leu Thr Gln Arg Tyr Arg Ile Gly Phe Arg Tyr Ala Ser Thr
   545 550 555 560
   Val Asp Phe Asp Phe Phe Val Ser Arg Gly Gly Thr Thr Val Asn Asn
   565 570 575
   Phe Arg Phe Leu Arg Thr Met Asn Ser Gly Asp Glu Leu Lys Tyr Gly
   580 585 590
   Asn Phe Val Arg Arg Ala Phe Thr Thr Pro Phe Thr Phe Thr Gln Ile
   595 600 605
   Gln Asp Ile Ile Arg Thr Ser Ile Gln Gly Leu Ser Gly Asn Gly Glu
   610 615 620
   Val Tyr Ile Asp Lys Ile Glu Ile Ile Pro Val Thr Ala Thr Phe Glu
   625 630 635 640
   Ala Glu Tyr Asp Leu Glu Arg Ala Gln Glu Ala Val Asn Ala Leu Phe
   645 650 655
   Thr Asn Thr Asn Pro Arg Arg Leu Lys Thr Asp Val Thr Asp Tyr His
   660 665 670
   Ile Asp Gln Val Ser Asn Leu Val Ala Cys Leu Ser Asp Glu Phe Cys
   675 680 685
   Leu Asp Glu Lys Arg Glu Leu Leu Glu Lys Val Lys Tyr Ala Lys Arg
   690 695 700
   Leu Ser Asp Glu Arg Asn Leu Leu Gln Asp Pro Asn Phe Thr Ser Ile
   705 710 715 720
   Asn Lys Gln Pro Asp Phe Ile Ser Thr Asn Glu Gln Ser Asn Phe Thr
   725 730 735
   Ser Ile His Glu Gln Ser Glu His Gly Trp Trp Gly Ser Glu Asn Ile
   740 745 750
   Thr Ile Gln Glu Gly Asn Asp Val Phe Lys Glu Asn Tyr Val Thr Leu
   755 760 765
   Pro Gly Thr Phe Asn Glu Cys Tyr Pro Thr Tyr Leu Tyr Gln Lys Ile
   770 775 780
   Gly Glu Ser Glu Leu Lys Ala Tyr Thr Arg Tyr Gln Leu Arg Gly Tyr
   785 790 795 800
   Ile Glu Asp Ser Gln Asp Leu Glu Ile Tyr Leu Ile Arg Tyr Asn Ala
   805 810 815
   Lys His Glu Thr Leu Asp Val Pro Gly Thr Glu Ser Leu Trp Pro Leu
   820 825 830
   Ser Val Glu Ser Pro Ile Gly Arg Cys Gly Glu Pro Asn Arg Cys Ala
   835 840 845
   Pro His Phe Glu Trp Asn Pro Asp Leu Asp Cys Ser Cys Arg Asp Gly
   850 855 860
   Glu Lys Cys Ala His His Ser His His Phe Ser Leu Asp Ile Asp Val
   865 870 875 880
   Gly Cys Thr Asp Leu His Glu Asn Leu Gly Val Trp Val Val Phe Lys
   885 890 895
   Ile Lys Thr Gln Glu Gly His Ala Arg Leu Gly Asn Leu Glu Phe Ile
   900 905 910
   Glu Glu Lys Pro Leu Leu Gly Glu Ala Leu Ser Arg Val Lys Arg Ala
   915 920 925
   Glu Lys Lys Trp Arg Asp Lys Arg Glu Lys Leu Gln Leu Glu Thr Lys
   930 935 940
   Arg Val Tyr Thr Glu Ala Lys Glu Ala Val Asp Ala Leu Phe Val Asp
   945 950 955 960
   Ser Gln Tyr Asp Arg Leu Gln Ala Asp Thr Asn Ile Gly Met Ile His
   965 970 975
   Ala Ala Asp Lys Leu Val His Arg Ile Arg Glu Ala Tyr Leu Ser Glu
   980 985 990
   Leu Pro Val Ile Pro Gly Val Asn Ala Glu Ile Phe Glu Glu Leu Glu
   995 1000 1005
   Gly His Ile Ile Thr Ala Ile Ser Leu Tyr Asp Ala Arg Asn Val Val
   1010 1015 1020
   Lys Asn Gly Asp Phe Asn Asn Gly Leu Thr Cys Trp Asn Val Lys Gly
   1025 1030 1035 1040
   His Val Asp Val Gln Gln Ser His His Arg Ser Asp Leu Val Ile Pro
   1045 1050 1055
   Glu Trp Glu Ala Glu Val Ser Gln Ala Val Arg Val Cys Pro Gly Cys
   1060 1065 1070
   Gly Tyr Ile Leu Arg Val Thr Ala Tyr Lys Glu Gly Tyr Gly Glu Gly
   1075 1080 1085
   Cys Val Thr Ile His Glu Ile Glu Asn Asn Thr Asp Glu Leu Lys Phe
   1090 1095 1100
   Lys Asn Arg Glu Glu Glu Glu Val Tyr Pro Thr Asp Thr Gly Thr Cys
   1105 1110 1115 1120
   Asn Asp Tyr Thr Ala His Gln Gly Thr Ala Gly Cys Ala Asp Ala Cys
   1125 1130 1135
   Asn Ser Arg Asn Ala Gly Tyr Glu Asp Ala Tyr Glu Val Asp Thr Thr
   1140 1145 1150
   Ala Ser Val Asn Tyr Lys Pro Thr Tyr Glu Glu Glu Thr Tyr Thr Asp
   1155 1160 1165
   Val Arg Arg Asp Asn His Cys Glu Tyr Asp Arg Gly Tyr Val Asn Tyr
   1170 1175 1180
   Pro Pro Val Pro Ala Gly Tyr Val Thr Lys Glu Leu Glu Tyr Phe Pro
   1185 1190 1195 1200
   Glu Thr Asp Thr Val Trp Ile Glu Ile Gly Glu Thr Glu Gly Lys Phe
   1205 1210 1215
   Ile Val Asp Ser Val Glu Leu Leu Leu Met Glu Glu
   1220 1225
<210> 9
   <211> 1189
   <212> PRT
   <213> Bacillus thuringiensis
<400> 9
   Met Glu Glu Asn Asn Gln Asn Gln Cys Ile Pro Tyr Asn Cys Leu Ser
    1               5                  10                  15
   Asn Pro Glu Glu Val Leu Leu Asp Gly Glu Arg Ile Ser Thr Gly Asn
               20                  25                  30
   Ser Ser Ile Asp Ile Ser Leu Ser Leu Val Gln Phe Leu Val Ser Asn
           35                  40                  45
   Phe Val Pro Gly Gly Gly Phe Leu Val Gly Leu Ile Asp Phe Val Trp
       50                  55                  60
   Gly Ile Val Gly Pro Ser Gln Trp Asp Ala Phe Leu Val Gln Ile Glu
   65                  70                  75                  80
   Gln Leu Ile Asn Glu Arg Ile Ala Glu Phe Ala Arg Asn Ala Ala Ile
                   85                  90                  95
   Ala Asn Leu Glu Gly Leu Gly Asn Asn Phe Asn Ile Tyr Val Glu Ala
               100                 105                 110
   Phe Lys Glu Trp Glu Glu Asp Pro Asn Asn Pro Ala Thr Arg Thr Arg
           115                 120                 125
   Val Ile Asp Arg Phe Arg Ile Leu Asp Gly Leu Leu Glu Arg Asp Ile
       130                 135                 140
   Pro Ser Phe Arg Ile Ser Gly Phe Glu Val Pro Leu Leu Ser Val Tyr
   145                 150                 155                 160
   Ala Gln Ala Ala Asn Leu His Leu Ala Ile Leu Arg Asp Ser Val Ile
                   165                 170                 175
   Phe Gly Glu Arg Trp Gly Leu Thr Thr Ile Asn Val Asn Glu Asn Tyr
               180                 185                 190
   Asn Arg Leu Ile Arg His Ile Asp Glu Tyr Ala Asp His Cys Ala Asn
           195                 200                 205
   Thr Tyr Asn Arg Gly Leu Asn Asn Leu Pro Lys Ser Thr Tyr Gln Asp
       210                 215                 220
   Trp Ile Thr Tyr Asn Arg Leu Arg Arg Asp Leu Thr Leu Thr Val Leu
   225                 230                 235                 240
   Asp Ile Ala Ala Phe Phe Pro Asn Tyr Asp Asn Arg Arg Tyr Pro Ile
                   245                 250                 255
   Gln Pro Val Gly Gln Leu Thr Arg Glu Val Tyr Thr Asp Pro Leu Ile
               260                 265                 270
   Asn Phe Asn Pro Gln Leu Gln Ser Val Ala Gln Leu Pro Thr Phe Asn
           275                 280                 285
   Val Met Glu Ser Ser Ala Ile Arg Asn Pro His Leu Phe Asp Ile Leu
       290                 295                 300
   Asn Asn Leu Thr Ile Phe Thr Asp Trp Phe Ser Val Gly Arg Asn Phe
   305                 310                 315                 320
   Tyr Trp Gly Gly His Arg Val Ile Ser Ser Leu Ile Gly Gly Gly Asn
                   325                 330                 335
   Ile Thr Ser Pro Ile Tyr Gly Arg Glu Ala Asn Gln Glu Pro Pro Arg
               340                 345                 350
   Ser Phe Thr Phe Asn Gly Pro Val Phe Arg Thr Leu Ser Asn Pro Thr
           355                 360                 365
   Leu Arg Leu Leu Gln Gln Pro Trp Pro Ala Pro Pro Phe Asn Leu Arg
       370                 375                 380
   Gly Val Glu Gly Val Glu Phe Ser Thr Pro Thr Asn Ser Phe Thr Tyr
   385                 390                 395                 400
   Arg Gly Arg Gly Thr Val Asp Ser Leu Thr Glu Leu Pro Pro Glu Asp
                   405                 410                 415
   Asn Ser Val Pro Pro Arg Glu Gly Tyr Ser His Arg Leu Cys His Ala
               420                 425                 430
   Thr Phe Val Gln Arg Ser Gly Thr Pro Phe Leu Thr Thr Gly Val Val
           435                 440                 445
   Phe Ser Trp Thr His Arg Ser Ala Thr Leu Thr Asn Thr Ile Asp Pro
       450                 455                 460
   Glu Arg Ile Asn Gln Ile Pro Leu Val Lys Gly Phe Arg Val Trp Gly
   465                 470                 475                 480
   Gly Thr Ser Val Ile Thr Gly Pro Gly Phe Thr Gly Gly Asp Ile Leu
                   485                 490                 495
   Arg Arg Asn Thr Phe Gly Asp Phe Val Ser Leu Gln Val Asn Ile Asn
               500                 505                 510
   Ser Pro Ile Thr Gln Arg Tyr Arg Leu Arg Phe Arg Tyr Ala Ser Ser
           515                 520                 525
   Arg Asp Ala Arg Val Ile Val Leu Thr Gly Ala Ala Ser Thr Gly Val
       530                 535                 540
   Gly Gly Gln Val Ser Val Asn Met Pro Leu Gln Lys Thr Met Glu Ile
   545                 550                 555                 560
   Gly Glu Asn Leu Thr Ser Arg Thr Phe Arg Tyr Thr Asp Phe Ser Asn
                   565                 570                 575
   Pro Phe Ser Phe Arg Ala Asn Pro Asp Ile Ile Gly Ile Ser Glu Gln
               580                 585                 590
   Pro Leu Phe Gly Ala Gly Ser Ile Ser Ser Gly Glu Leu Tyr Ile Asp
           595                 600                 605
   Lys Ile Glu Ile Ile Leu Ala Asp Ala Thr Phe Glu Ala Glu Ser Asp
       610                 615                 620
   Leu Glu Arg Ala Gln Lys Ala Val Asn Ala Leu Phe Thr Ser Ser Asn
   625                 630                 635                 640
   Gln Ile Gly Leu Lys Thr Asp Val Thr Asp Tyr His Ile Asp Gln Val
                   645                 650                 655
   Ser Asn Leu Val Asp Cys Leu Ser Asp Glu Phe Cys Leu Asp Glu Lys
               660                 665                 670
   Arg Glu Leu Ser Glu Lys Val Lys His Ala Lys Arg Leu Ser Asp Glu
           675                 680                 685
   Arg Asn Leu Leu Gln Asp Pro Asn Phe Arg Gly Ile Asn Arg Gln Pro
       690                 695                 700
   Asp Arg Gly Trp Arg Gly Ser Thr Asp Ile Thr Ile Gln Gly Gly Asp
   705                 710                 715                 720
   Asp Val Phe Lys Glu Asn Tyr Val Thr Leu Pro Gly Thr Val Asp Glu
                   725                 730                 735
   Cys Tyr Pro Thr Tyr Leu Tyr Gln Lys Ile Asp Glu Ser Lys Leu Lys
               740                 745                 750
   Ala Tyr Thr Arg Tyr Glu Leu Arg Gly Tyr Ile Glu Asp Ser Gln Asp
           755                 760                 765
   Leu Glu Ile Tyr Leu Ile Arg Tyr Asn Ala Lys His Glu Ile Val Asn
       770                 775                 780
   Val Pro Gly Thr Gly Ser Leu Trp Pro Leu Ser Ala Gln Ser Pro Ile
   785                 790                 795                 800
   Gly Lys Cys Gly Glu Pro Asn Arg Cys Ala Pro His Leu Glu Trp Asn
                   805                 810                 815
   Pro Asp Leu Asp Cys Ser Cys Arg Asp Gly Glu Lys Cys Ala His His
               820                 825                 830
   Ser His His Phe Thr Leu Asp Ile Asp Val Gly Cys Thr Asp Leu Asn
           835                 840                 845
   Glu Asp Leu Gly Val Trp Val Ile Phe Lys Ile Lys Thr Gln Asp Gly
       850                 855                 860
   His Ala Arg Leu Gly Asn Leu Glu Phe Leu Glu Glu Lys Pro Leu Leu
   865                 870                 875                 880
   Gly Glu Ala Leu Ala Arg Val Lys Arg Ala Glu Lys Lys Trp Arg Asp
                   885                 890                 895
   Lys Arg Glu Lys Leu Gln Leu Glu Thr Asn Ile Val Tyr Lys Glu Ala
               900                 905                 910
   Lys Glu Ser Val Asp Ala Leu Phe Val Asn Ser Gln Tyr Asp Arg Leu
           915                 920                 925
   Gln Val Asp Thr Asn Ile Ala Met Ile His Ala Ala Asp Lys Arg Val
       930                 935                 940
   His Arg Ile Arg Glu Ala Tyr Leu Pro Glu Leu Ser Val Ile Pro Gly
   945                 950                 955                 960
   Val Asn Ala Ala Ile Phe Glu Glu Leu Glu Gly Arg Ile Phe Thr Ala
                   965                 970                 975
   Tyr Ser Leu Tyr Asp Ala Arg Asn Val Ile Lys Asn Gly Asp Phe Asn
               980                 985                 990
   Asn Gly Leu Leu Cys Trp Asn Val Lys Gly His Val Asp Val Glu Glu
           995                 1000                1005
   Gln Asn Asn His Arg Ser Val Leu Val Ile Pro Glu Trp Glu Ala Glu
   1010 1015 1020
   Val Ser Gln Glu Val Arg Val Cys Pro Gly Arg Gly Tyr Ile Leu Arg
   1025 1030 1035 1040
   Val Thr Ala Tyr Lys Glu Gly Tyr Gly Glu Gly Cys Val Thr Ile His
   1045 1050 1055
   Glu Ile Glu Asp Asn Thr Asp Glu Leu Lys Phe Ser Asn Cys Val Glu
   1060 1065 1070
   Glu Glu Val Tyr Pro Asn Asn Thr Val Thr Cys Asn Asn Tyr Thr Gly
   1075 1080 1085
   Thr Gln Glu Glu Tyr Glu Gly Thr Tyr Thr Ser Arg Asn Gln Gly Tyr
   1090 1095 1100
   Asp Glu Ala Tyr Gly Asn Asn Pro Ser Val Pro Ala Asp Tyr Ala Ser
   1105 1110 1115 1120
   Val Tyr Glu Glu Lys Ser Tyr Thr Asp Gly Arg Arg Glu Asn Pro Cys
   1125 1130 1135
   Glu Ser Asn Arg Gly Tyr Gly Asp Tyr Thr Pro Leu Pro Ala Gly Tyr
   1140 1145 1150
   Val Thr Lys Asp Leu Glu Tyr Phe Pro Glu Thr Asp Lys Val Trp Ile
   1155 1160 1165
   Glu Ile Gly Glu Thr Glu Gly Thr Phe Ile Val Asp Ser Val Glu Leu
   1170 1175 1180
   Leu Leu Met Glu Glu
   1185
<210> 10
   <211> 1385
   <212> PRT
   <213> Bacillus thuringiensis
<400> 10
   Met Ala Ile Leu Asn Glu Leu Tyr Pro Ser Val Pro Tyr Asn Val Leu
   1               5                   10                  15
   Ala Tyr Thr Pro Pro Ser Phe Leu Pro Asp Ala Gly Thr Gln Ala Thr
               20                  25                  30
   Pro Ala Asp Leu Thr Ala Tyr Glu Gln Leu Leu Lys Asn Leu Glu Lys
           35                  40                  45
   Gly Ile Asn Ala Gly Thr Tyr Ser Lys Ala Ile Ala Asp Val Leu Lys
       50                  55                  60
   Gly Ile Phe Ile Asp Asp Thr Ile Asn Tyr Gln Thr Tyr Val Asn Ile
   65                  70                  75                  80
   Gly Leu Ser Leu Ile Thr Leu Ala Val Pro Glu Ile Gly Ile Phe Thr
                   85                  90                  95
   Pro Phe Ile Gly Leu Phe Phe Ala Ala Leu Asn Lys His Asp Ala Pro
               100                 105                 110
   Pro Pro Pro Asn Ala Lys Asp Ile Phe Glu Ala Met Lys Pro Ala Ile
           115                 120                 125
   Gln Glu Met Ile Asp Arg Thr Leu Thr Ala Asp Glu Gln Thr Phe Leu
       130                 135                 140
   Asn Gly Glu Ile Ser Gly Leu Gln Asn Leu Ala Ala Arg Tyr Gln Ser
   145                 150                 155                 160
   Thr Met Asp Asp Ile Gln Ser His Gly Gly Phe Asn Lys Val Asp Ser
                   165                 170                 175
   Gly Leu Ile Lys Lys Phe Thr Asp Glu Val Leu Ser Leu Asn Ser Phe
               180                 185                 190
   Tyr Thr Asp Arg Leu Pro Val Phe Ile Thr Asp Asn Thr Ala Asp Arg
           195                 200                 205
   Thr Leu Leu Gly Leu Pro Tyr Tyr Ala Ile Leu Ala Ser Met His Leu
       210                 215                 220
   Met Leu Leu Arg Asp Ile Ile Thr Lys Gly Pro Thr Trp Asp Ser Lys
   225                 230                 235                 240
   Ile Asn Phe Thr Pro Asp Ala Ile Asp Ser Phe Lys Thr Asp Ile Lys
                   245                 250                 255
   Asn Asn Ile Lys Leu Tyr Ser Lys Thr Ile Tyr Asp Val Phe Gln Lys
               260                 265                 270
   Gly Leu Ala Ser Tyr Gly Thr Pro Ser Asp Leu Glu Ser Phe Ala Lys
           275                 280                 285
   Lys Gln Lys Tyr Ile Glu Ile Met Thr Thr His Cys Leu Asp Phe Ala
       290                 295                 300
   Arg Leu Phe Pro Thr Phe Asp Pro Asp Leu Tyr Pro Thr Gly Ser Gly
   305                 310                 315                 320
   Asp Ile Ser Leu Gln Lys Thr Arg Arg Ile Leu Ser Pro Phe Ile Pro
                   325                 330                 335
   Ile Arg Thr Ala Asp Gly Leu Thr Leu Asn Asn Thr Ser Ile Asp Thr
               340                 345                 350
   Ser Asn Trp Pro Asn Tyr Glu Asn Gly Asn Gly Ala Phe Pro Asn Pro
           355                 360                 365
   Lys Glu Arg Ile Leu Lys Gln Phe Lys Leu Tyr Pro Ser Trp Arg Ala
       370                 375                 380
   Gly Gln Tyr Gly Gly Leu Leu Gln Pro Tyr Leu Trp Ala Ile Glu Val
   385                 390                 395                 400
   Gln Asp Ser Val Glu Thr Arg Leu Tyr Gly Gln Leu Pro Ala Val Asp
                   405                 410                 415
   Pro Gln Ala Gly Pro Asn Tyr Val Ser Ile Asp Ser Ser Asn Pro Ile
               420                 425                 430
   Ile Gln Ile Asn Met Asp Thr Trp Lys Thr Pro Pro Gln Gly Ala Ser
           435                 440                 445
   Gly Trp Asn Thr Asn Leu Met Arg Gly Ser Val Ser Gly Leu Ser Phe
       450                 455                 460
   Leu Gln Arg Asp Gly Thr Arg Leu Ser Ala Gly Met Gly Gly Gly Phe
   465                 470                 475                 480
   Ala Asp Thr Ile Tyr Ser Leu Pro Ala Thr His Tyr Leu Ser Tyr Leu
                   485                 490                 495
   Tyr Gly Thr Pro Tyr Gln Thr Ser Asp Asn Tyr Ser Gly His Val Gly
               500                 505                 510
   Ala Leu Val Gly Val Ser Thr Pro Gln Glu Ala Thr Leu Pro Asn Ile
           515                 520                 525
   Ile Gly Gln Pro Asp Glu Gln Gly Asn Val Ser Thr Met Gly Phe Pro
       530                 535                 540
   Phe Glu Lys Ala Ser Tyr Gly Gly Thr Val Val Lys Glu Trp Leu Asn
   545                 550                 555                 560
   Gly Ala Asn Ala Met Lys Leu Ser Pro Gly Gln Ser Ile Gly Ile Pro
                   565                 570                 575
   Ile Thr Asn Val Thr Ser Gly Glu Tyr Gln Ile Arg Cys Arg Tyr Ala
               580                 585                 590
   Ser Asn Asp Asn Thr Asn Val Phe Phe Asn Val Asp Thr Gly Gly Ala
           595                 600                 605
   Asn Pro Ile Phe Gln Gln Ile Asn Phe Ala Ser Thr Val Asp Asn Asn
       610                 615                 620
   Thr Gly Val Gln Gly Ala Asn Gly Val Tyr Val Val Lys Ser Ile Ala
   625                 630                 635                 640
   Thr Thr Asp Asn Ser Phe Thr Glu Ile Pro Ala Lys Thr Ile Asn Val
                   645                 650                 655
   His Leu Thr Asn Gln Gly Ser Ser Asp Val Phe Leu Asp Arg Ile Glu
               660                 665                 670
   Phe Ile Pro Phe Ser Leu Pro Leu Ile Tyr His Gly Ser Tyr Asn Thr
           675                 680                 685
   Ser Ser Gly Ala Asp Asp Val Leu Trp Ser Ser Ser Asn Met Asn Tyr
       690                 695                 700
   Tyr Asp Ile Ile Val Asn Gly Gln Ala Asn Ser Ser Ser Ile Ala Ser
   705                 710                 715                 720
   Ser Met His Leu Leu Asn Lys Gly Lys Val Ile Lys Thr Ile Asp Ile
                   725                 730                 735
   Pro Gly His Ser Glu Thr Phe Phe Ala Thr Phe Pro Val Pro Glu Gly
               740                 745                 750
   Phe Asn Glu Val Arg Ile Leu Ala Gly Leu Pro Glu Val Ser Gly Asn
           755                 760                 765
   Ile Thr Val Gln Ser Asn Asn Pro Pro Gln Pro Ser Asn Asn Gly Gly
       770                 775                 780
   Gly Asp Gly Gly Gly Asn Gly Gly Gly Asp Gly Gly Gln Tyr Asn Phe
   785                 790                 795                 800
   Ser Leu Ser Gly Ser Asp His Thr Thr Ile Tyr His Gly Lys Leu Glu
                   805                 810                 815
   Thr Gly Ile His Val Gln Gly Asn Tyr Thr Tyr Thr Gly Thr Pro Val
               820                 825                 830
   Leu Ile Leu Asn Ala Tyr Arg Asn Asn Thr Val Val Ser Ser Ile Pro
           835                 840                 845
   Val Tyr Ser Pro Phe Asp Ile Thr Ile Gln Thr Glu Ala Asp Ser Leu
       850                 855                 860
   Glu Leu Glu Leu Gln Pro Arg Tyr Gly Phe Ala Thr Val Asn Gly Thr
   865                 870                 875                 880
   Ala Thr Val Lys Ser Pro Asn Val Asn Tyr Asp Arg Ser Phe Lys Leu
                   885                 890                 895
   Pro Ile Asp Leu Gln Asn Ile Thr Thr Gln Val Asn Ala Leu Phe Ala
               900                 905                 910
   Ser Gly Thr Gln Asn Met Leu Ala His Asn Val Ser Asp His Asp Ile
           915                 920                 925
   Glu Glu Val Val Leu Lys Val Asp Ala Leu Ser Asp Glu Val Phe Gly
       930                 935                 940
   Asp Glu Lys Lys Ala Leu Arg Lys Leu Val Asn Gln Ala Lys Arg Leu
   945                 950                 955                 960
   Ser Arg Ala Arg Asn Leu Leu Ile Gly Gly Ser Phe Glu Asn Trp Asp
                   965                 970                 975
   Ala Trp Tyr Lys Gly Arg Asn Val Val Thr Val Ser Asp His Glu Leu
               980                 985                 990
   Phe Lys Ser Asp His Val Leu Leu Pro Pro Pro Gly Leu Ser Pro Ser
           995                 1000                1005
   Tyr Ile Phe Gln Lys Val Glu Glu Ser Lys Leu Lys Pro Asn Thr Arg
       1010                1015                1020
   Tyr Ile Val Ser Gly Phe Ile Ala His Gly Lys Asp Leu Glu Ile Val
   1025                1030                1035                1040
   Val Ser Arg Tyr Gly Gln Glu Val Gln Lys Val Val Gln Val Pro Tyr
                   1045                1050                1055
   Gly Glu Ala Phe Pro Leu Thr Ser Asn Gly Pro Val Cys Cys Pro Pro
               1060                1065                1070
   Arg Ser Thr Ser Asn Gly Thr Leu Gly Asp Pro His Phe Phe Ser Tyr
           1075                1080                1085
   Ser Ile Asp Val Gly Ala Leu Asp Leu Gln Ala Asn Pro Gly Ile Glu
       1090                1095                1100
   Phe Gly Leu Arg Ile Val Asn Pro Thr Gly Met Ala Arg Val Ser Asn
   1105                1110                1115                1120
   Leu Glu Ile Arg Glu Asp Arg Pro Leu Ala Ala Asn Glu Ile Arg Gln
                   1125                1130                1135
   Val Gln Arg Val Ala Arg Asn Trp Arg Thr Glu Tyr Glu Lys Glu Arg
               1140                1145                1150
   Ala Glu Val Thr Ser Leu Ile Gln Pro Val Ile Asn Arg Ile Asn Gly
           1155                1160                1165
   Leu Tyr Glu Asn Gly Asn Trp Asn Gly Ser Ile Arg Ser Asp Ile Ser
       1170                1175                1180
   Tyr Gln Asn Ile Asp Ala Ile Val Leu Pro Thr Leu Pro Lys Leu Arg
   1185 1190 1195 1200
   His Trp Phe Met Ser Asp Arg Phe Ser Glu Gln Gly Asp Ile Met Ala
   1205 1210 1215
   Lys Phe Gln Gly Ala Leu Asn Arg Ala Tyr Ala Gln Leu Glu Gln Ser
   1220 1225 1230
   Thr Leu Leu His Asn Gly His Phe Thr Lys Asp Ala Ala Asn Trp Thr
   1235 1240 1245
   Ile Glu Gly Asp Ala His Gln Ile Thr Leu Glu Asp Gly Arg Arg Val
   1250 1255 1260
   Leu Arg Leu Pro Asp Trp Ser Ser Ser Val Ser Gln Met Ile Glu Ile
   1265 1270 1275 1280
   Glu Asn Phe Asn Pro Asp Lys Glu Tyr Asn Leu Val Phe His Gly Gln
   1285 1290 1295
   Gly Glu Gly Thr Val Thr Leu Glu His Gly Glu Glu Thr Lys Tyr Ile
   1300 1305 1310
   Glu Thr His Thr His His Phe Ala Asn Phe Thr Thr Ser Gln Arg Gln
   1315 1320 1325
   Gly Leu Thr Phe Glu Ser Asn Lys Val Thr Val Thr Ile Ser Ser Glu
   1330 1335 1340
   Asp Gly Glu Phe Leu Val Asp Asn Ile Ala Leu Val Glu Ala Pro Leu
   1345 1350 1355 1360
   Pro Thr Asp Asp Gln Asn Ser Glu Gly Asn Thr Ala Ser Ser Thr Asn
   1365 1370 1375
   Ser Asp Thr Ser Met Asn Asn Asn Gln
   1380 1385
<210> 11
   <211> 1289
   <212> PRT
   <213> Bacillus thuringiensis
<400> 11
   Met Ala Ile Leu Asn Glu Leu Tyr Pro Ser Val Pro Tyr Asn Val Leu
   1 5 10 15
   Ala Tyr Thr Pro Pro Ser Phe Leu Pro Asp Ala Gly Thr Gln Ala Thr
   20 25 30
   Pro Ala Asp Leu Thr Ala Tyr Glu Gln Leu Leu Lys Asn Leu Glu Lys
   35 40 45
   Gly Ile Asn Ala Gly Thr Tyr Ser Lys Ala Ile Ala Asp Val Leu Lys
   50 55 60
   Gly Ile Phe Ile Asp Asp Thr Ile Asn Tyr Gln Thr Tyr Val Asn Ile
   65 70 75 80
   Gly Leu Ser Leu Ile Thr Leu Ala Val Pro Glu Ile Gly Ile Phe Thr
   85 90 95
   Pro Phe Ile Gly Leu Phe Phe Ala Ala Leu Asn Lys His Asp Ala Pro
   100 105 110
   Pro Pro Pro Asn Ala Lys Asp Ile Phe Glu Ala Met Lys Pro Ala Ile
   115 120 125
   Gln Glu Met Ile Asp Arg Thr Leu Thr Ala Asp Glu Gln Thr Phe Leu
   130 135 140
   Asn Gly Glu Ile Ser Gly Leu Gln Asn Leu Ala Ala Arg Tyr Gln Ser
   145 150 155 160
   Thr Met Asp Asp Ile Gln Ser His Gly Gly Phe Asn Lys Val Asp Ser
   165 170 175
   Gly Leu Ile Lys Lys Phe Thr Asp Glu Val Leu Ser Leu Asn Ser Phe
   180 185 190
   Tyr Thr Asp Arg Leu Pro Val Phe Ile Thr Asp Asn Thr Ala Asp Arg
   195 200 205
   Thr Leu Leu Gly Leu Pro Tyr Tyr Ala Ile Leu Ala Ser Met His Leu
       210                 215                 220
   Met Leu Leu Arg Asp Ile Ile Thr Lys Gly Pro Thr Trp Asp Ser Lys
   225                 230                 235                 240
   Ile Asn Phe Thr Pro Asp Ala Ile Asp Ser Phe Lys Thr Asp Ile Lys
                   245                 250                 255
   Asn Asn Ile Lys Leu Tyr Ser Lys Thr Ile Tyr Asp Val Phe Gln Lys
               260                 265                 270
   Gly Leu Ala Ser Tyr Gly Thr Pro Ser Asp Leu Glu Ser Phe Ala Lys
           275                 280                 285
   Lys Gln Lys Tyr Ile Glu Ile Met Thr Thr His Cys Leu Asp Phe Ala
       290                 295                 300
   Arg Leu Phe Pro Thr Phe Asp Pro Asp Leu Tyr Pro Thr Gly Ser Gly
   305                 310                 315                 320
   Asp Ile Ser Leu Gln Lys Thr Arg Arg Ile Leu Ser Pro Phe Ile Pro
                   325                 330                 335
   Ile Arg Thr Ala Asp Gly Leu Thr Leu Asn Asn Thr Ser Ile Asp Thr
               340                 345                 350
   Ser Asn Trp Pro Asn Tyr Glu Asn Gly Asn Gly Ala Phe Pro Asn Pro
           355                 360                 365
   Lys Glu Arg Ile Leu Lys Gln Phe Lys Leu Tyr Pro Ser Trp Arg Ala
       370                 375                 380
   Ala Gln Tyr Gly Gly Leu Leu Gln Pro Tyr Leu Trp Ala Ile Glu Val
   385                 390                 395                 400
   Gln Asp Ser Val Glu Thr Arg Leu Tyr Gly Gln Leu Pro Ala Val Asp
                   405                 410                 415
   Pro Gln Ala Gly Pro Asn Tyr Val Ser Ile Asp Ser Ser Asn Pro Ile
               420                 425                 430
   Ile Gln Ile Asn Met Asp Thr Trp Lys Thr Pro Pro Gln Gly Ala Ser
           435                 440                 445
   Gly Trp Asn Thr Asn Leu Met Arg Gly Ser Val Ser Gly Leu Ser Phe
       450                 455                 460
   Leu Gln Arg Asp Gly Thr Arg Leu Ser Ala Gly Met Gly Gly Gly Phe
   465                 470                 475                 480
   Ala Asp Thr Ile Tyr Ser Leu Pro Ala Thr His Tyr Leu Ser Tyr Leu
                   485                 490                 495
   Tyr Gly Thr Pro Tyr Gln Thr Ser Asp Asn Tyr Ser Gly His Val Gly
               500                 505                 510
   Ala Leu Val Gly Val Ser Thr Pro Gln Glu Ala Thr Leu Pro Asn Ile
           515                 520                 525
   Ile Gly Gln Pro Asp Glu Gln Gly Asn Val Ser Thr Met Gly Phe Pro
       530                 535                 540
   Phe Glu Lys Ala Ser Tyr Gly Gly Thr Val Val Lys Glu Trp Leu Asn
   545                 550                 555                 560
   Gly Ala Asn Ala Met Lys Leu Ser Pro Gly Gln Ser Ile Gly Ile Pro
                   565                 570                 575
   Ile Thr Asn Val Thr Ser Gly Glu Tyr Gln Ile Arg Cys Arg Tyr Ala
               580                 585                 590
   Ser Asn Asp Asn Thr Asn Val Phe Phe Asn Val Asp Thr Gly Gly Ala
           595                 600                 605
   Asn Pro Ile Phe Gln Gln Ile Asn Phe Ala Ser Thr Val Asp Asn Asn
       610                 615                 620
   Thr Gly Val Gln Gly Ala Asn Gly Val Tyr Val Val Lys Ser Ile Ala
   625                 630                 635                 640
   Thr Thr Asp Asn Ser Phe Thr Val Lys Ile Pro Ala Lys Thr Ile Asn
                   645                 650                 655
   Val His Leu Thr Asn Gln Gly Ser Ser Asp Val Phe Leu Asp Arg Ile
               660                 665                 670
   Glu Phe Val Pro Ile Leu Glu Ser Asn Thr Val Thr Ile Phe Asn Asn
           675                 680                 685
   Ser Tyr Thr Thr Gly Ser Ala Asn Leu Ile Pro Ala Ile Ala Pro Leu
   690 695 700
   Trp Ser Thr Ser Ser Asp Lys Ala Leu Thr Gly Ser Met Ser Ile Thr
   705 710 715 720
   Gly Arg Thr Thr Pro Asn Ser Asp Asp Ala Leu Leu Arg Phe Phe Lys
   725 730 735
   Thr Asn Tyr Asp Thr Gln Thr Ile Pro Ile Pro Gly Ser Gly Lys Asp
   740 745 750
   Phe Thr Asn Thr Leu Glu Ile Gln Asp Ile Val Ser Ile Asp Ile Phe
   755 760 765
   Val Gly Ser Gly Leu His Gly Ser Asp Gly Ser Ile Lys Leu Asp Phe
   770 775 780
   Thr Asn Asn Asn Ser Gly Ser Gly Gly Ser Pro Lys Ser Phe Thr Glu
   785 790 795 800
   Gln Asn Asp Leu Glu Asn Ile Thr Thr Gln Val Asn Ala Leu Phe Thr
   805 810 815
   Ser Asn Thr Gln Asp Ala Leu Ala Thr Asp Val Ser Asp His Asp Ile
   820 825 830
   Glu Glu Val Val Leu Lys Val Asp Ala Leu Ser Asp Glu Val Phe Gly
   835 840 845
   Lys Glu Lys Lys Thr Leu Arg Lys Phe Val Asn Gln Ala Lys Arg Leu
   850 855 860
   Ser Lys Ala Arg Asn Leu Leu Val Gly Gly Asn Phe Asp Asn Leu Asp
   865 870 875 880
   Ala Trp Tyr Arg Gly Arg Asn Val Val Asn Val Ser Asn His Glu Leu
   885 890 895
   Leu Lys Ser Asp His Val Leu Leu Pro Pro Pro Gly Leu Ser Pro Ser
   900 905 910
   Tyr Ile Phe Gln Lys Val Glu Glu Ser Lys Leu Lys Arg Asn Thr Arg
   915 920 925
   Tyr Thr Val Ser Gly Phe Ile Ala His Ala Thr Asp Leu Glu Ile Val
   930 935 940
   Val Ser Arg Tyr Gly Gln Glu Ile Lys Lys Val Val Gln Val Pro Tyr
   945 950 955 960
   Gly Glu Ala Phe Pro Leu Thr Ser Ser Gly Pro Val Cys Cys Ile Pro
   965 970 975
   His Ser Thr Ser Asn Gly Thr Leu Gly Asn Pro His Phe Phe Ser Tyr
   980 985 990
   Ser Ile Asp Val Gly Ala Leu Asp Val Asp Thr Asn Pro Gly Ile Glu
   995 1000 1005
   Phe Gly Leu Arg Ile Val Asn Pro Thr Gly Met Ala Arg Val Ser Asn
   1010 1015 1020
   Leu Glu Ile Arg Glu Asp Arg Pro Leu Ala Ala Asn Glu Ile Arg Gln
   1025 1030 1035 1040
   Val Gln Arg Val Ala Arg Asn Trp Arg Thr Glu Tyr Glu Lys Glu Arg
   1045 1050 1055
   Ala Glu Val Thr Ser Leu Ile Gln Pro Val Ile Asn Arg Ile Asn Gly
   1060 1065 1070
   Leu Tyr Asp Asn Gly Asn Trp Asn Gly Ser Ile Arg Ser Asp Ile Ser
   1075 1080 1085
   Tyr Gln Asn Ile Asp Ala Ile Val Leu Pro Thr Leu Pro Lys Leu Arg
   1090 1095 1100
   His Trp Phe Met Ser Asp Arg Phe Ser Glu Gln Gly Asp Ile Met Ala
   1105 1110 1115 1120
   Lys Phe Gln Gly Ala Leu Asn Arg Ala Tyr Ala Gln Leu Glu Gln Asn
   1125 1130 1135
   Thr Leu Leu His Asn Gly His Phe Thr Lys Asp Ala Ala Asn Trp Thr
   1140 1145 1150
   Val Glu Gly Asp Ala His Gln Val Val Leu Glu Asp Gly Lys Arg Val
   1155 1160 1165
   Leu Arg Leu Pro Asp Trp Ser Ser Ser Val Ser Gln Thr Ile Glu Ile
       1170                1175                1180
   Glu Asn Phe Asp Pro Asp Lys Glu Tyr Gln Leu Val Phe His Gly Gln
   1185                1190                1195                1200
   Gly Glu Gly Thr Val Thr Leu Glu His Gly Glu Glu Thr Lys Tyr Ile
                   1205                1210                1215
   Glu Thr His Thr His His Phe Ala Asn Phe Thr Thr Ser Gln Arg Gln
               1220                1225                1230
   Gly Leu Thr Phe Glu Ser Asn Lys Val Thr Val Thr Ile Ser Ser Glu
           1235                1240                1245
   Asp Gly Glu Phe Leu Val Asp Asn Ile Ala Leu Val Glu Ala Pro Leu
       1250                1255                1260
   Pro Thr Asp Asp Gln Asn Ser Glu Gly Asn Thr Ala Ser Ser Thr Asn
   1265                1270                1275                1280
   Ser Asp Thr Ser Met Asn Asn Asn Gln
                   1285
<210> 12
   <211> 1245
   <212> PRT
   <213> Bacillus thuringiensis
<400> 12
   Met Ala Thr Ile Asn Glu Leu Tyr Pro Val Pro Tyr Asn Val Leu Ala
    1               5                   10                  15
   His Pro Ile Lys Glu Val Asp Asp Pro Tyr Ser Trp Ser Asn Leu Leu
               20                  25                  30
   Lys Gly Ile Gln Glu Gly Trp Glu Glu Trp Gly Lys Thr Gly Gln Lys
           35                  40                  45
   Lys Leu Phe Glu Asp His Leu Thr Ile Ala Trp Asn Leu Tyr Lys Thr
       50                  55                  60
   Gly Lys Leu Asp Tyr Phe Ala Leu Thr Lys Ala Ser Ile Ser Leu Ile
   65                  70                  75                  80
   Gly Phe Ile Pro Gly Ala Glu Ala Ala Val Pro Phe Ile Asn Met Phe
                   85                  90                  95
   Val Asp Phe Val Trp Pro Lys Leu Phe Gly Ala Asn Thr Glu Gly Lys
               100                 105                 110
   Asp Gln Gln Leu Phe Asn Ala Ile Met Asp Ala Val Asn Lys Met Val
           115                 120                 125
   Asp Asn Lys Phe Leu Ser Tyr Asn Leu Ser Thr Leu Asn Lys Thr Ile
       130                 135                 140
   Glu Gly Leu Gln Gly Asn Leu Gly Leu Phe Gln Asn Ala Ile Gln Val
   145                 150                 155                 160
   Ala Ile Cys Gln Gly Ser Thr Pro Glu Arg Val Asn Phe Asp Gln Asn
                   165                 170                 175
   Cys Thr Pro Cys Asn Pro Asn Gln Pro Cys Lys Asp Asp Leu Asp Arg
               180                 185                 190
   Val Ala Ser Arg Phe Asp Thr Ala Asn Ser Gln Phe Thr Gln His Leu
           195                 200                 205
   Pro Glu Phe Lys Asn Pro Trp Ser Asp Glu Asn Ser Thr Gln Glu Phe
       210                 215                 220
   Lys Arg Thr Ser Val Glu Leu Thr Leu Pro Met Tyr Thr Thr Val Ala
   225                 230                 235                 240
   Thr Leu His Leu Leu Leu Tyr Glu Gly Tyr Ile Glu Phe Met Thr Lys
                   245                 250                 255
   Trp Asn Phe His Asn Glu Gln Tyr Leu Asn Asn Leu Lys Val Glu Leu
               260                 265                 270
   Gln Gln Leu Ile His Ser Tyr Ser Glu Thr Val Arg Thr Ser Phe Leu
           275                 280                 285
   Gln Phe Leu Pro Thr Leu Asn Asn Arg Ser Lys Ser Ser Val Asn Ala
       290                 295                 300
   Tyr Asn Arg Tyr Val Arg Asn Met Thr Val Asn Cys Leu Asp Ile Ala
   305                 310                 315                 320
   Ala Thr Trp Pro Thr Phe Asp Thr His Asn Tyr His Gln Gly Gly Lys
                   325                 330                 335
   Leu Asp Leu Thr Arg Ile Ile Leu Ser Asp Thr Ala Gly Pro Ile Glu
               340                 345                 350
   Glu Tyr Thr Thr Gly Asp Lys Thr Ser Gly Pro Glu His Ser Asn Ile
           355                 360                 365
   Thr Pro Asn Asn Ile Leu Asp Thr Pro Ser Pro Thr Tyr Gln His Ser
       370                 375                 380
   Phe Val Ser Val Asp Ser Ile Val Tyr Ser Arg Lys Glu Leu Gln Gln
   385                 390                 395                 400
   Leu Asp Ile Ala Thr Tyr Ser Thr Asn Asn Ser Asn Asn Cys His Pro
                   405                 410                 415
   Tyr Gly Leu Arg Leu Ser Tyr Thr Asp Gly Ser Arg Tyr Asp Tyr Gly
               420                 425                 430
   Asp Asn Gln Pro Asp Phe Thr Thr Ser Asn Asn Asn Tyr Cys His Asn
           435                 440                 445
   Ser Tyr Thr Ala Pro Ile Thr Leu Val Asn Ala Arg His Leu Tyr Asn
       450                 455                 460
   Ala Lys Gly Ser Leu Gln Asn Val Glu Ser Leu Val Val Ser Thr Val
   465                 470                 475                 480
   Asn Gly Gly Ser Gly Ser Cys Ile Cys Asp Ala Trp Ile Asn Tyr Leu
                   485                 490                 495
   Arg Pro Pro Gln Thr Ser Lys Asn Glu Ser Arg Pro Asp Gln Lys Ile
               500                 505                 510
   Asn Val Leu Tyr Pro Ile Thr Glu Thr Val Asn Lys Gly Thr Gly Gly
           515                 520                 525
   Asn Leu Gly Val Ile Ser Ala Tyr Val Pro Met Glu Leu Val Pro Glu
       530                 535                 540
   Asn Val Ile Gly Asp Val Asn Ala Asp Thr Lys Leu Pro Leu Thr Gln
   545                 550                 555                 560
   Leu Lys Gly Phe Pro Phe Glu Lys Tyr Gly Ser Glu Tyr Asn Asn Arg
                   565                 570                 575
   Gly Ile Ser Leu Val Arg Glu Trp Ile Asn Gly Asn Asn Ala Val Lys
               580                 585                 590
   Leu Ser Asn Ser Gln Ser Val Gly Ile Gln Ile Thr Asn Gln Thr Lys
           595                 600                 605
   Gln Lys Tyr Glu Ile Arg Cys Arg Tyr Ala Ser Lys Gly Asp Asn Asn
       610                 615                 620
   Val Tyr Phe Asn Val Asp Leu Ser Glu Asn Pro Phe Arg Asn Ser Ile
   625                 630                 635                 640
   Ser Phe Gly Ser Thr Glu Ser Ser Val Val Gly Val Gln Gly Glu Asn
                   645                 650                 655
   Gly Lys Tyr Ile Leu Lys Ser Ile Thr Thr Val Glu Ile Pro Ala Gly
               660                 665                 670
   Ser Phe Tyr Val His Ile Thr Asn Gln Gly Ser Ser Asp Leu Phe Leu
           675                 680                 685
   Asp Arg Ile Glu Phe Val Pro Lys Ile Gln Phe Gln Phe Cys Asp Asn
       690                 695                 700
   Asn Asn Leu His Cys Asp Cys Asn Asn Pro Val Asp Thr Asp Cys Thr
   705                 710                 715                 720
   Phe Cys Cys Val Cys Thr Ser Leu Thr Asp Cys Asp Cys Asn Asn Pro
                   725                 730                 735
   Arg Gly Leu Asp Cys Thr Leu Cys Cys Gln Val Glu Asn Gln Leu Pro
               740                 745                 750
   Ser Phe Val Thr Leu Thr Asp Leu Gln Asn Ile Thr Thr Gln Val Asn
           755                 760                 765
   Ala Leu Val Ala Ser Ser Glu His Asp Thr Leu Ala Thr Asp Val Ser
       770                 775                 780
   Asp Tyr Glu Ile Glu Glu Val Val Leu Lys Val Asp Ala Leu Ser Gly
   785                 790                 795                 800
   Glu Val Phe Gly Lys Glu Lys Lys Ala Leu Arg Lys Leu Val Asn His
                   805                 810                 815
   Thr Lys Arg Leu Ser Lys Ala Arg Asn Leu Leu Ile Gly Gly Asn Phe
               820                 825                 830
   Asp Asn Leu Asp Ala Trp Tyr Arg Gly Arg Asn Val Val Asn Val Ser
           835                 840                 845
   Asp His Glu Leu Phe Lys Ser Asp His Val Leu Leu Pro Pro Pro Thr
       850                 855                 860
   Leu Tyr Ser Ser Tyr Met Phe Gln Lys Val Glu Glu Ser Lys Leu Lys
   865                 870                 875                 880
   Ala Asn Thr Arg Tyr Thr Val Ser Gly Phe Ile Ala His Ala Glu Asp
                   885                 890                 895
   Leu Glu Ile Val Val Ser Arg Tyr Gly Gln Glu Val Lys Lys Val Val
               900                 905                 910
   Gln Val Pro Tyr Gly Glu Ala Phe Pro Leu Thr Ser Arg Gly Ala Ile
           915                 920                 925
   Cys Cys Pro Pro Arg Ser Thr Ser Asn Gly Lys Pro Ala Asp Pro His
       930                 935                 940
   Phe Phe Ser Tyr Ser Ile Asp Val Gly Thr Leu Asp Val Glu Ala Asn
   945                 950                 955                 960
   Pro Gly Ile Glu Leu Gly Leu Arg Ile Val Glu Arg Thr Gly Met Ala
                   965                 970                 975
   Arg Val Ser Asn Leu Glu Ile Arg Glu Asp Arg Pro Leu Lys Lys Asn
               980                 985                 990
   Glu Leu Arg Asn Val Gln Arg Ala Ala Arg Asn Trp Arg Thr Ala Tyr
           995                 1000                1005
   Asp Gln Glu Arg Ala Glu Val Thr Ala Leu Ile Gln Pro Val Leu Asn
       1010                1015                1020
   Gln Ile Asn Ala Leu Tyr Glu Asn Glu Asp Trp Asn Gly Ala Ile Arg
   1025                1030                1035                1040
   Ser Gly Val Ser Tyr His Asp Leu Glu Ala Ile Val Leu Pro Thr Leu
                   1045                1050                1055
   Pro Lys Leu Asn His Trp Phe Met Ser Asp Met Leu Gly Glu Gln Gly
               1060                1065                1070
   Ser Ile Leu Ala Gln Phe Gln Glu Ala Leu Asp Arg Ala Tyr Thr Gln
           1075                1080                1085
   Leu Glu Glu Ser Thr Ile Leu His Asn Gly His Phe Thr Thr Asp Ala
       1090                1095                1100
   Ala Asn Trp Thr Ile Glu Gly Asp Ala His His Ala Ile Leu Glu Asp
   1105                1110                1115                1120
   Gly Arg Arg Val Leu Arg Leu Pro Asp Trp Ser Ser Ser Val Ser Gln
                   1125                1130                1135
   Thr Ile Glu Ile Glu Asn Phe Asp Pro Asp Lys Glu Tyr Gln Leu Val
               1140                1145                1150
   Phe His Ala Gln Gly Glu Gly Thr Val Ser Leu Gln His Gly Glu Glu
           1155                1160                1165
   Gly Glu Tyr Val Glu Thr His Pro His Lys Ser Ala Asn Phe Thr Thr
       1170                1175                1180
   Ser His Arg Gln Gly Val Thr Phe Glu Thr Asn Lys Val Thr Val Glu
   1185                1190                1195                1200
   Ile Thr Ser Glu Asp Gly Glu Phe Leu Val Asp His Ile Ala Leu Val
                   1205                1210                1215
   Glu Ala Pro Leu Pro Thr Asp Asp Gln Ser Ser Asp Gly Asn Thr Thr
               1220                1225                1230
   Ser Asn Thr Asn Ser Asn Thr Ser Met Asn Asn Asn Gln
           1235                1240                1245
<210> 13
   <211> 1257
   <212> PRT
   <213> Bacillus thuringiensis
<400> 13
   Met Ala Thr Leu Asn Glu Val Tyr Pro Val Asn Tyr Asn Val Leu Ser
   1               5                   10                  15
   Ser Asp Ala Phe Gln Gln Leu Asp Thr Thr Gly Phe Lys Ser Lys Tyr
               20                  25                  30
   Asp Glu Met Ile Lys Ala Phe Glu Lys Lys Trp Lys Lys Gly Ala Lys
           35                  40                  45
   Gly Lys Asp Leu Leu Asp Val Ala Trp Thr Tyr Ile Thr Thr Gly Glu
       50                  55                  60
   Ile Asp Pro Leu Asn Val Ile Lys Gly Val Leu Ser Val Leu Thr Leu
   65                  70                  75                  80
   Ile Pro Glu Val Gly Thr Val Ala Ser Ala Ala Ser Thr Ile Val Ser
                   85                  90                  95
   Phe Ile Trp Pro Lys Ile Phe Gly Asp Lys Pro Asn Ala Lys Asn Ile
               100                 105                 110
   Phe Glu Glu Leu Lys Pro Gln Ile Glu Ala Leu Ile Gln Gln Asp Ile
           115                 120                 125
   Thr Asn Tyr Gln Asp Ala Ile Asn Gln Lys Lys Phe Asp Ser Leu Gln
       130                 135                 140
   Lys Thr Ile Asn Leu Tyr Thr Val Ala Ile Asp Asn Asn Asp Tyr Val
   145                 150                 155                 160
   Thr Ala Lys Thr Gln Leu Glu Asn Leu Asn Ser Ile Leu Thr Ser Asp
                   165                 170                 175
   Ile Ser Ile Phe Ile Pro Glu Gly Tyr Glu Thr Gly Gly Leu Pro Tyr
               180                 185                 190
   Tyr Ala Met Val Ala Asn Ala His Ile Leu Leu Leu Arg Asp Ala Ile
           195                 200                 205
   Val Asn Ala Glu Lys Leu Gly Phe Ser Asp Lys Glu Val Asp Thr His
       210                 215                 220
   Lys Lys Tyr Ile Lys Met Thr Ile His Asn His Thr Glu Ala Val Ile
   225                 230                 235                 240
   Lys Ala Phe Leu Asn Gly Leu Asp Lys Phe Lys Ser Leu Asp Val Asn
                   245                 250                 255
   Ser Tyr Asn Lys Lys Ala Asn Tyr Ile Lys Gly Met Thr Glu Met Val
               260                 265                 270
   Leu Asp Leu Val Ala Leu Trp Pro Thr Phe Asp Pro Asp His Tyr Gln
           275                 280                 285
   Lys Glu Val Glu Ile Glu Phe Thr Arg Thr Ile Ser Ser Pro Ile Tyr
       290                 295                 300
   Gln Pro Val Pro Lys Asn Met Gln Asn Thr Ser Ser Ser Ile Val Pro
   305                 310                 315                 320
   Ser Asp Leu Phe His Tyr Gln Gly Asp Leu Val Lys Leu Glu Phe Ser
                   325                 330                 335
   Thr Arg Thr Asp Asn Asp Gly Leu Ala Lys Ile Phe Thr Gly Ile Arg
               340                 345                 350
   Asn Thr Phe Tyr Lys Ser Pro Asn Thr His Glu Thr Tyr His Val Asp
           355                 360                 365
   Phe Ser Tyr Asn Thr Gln Ser Ser Gly Asn Ile Ser Arg Gly Ser Ser
       370                 375                 380
   Asn Pro Ile Pro Ile Asp Leu Asn Asn Pro Ile Ile Ser Thr Cys Ile
   385                 390                 395                 400
   Arg Asn Ser Phe Tyr Lys Ala Ile Ala Gly Ser Ser Val Leu Val Asn
                   405                 410                 415
   Phe Lys Asp Gly Thr Gln Gly Tyr Ala Phe Ala Gln Ala Pro Thr Gly
   420 425 430
   Gly Ala Trp Asp His Ser Phe Ile Glu Ser Asp Gly Ala Pro Glu Gly
   435 440 445
   His Lys Leu Asn Tyr Ile Tyr Thr Ser Pro Gly Asp Thr Leu Arg Asp
   450 455 460
   Phe Ile Asn Val Tyr Thr Leu Ile Ser Thr Pro Thr Ile Asn Glu Leu
   465 470 475 480
   Ser Thr Glu Lys Ile Lys Gly Phe Pro Ala Glu Lys Gly Tyr Ile Lys
   485 490 495
   Asn Gln Gly Ile Met Lys Tyr Tyr Gly Lys Pro Glu Tyr Ile Asn Gly
   500 505 510
   Ala Gln Pro Val Asn Leu Glu Asn Gln Gln Thr Leu Ile Phe Glu Phe
   515 520 525
   His Ala Ser Lys Thr Ala Gln Tyr Thr Ile Arg Ile Arg Tyr Ala Ser
   530 535 540
   Thr Gln Gly Thr Lys Gly Tyr Phe Arg Leu Asp Asn Gln Glu Leu Gln
   545 550 555 560
   Thr Leu Asn Ile Pro Thr Ser His Asn Gly Tyr Val Thr Gly Asn Ile
   565 570 575
   Gly Glu Asn Tyr Asp Leu Tyr Thr Ile Gly Ser Tyr Thr Ile Thr Glu
   580 585 590
   Gly Asn His Thr Leu Gln Ile Gln His Asn Asp Lys Asn Gly Met Val
   595 600 605
   Leu Asp Arg Ile Glu Phe Val Pro Lys Asp Ser Leu Gln Asp Ser Pro
   610 615 620
   Gln Asp Ser Pro Pro Glu Val His Glu Ser Thr Ile Ile Phe Asp Lys
   625 630 635 640
   Ser Ser Pro Thr Ile Trp Ser Ser Asn Lys His Ser Tyr Ser His Ile
   645 650 655
   His Leu Glu Gly Ser Tyr Thr Ser Gln Gly Ser Tyr Pro His Asn Leu
   660 665 670
   Leu Ile Asn Leu Phe His Pro Thr Asp Pro Asn Arg Asn His Thr Ile
   675 680 685
   His Val Asn Asn Gly Asp Met Asn Val Asp Tyr Gly Lys Asp Ser Val
   690 695 700
   Ala Asp Gly Leu Asn Phe Asn Lys Ile Thr Ala Thr Ile Pro Ser Asp
   705 710 715 720
   Ala Trp Tyr Ser Gly Thr Ile Thr Ser Met His Leu Phe Asn Asp Asn
   725 730 735
   Asn Phe Lys Thr Ile Thr Pro Lys Phe Glu Leu Ser Asn Glu Leu Glu
   740 745 750
   Asn Ile Thr Thr Gln Val Asn Ala Leu Phe Ala Ser Ser Ala Gln Asp
   755 760 765
   Thr Leu Ala Ser Asn Val Ser Asp Tyr Trp Ile Glu Gln Val Val Met
   770 775 780
   Lys Val Asp Ala Leu Ser Asp Glu Val Phe Gly Lys Glu Lys Lys Ala
   785 790 795 800
   Leu Arg Lys Leu Val Asn Gln Ala Lys Arg Leu Ser Lys Ile Arg Asn
   805 810 815
   Leu Leu Ile Gly Gly Asn Phe Asp Asn Leu Val Ala Trp Tyr Met Gly
   820 825 830
   Lys Asp Val Val Lys Glu Ser Asp His Glu Leu Phe Lys Ser Asp His
   835 840 845
   Val Leu Leu Pro Pro Pro Thr Phe His Pro Ser Tyr Ile Phe Gln Lys
   850 855 860
   Val Glu Glu Ser Lys Leu Lys Pro Asn Thr Arg Tyr Thr Ile Ser Gly
   865 870 875 880
   Phe Ile Ala His Gly Glu Asp Val Glu Leu Val Val Ser Arg Tyr Gly
   885 890 895
   Gln Glu Ile Gln Lys Val Met Gln Val Pro Tyr Glu Glu Ala Leu Pro
               900                 905                 910
   Leu Thr Ser Glu Ser Asn Ser Ser Cys Cys Val Pro Asn Leu Asn Ile
           915                 920                 925
   Asn Glu Thr Leu Ala Asp Pro His Phe Phe Ser Tyr Ser Ile Asp Val
       930                 935                 940
   Gly Ser Leu Glu Met Glu Ala Asn Pro Gly Ile Glu Phe Gly Leu Arg
   945                 950                 955                 960
   Ile Val Lys Pro Thr Gly Met Ala Arg Val Ser Asn Leu Glu Ile Arg
                   965                 970                 975
   Glu Asp Arg Pro Leu Thr Ala Lys Glu Ile Arg Gln Val Gln Arg Ala
               980                 985                 990
   Ala Arg Asp Trp Lys Gln Asn Tyr Glu Gln Glu Arg Thr Glu Ile Thr
           995                 1000                1005
   Ala Ile Ile Gln Pro Val Leu Asn Gln Ile Asn Ala Leu Tyr Glu Asn
       1010                1015                1020
   Glu Asp Trp Asn Gly Ser Ile Arg Ser Asn Val Ser Tyr His Asp Leu
   1025                1030                1035                1040
   Glu Gln Ile Met Leu Pro Thr Leu Leu Lys Thr Glu Glu Ile Asn Cys
                   1045                1050                1055
   Asn Tyr Asp His Pro Ala Phe Leu Leu Lys Val Tyr His Trp Phe Met
               1060                1065                1070
   Thr Asp Arg Ile Gly Glu His Gly Thr Ile Leu Ala Arg Phe Gln Glu
           1075                1080                1085
   Ala Leu Asp Arg Ala Tyr Thr Gln Leu Glu Ser Arg Asn Leu Leu His
       1090                1095                1100
   Asn Gly His Phe Thr Thr Asp Thr Ala Asn Trp Thr Ile Glu Gly Asp
   1105                1110                1115                1120
   Ala His His Thr Ile Leu Glu Asp Gly Arg Arg Val Leu Arg Leu Pro
                   1125                1130                1135
   Asp Trp Ser Ser Asn Ala Thr Gln Thr Ile Glu Ile Glu Asp Phe Asp
               1140                1145                1150
   Leu Asp Gln Glu Tyr Gln Leu Leu Ile His Ala Lys Gly Lys Gly Ser
           1155                1160                1165
   Ile Thr Leu Gln His Gly Glu Glu Asn Glu Tyr Val Glu Thr His Thr
       1170                1175                1180
   His His Thr Asn Asp Phe Ile Thr Ser Gln Asn Ile Pro Phe Thr Phe
   1185                1190                1195                1200
   Lys Gly Asn Gln Ile Glu Val His Ile Thr Ser Glu Asp Gly Glu Phe
                   1205                1210                1215
   Leu Ile Asp His Ile Thr Val Ile Glu Val Ser Lys Thr Asp Thr Asn
               1220                1225                1230
   Thr Asn Ile Ile Glu Asn Ser Pro Ile Asn Thr Ser Met Asn Ser Asn
           1235                1240                1245
   Val Arg Val Asp Ile Pro Arg Ser Leu
       1250                1255
<210> 14
   <211> 1167
   <212> PRT
   <213> Unknown
<220>
   <223> SEQ ID NO:5 from US Patent No. 5,589,382
<400> 14
   Met Thr Asn Pro Thr Ile Leu Tyr Pro Ser Tyr His Asn Val Leu Ala
    1               5                  10                  15
   His Pro Ile Arg Leu Asp Ser Phe Phe Asp Pro Phe Val Glu Thr Phe
                   20                  25                  30
   Lys Asp Leu Lys Gly Ala Trp Glu Glu Phe Gly Lys Thr Gly Tyr Met
               35                  40                  45
   Asp Pro Leu Lys Gln His Leu Gln Ile Ala Trp Asp Thr Ser Gln Asn
       50                  55                  60
   Gly Thr Val Asp Tyr Leu Ala Leu Thr Lys Ala Ser Ile Ser Leu Ile
   65                  70                  75                  80
   Gly Leu Ile Pro Gly Ala Asp Ala Val Val Pro Phe Ile Asn Met Phe
                   85                  90                  95
   Val Asp Phe Ile Phe Pro Lys Leu Phe Gly Arg Gly Ser Gln Gln Asn
               100                 105                 110
   Ala Gln Ala Gln Phe Phe Glu Leu Ile Ile Glu Lys Val Lys Glu Leu
           115                 120                 125
   Val Asp Glu Asp Phe Arg Asn Phe Thr Leu Asn Asn Leu Leu Asn Tyr
       130                 135                 140
   Leu Asp Gly Met Gln Thr Ala Leu Ser His Phe Gln Asn Asp Val Gln
   145                 150                 155                 160
   Ile Ala Ile Cys Gln Gly Glu Gln Pro Gly Leu Met Leu Asp Gln Thr
                   165                 170                 175
   Pro Thr Ala Cys Thr Pro Thr Thr Asp His Leu Ile Ser Val Arg Glu
               180                 185                 190
   Ser Phe Lys Asp Ala Arg Thr Thr Ile Glu Thr Ala Leu Pro His Phe
           195                 200                 205
   Lys Asn Pro Met Leu Ser Thr Asn Asp Asn Thr Pro Asp Phe Asn Ser
       210                 215                 220
   Asp Thr Val Leu Leu Thr Leu Pro Met Tyr Thr Thr Gly Ala Thr Leu
   225                 230                 235                 240
   Asn Leu Ile Leu His Gln Gly Tyr Ile Gln Phe Ala Glu Arg Trp Lys
                   245                 250                 255
   Ser Val Asn Tyr Asp Glu Ser Phe Ile Asn Gln Thr Lys Val Asp Leu
               260                 265                 270
   Gln Arg Arg Ile Gln Asp Tyr Ser Thr Thr Val Ser Thr Thr Phe Glu
           275                 280                 285
   Lys Phe Lys Pro Thr Leu Asn Pro Ser Asn Lys Glu Ser Val Asn Lys
       290                 295                 300
   Tyr Asn Arg Tyr Val Arg Ser Met Thr Leu Gln Ser Leu Asp Ile Ala
   305                 310                 315                 320
   Ala Thr Trp Pro Thr Leu Asp Asn Val Asn Tyr Pro Ser Asn Val Asp
                   325                 330                 335
   Ile Gln Leu Asp Gln Thr Arg Leu Val Phe Ser Asp Val Ala Gly Pro
               340                 345                 350
   Trp Glu Gly Asn Asp Asn Ile Thr Ser Asn Ile Ile Asp Val Leu Thr
           355                 360                 365
   Pro Ile Asn Thr Gly Ile Gly Phe Gln Glu Ser Ser Asp Leu Arg Lys
       370                 375                 380
   Phe Thr Tyr Pro Arg Ile Glu Leu Gln Ser Met Gln Phe His Gly Gln
   385                 390                 395                 400
   Tyr Val Asn Ser Lys Ser Val Glu His Cys Tyr Ser Asp Gly Leu Lys
                   405                 410                 415
   Leu Asn Tyr Lys Asn Lys Thr Ile Thr Ala Gly Val Ser Asn Ile Asp
               420                 425                 430
   Glu Ser Asn Gln Asn Asn Lys His Asn Tyr Gly Pro Val Ile Asn Ser
           435                 440                 445
   Pro Ile Thr Asp Ile Asn Val Asn Ser Gln Asn Ser Gln Tyr Leu Asp
       450                 455                 460
   Leu Asn Ser Val Met Val Asn Gly Gly Gln Lys Val Thr Gly Cys Ser
   465                 470                 475                 480
   Pro Leu Ser Ser Asn Gly Asn Ser Asn Asn Ala Ala Leu Pro Asn Gln
                   485                 490                 495
   Lys Ile Asn Val Ile Tyr Ser Val Gln Ser Asn Asp Lys Pro Glu Lys
           500                 505                 510
   His Ala Asp Thr Tyr Arg Lys Trp Gly Tyr Met Ser Ser His Ile Pro
               515                 520                 525
   Tyr Asp Leu Val Pro Glu Asn Val Ile Gly Asp Ile Asp Pro Asp Thr
       530                 535                 540
   Lys Gln Pro Ser Leu Leu Leu Lys Gly Phe Pro Ala Glu Lys Gly Tyr
   545                 550                 555                 560
   Gly Asp Ser Ile Ala Tyr Val Ser Glu Pro Leu Asn Gly Ala Asn Ala
                   565                 570                 575
   Val Lys Leu Thr Ser Tyr Gln Val Leu Gln Met Glu Val Thr Asn Gln
               580                 585                 590
   Thr Thr Gln Lys Tyr Arg Ile Arg Ile Arg Tyr Ala Thr Gly Gly Asp
           595                 600                 605
   Thr Ala Ala Ser Ile Trp Phe His Ile Ile Gly Pro Ser Gly Asn Asp
       610                 615                 620
   Leu Thr Asn Glu Gly His Asn Phe Ser Ser Val Ser Ser Arg Asn Lys
   625                 630                 635                 640
   Met Phe Val Gln Gly Asn Asn Gly Lys Tyr Val Leu Asn Ile Leu Thr
                   645                 650                 655
   Asp Ser Ile Glu Leu Pro Ser Gly Gln Gln Thr Ile Leu Ile Gln Asn
               660                 665                 670
   Thr Asn Ser Gln Asp Leu Phe Leu Asp Arg Ile Glu Phe Ile Ser Leu
           675                 680                 685
   Pro Ser Thr Ser Thr Pro Thr Ser Thr Asn Phe Val Glu Pro Glu Ser
       690                 695                 700
   Leu Glu Lys Ile Ile Asn Gln Val Asn Gln Leu Phe Ser Ser Ser Ser
   705                 710                 715                 720
   Gln Thr Glu Leu Ala His Thr Val Ser Asp Tyr Lys Ile Asp Gln Val
                   725                 730                 735
   Val Leu Lys Val Asn Ala Leu Ser Asp Asp Val Phe Gly Val Glu Lys
               740                 745                 750
   Lys Ala Leu Arg Lys Leu Val Asn Gln Ala Lys Gln Leu Ser Lys Ala
           755                 760                 765
   Arg Asn Val Leu Val Gly Gly Asn Phe Glu Lys Gly His Glu Trp Ala
       770                 775                 780
   Leu Ser Arg Glu Ala Thr Met Val Ala Asn His Glu Leu Phe Lys Gly
   785                 790                 795                 800
   Asp His Leu Leu Leu Pro Pro Pro Thr Leu Tyr Pro Ser Tyr Ala Tyr
                   805                 810                 815
   Gln Lys Ile Asp Glu Ser Lys Leu Lys Ser Asn Thr Arg Tyr Thr Val
               820                 825                 830
   Ser Gly Phe Ile Ala Gln Ser Glu His Leu Glu Val Val Val Ser Arg
           835                 840                 845
   Tyr Gly Lys Glu Val His Asp Met Leu Asp Ile Pro Tyr Glu Glu Ala
       850                 855                 860
   Leu Pro Ile Ser Ser Asp Glu Ser Pro Asn Cys Cys Lys Pro Ala Ala
   865                 870                 875                 880
   Cys Gln Cys Ser Ser Cys Asp Gly Ser Gln Ser Asp Ser His Phe Phe
                   885                 890                 895
   Ser Tyr Ser Ile Asp Val Gly Ser Leu Gln Ser Asp Val Asn Leu Gly
               900                 905                 910
   Ile Glu Phe Gly Leu Arg Ile Ala Lys Pro Asn Gly Phe Ala Lys Ile
           915                 920                 925
   Ser Asn Leu Glu Ile Lys Glu Asp Arg Pro Leu Thr Glu Lys Glu Ile
       930                 935                 940
   Lys Lys Val Gln Arg Lys Glu Gln Lys Trp Lys Lys Ala Phe Asn Gln
   945                 950                 955                 960
   Glu Gln Ala Glu Val Ala Thr Thr Leu Gln Pro Thr Leu Asp Gln Ile
                   965                 970                 975
   Asn Ala Leu Tyr Gln Asn Glu Asp Trp Asn Gly Ser Val His Pro Ala
                   980                     985                     990
   Ser Asp Tyr Gln His Leu Ser Ala Val Val Val Pro Thr Leu Pro Lys
               995                     1000                    1005
   Gln Arg His Trp Phe Met Glu Gly Arg Glu Gly Glu His Val Val Leu
       1010                    1015                    1020
   Thr Gln Gln Phe Gln Gln Ala Leu Asp Arg Ala Phe Gln Gln Ile Glu
   1025                    1030                    1035                1040
   Glu Gln Asn Leu Ile His Asn Gly Asn Leu Ala Asn Gly Leu Thr Asp
                   1045                    1050                    1055
   Trp Thr Val Thr Gly Asp Ala Gln Leu Thr Ile Phe Asp Glu Asp Pro
               1060                    1065                    1070
   Val Leu Glu Leu Ala His Trp Asp Ala Ser Ile Ser Gln Thr Ile Glu
           1075                    1080                    1085
   Ile Met Asp Phe Glu Gly Arg His Arg Ile Gln Thr Ala Cys Thr Trp
       1090                    1095                    1100
   Lys Arg Gln Arg Asn Ser Tyr Arg Ser Thr Trp Arg Lys Arg Leu Glu
   1105                    1110                    1115                1120
   Thr Met Thr Phe Asn Thr Thr Ser Phe Thr Thr Gln Glu Gln Thr Phe
                   1125                    1130                    1135
   Tyr Phe Glu Gly Asp Thr Val Asp Val His Val Gln Ser Glu Asn Asn
               1140                    1145                    1150
   Thr Phe Leu Ile Asp Ser Val Glu Leu Ile Glu Ile Ile Glu Glu
           1155                    1160                    1165
<210> 15
   <211> 1286
   <212> PRT
   <213> Bacillus thuringiensis
<400> 15
   Met Ala Asp Leu Thr Glu Leu Tyr Pro Ser Tyr His Asn Val Leu Ala
    1               5                   10                  15
   Arg Pro Ile Arg Leu Asp Ser Ile Phe Asp Pro Phe Ile Asp Ile Phe
               20                  25                  30
   Asn Ala Leu Lys Gly Gly Trp Glu Glu Phe Ala Lys Thr Gly Tyr Lys
           35                  40                  45
   Asp Pro Leu Glu Gln His Leu Lys Ile Ala Trp Asn Ala Ser Gln Asn
       50                  55                  60
   Gly Thr Ile Asp Tyr Leu Ala Leu Thr Lys Ala Ser Ile Ser Phe Ile
   65                  70                  75                  80
   Gly Leu Ile Pro Asp Ala Asp Ala Val Val Pro Phe Ile Asn Met Phe
                   85                  90                  95
   Val Asp Phe Ile Phe Pro Lys Leu Phe Gly Glu Gly Ser Gln Gln Asn
               100                 105                 110
   Ser Gln Ala Gln Phe Phe Glu Leu Ile Ile Glu Lys Val Lys Glu Ile
           115                 120                 125
   Val Asp Gln Glu Phe Arg Asn Phe Thr Leu Asn Thr Leu Leu Asn Asp
       130                 135                 140
   Leu Asp Gly Met Gln Thr Thr Leu Glu His Phe Gln Asn Asp Val Gln
   145                 150                 155                 160
   Ile Ala Ile Cys Gln Gly Glu Gln Pro Gly Leu Ile Leu Asp Glu Lys
                   165                 170                 175
   His Pro Pro Cys Thr Pro Thr Lys Asn His Leu Val Ser Val Lys Glu
               180                 185                 190
   Ser Phe Lys Asn Ala Arg Thr Ser Ile Glu Thr Val Leu Pro His Phe
           195                 200                 205
   Lys Asn Pro Met Thr Asn Asn Lys Thr Pro Asp Phe Asn Ser Asp Thr
       210                 215                 220
   Val Leu Leu Thr Leu Pro Met Tyr Thr Thr Ala Ala Thr Leu Asn Leu
   225                 230                 235                 240
   Ile Leu His Gln Gly Tyr Ile Gln Phe Val Glu Arg Trp Lys Ser Val
                   245                 250                 255
   Asp Tyr Asp Glu Ala Phe Ile Asn Gln Thr Lys Ala Asp Leu Gln His
               260                 265                 270
   Arg Ile Gln Glu Tyr Ser Thr Thr Val Ser Thr Thr Phe Glu Lys Phe
           275                 280                 285
   Lys Pro Thr Leu Ser Asn Lys Lys Ser Ser Ile Asn Thr Tyr Asn Lys
       290                 295                 300
   Tyr Val Arg Ser Met Thr Leu Asn Cys Leu Asp Ile Ala Ala Thr Trp
   305                 310                 315                 320
   Pro Thr Leu Asp Asn Val Asn Tyr Pro Ser Asn Val Glu Ile Gln Leu
                   325                 330                 335
   Asp Gln Thr Arg Leu Val Phe Ser Asn Leu Val Gly Pro Phe Glu Gly
               340                 345                 350
   Asn Asp Asp Ile Ser Thr Tyr Thr Arg Arg Ser Ile Met Asn Tyr Ser
           355                 360                 365
   Lys Gly Asp Thr Pro Gly Asp Val Asn Ser Ala Ile Gln Ser Leu Arg
       370                 375                 380
   Tyr Pro Arg Leu Glu Leu Ser Lys Val Gln Phe Tyr Thr His Asp Gln
   385                 390                 395                 400
   Arg Ser Asn Gly Val Arg His Cys Tyr Thr Ser Gly Phe Asn Leu Thr
                   405                 410                 415
   Phe Asn Asp Asn Ser Ser Met Ser Ala Lys Gln Asp Glu Ser Ala Thr
               420                 425                 430
   Ala Asp Ser Pro Pro Leu Thr Ala Pro Ile Lys Asn Met Asn Ala Asn
           435                 440                 445
   Ser Gln Asn Ser Gln Tyr Tyr Asp Tyr Ser Ser Ile Asn Ile Asp Asn
       450                 455                 460
   Gln Gly Gly Gly Gly Cys Ser Ala Phe Pro Ser Tyr Gln Ser Asn Asn
   465                 470                 475                 480
   Pro Ile Leu Pro Asn Gln Lys Ile Asn Val Phe Tyr Pro Tyr Gly Ser
                   485                 490                 495
   Ser Ala His Pro Ile Asp Pro His Thr Thr Asp Pro Asp Thr Trp Phe
               500                 505                 510
   Lys Leu Gly Tyr Val Ser Ser His Ile Pro Tyr Asp Leu Thr Pro Gln
           515                 520                 525
   Asn Val Ile Gly Glu Ile Asp Gln Asp Thr Lys Gln Pro Ser Leu Ile
       530                 535                 540
   Leu Lys Gly Phe Pro Ala Glu Lys Gly Tyr Gly Gly Ser Ile Glu Tyr
   545                 550                 555                 560
   Val Ser Glu Pro Leu Asn Gly Ala Asn Ala Ala Lys Leu Thr Leu Asn
                   565                 570                 575
   Gln Ile Leu Tyr Met Gln Val Thr Asn Leu Thr Thr Gln Lys Tyr Gln
               580                 585                 590
   Ile Arg Leu Arg Tyr Ala Thr Lys Asn Asp Thr Thr Ala Ser Val Trp
           595                 600                 605
   Phe His Ile Ile Gly Pro Asn Asn Gln Asp Ile Ile Asn His Ser Pro
       610                 615                 620
   Asp Ile Pro Pro Arg Ser Asn Asn Lys Met Phe Val Gln Gly Glu Asn
   625                 630                 635                 640
   Gly Lys Tyr Val Leu Asp Thr Leu Val Asp Ser Ile Glu Leu Pro Ser
                   645                 650                 655
   Gly Gln Leu Thr Ile Leu Ile Gln Asn Ile Asn Pro Asp Gln Asp Leu
               660                 665                 670
   Phe Leu Asp Arg Ile Glu Phe Val Pro Ile Pro Thr Leu Pro Thr Asn
           675                 680                 685
   Pro Asn Ile Ser Ile Pro Lys Thr Asp Thr Ser Pro Lys Asp Ser Lys
       690                 695                 700
   Val Leu Trp Glu Ala Ser Pro Asp Ile Pro Ile Ala Asn Thr Ile Thr
   705                 710                 715                 720
   Leu Thr Gly Ser Val Tyr Asp Phe Ala Asp Ile Thr Phe Glu Leu Tyr
                   725                 730                 735
   Lys Asn Gly Asn Met Val Thr Ser Tyr Pro Ile Lys Gly Pro Gly Pro
               740                 745                 750
   Ile Pro His Arg Ser His Gly Asn Tyr Val Ser Cys Ser Gln Gly Ile
           755                 760                 765
   Leu Ser Tyr Asn Tyr Glu Asn Lys Pro Val Leu Asp Gly Phe Asp Gln
       770                 775                 780
   Leu Arg Ile Asn Ile Asn Ser Asp Pro Ser Phe Tyr Asp Ser Asn Ser
   785                 790                 795                 800
   Gly Cys Asp Thr Lys Asn Gln Tyr Ser Ala Glu Ile Lys Ile Asn Pro
                   805                 810                 815
   Asn Leu Ser Ala Thr Thr Asp Leu Glu Lys Ile Thr Asn Gln Val Asn
               820                 825                 830
   Gln Leu Phe Thr Ser Ser Ser Gln Thr Glu Leu Ala Asn Thr Ile Thr
           835                 840                 845
   Asp Tyr Arg Ile Asp Gln Ile Val Met Lys Val Asp Ala Leu Ser Asn
       850                 855                 860
   Asn Val Phe Gly Val Glu Lys Lys Ala Leu Arg Lys Leu Val Asn Gln
   865                 870                 875                 880
   Ala Lys Gln Leu Ser Lys Ala Arg Asn Val Leu Ala Gly Gly Asn Phe
                   885                 890                 895
   Glu Lys Gly His Glu Trp Val Leu Gly Arg Glu Ala Thr Met Ile Ala
               900                 905                 910
   Asn His Glu Leu Phe Lys Gly Asp His Leu Leu Leu Pro Pro Pro Thr
           915                 920                 925
   Leu Tyr Pro Ser Tyr Ala Tyr Gln Lys Ile Asp Glu Ser Lys Leu Lys
       930                 935                 940
   Ser Asn Thr Arg Tyr Thr Val Ser Gly Phe Ile Ala Gln Ser Glu His
   945                 950                 955                 960
   Leu Glu Val Ile Val Ser Arg Tyr Gly Lys Glu Val His Asp Met Leu
                   965                 970                 975
   Asp Val Pro Tyr Glu Glu Ala Leu Pro Ile Ser Ser Asp Glu Ser Pro
               980                 985                 990
   Asn Cys Cys Lys Pro Ala Thr Cys Gln Cys Pro Ser Cys Asp Gly Ser
           995                 1000                1005
   Gln Pro Asp Ser His Phe Phe Ser Tyr Ser Ile Asp Val Gly Ser Val
       1010                1015                1020
   Gln Ser Asp Val Asn Leu Gly Ile Glu Phe Gly Leu Arg Ile Ala Lys
   1025                1030                1035                1040
   Pro Asn Gly Phe Ala Lys Ile Ser Asn Leu Glu Ile Lys Glu Asp Arg
                   1045                1050                1055
   Pro Leu Thr Asp Gln Glu Ile Lys Lys Ile Gln Arg Lys Glu Gln Lys
               1060                1065                1070
   Trp Lys Lys Ala Phe Asp Gln Glu Gln Ala Glu Val Ala Ala Thr Phe
           1075                1080                1085
   Gln Pro Thr Leu Asp Gln Ile Asn Ala Leu Tyr Gln Asn Glu Asp Trp
       1090                1095                1100
   Asn Gly Ser Leu His Pro His Val Thr Tyr Gln His Leu Ser Ala Val
   1105                1110                1115                1120
   Val Leu Pro Thr Leu Pro Lys Gln Arg His Trp Phe Met Glu Asp Arg
                   1125                1130                1135
   Glu Gly Glu His Tyr Gly Val Thr Gln Gln Phe Gln Gln Ala Leu Asp
               1140                1145                1150
   Arg Gly Phe Gln Gln Ile Glu Glu Gln Asn Leu Ile His Asn Gly Ser
           1155                1160                1165
   Phe Ala Asn Gly Leu Thr Asp Trp Thr Val Thr Gly Asp Ala Gln Leu
       1170                1175                1180
   Thr Ile Phe Asp Glu Asp Pro Val Leu Glu Leu Ala His Trp Asp Ala
   1185                1190                1195                1200
   Ser Val Ser Gln Thr Ile Glu Ile Met Asp Phe Glu Glu Glu Thr Glu
                   1205                1210                1215
   Tyr Lys Leu Arg Val Arg Gly Lys Gly Lys Gly Thr Val Thr Val Gln
               1220                1225                1230
   His Gly Glu Glu Glu Leu Glu Thr Met Thr Phe Asn Thr Thr Ser Phe
           1235                1240                1245
   Thr Thr Gln Glu Gln Thr Phe Tyr Phe Glu Gly Asp Thr Val Asp Val
       1250                1255                1260
   His Val Gln Ser Glu Asn Asn Thr Phe Leu Val Asp Ser Val Glu Leu
   1265                1270                1275                1280
   Ile Glu Val Val Glu Glu
                   1285

## Description

### FIELD OF THE INVENTION

This invention relates to the field of molecular biology. Provided are novel genes that encode pesticidal proteins. These proteins and the nucleic acid sequences that encode them are useful in preparing pesticidal formulations and in the production of transgenic pest-resistant plants.

### BACKGROUND OF THE INVENTION

*Bacillus thuringiensis* is a Gram-positive spore forming soil bacterium characterized by its ability to produce crystalline inclusions that are specifically toxic to certain orders and species of insects, but are harmless to plants and other non-targeted organisms. For this reason, compositions including *Bacillus thuringiensis* strains or their insecticidal proteins can be used as environmentally-acceptable insecticides to control agricultural insect pests or insect vectors for a variety of human or animal diseases.

Crystal (Cry) proteins (delta-endotoxins) from *Bacillus thuringiensis* have potent insecticidal activity against predominantly Lepidopteran, Dipteran, and Coleopteran larvae. These proteins also have shown activity against *Hymenoptera, Homoptera*, *Phthiraptera*, *Mallophaga*, and *Acari* pest orders, as well as other invertebrate orders such as *Nemathelminthes*, *Platyhelminthes*, and *Sarcomastigorphora* (Feitelson (1993) "The Bacillus Thuringiensis family tree" in Advanced Engineered Pesticides, Marcel Dekker, Inc., New York, NY) These proteins were originally classified as CryI to CryV based primarily on their insecticidal activity. The major classes were *Lepidoptera-*specific (I), *Lepidoptera-* and *Diptera-*specific (II), *Coleoptera-*specific (III), *Diptera-*specific (IV), and nematode-specific (V) and (VI). The proteins were further classified into subfamilies; more highly related proteins within each family were assigned divisional letters such as Cry1A, Cry1B, Cry1C, etc. Even more closely related proteins within each division were given names such as *Cry1C1*, *Cry1C2*, etc.

A new nomenclature was recently described tor the Cry genes based upon amino acid sequence homology rather than insect target specificity (Crickmore et al. (1998) Microbiol. Mol. Biol. Rev. 62:807-813). In the new classification, each toxin is assigned a unique name incorporating a primary rank (an Arabic number), a secondary rank (an uppercase letter), a tertiary rank (a lowercase letter), and a quaternary rank (another Arabic number). In the new classification, Roman numerals have been exchanged for Arabic numerals in the primary rank.

The crystal protein does not exhibit insecticidal activity until it has been ingested and solubilized in the insect midgut. The ingested protoxin is hydrolyzed by proteases in the insect digestive tract to an active toxic molecule. (Höfte and Whiteley (1989) Microbiol. Rev. 53:242-255). This toxin binds to apical brush border receptors in the midgut of the target larvae and inserts into the apical membrane creating ion channels or pores, resulting in larval death.

Delta-endotoxins generally have five conserved sequence domains, and three conserved structural domains (see, for example, de Maagd et al. (2001) Trends Genetics 17:193-199). The first conserved structural domain consists of seven alpha helices and is involved in membrane insertion and pore formation. Domain II consists of three beta-sheets arranged in a Greek key configuration, and domain III consists of two antiparallel beta-sheets in "jelly-roll" formation (de Maagd *et al.,* 2001, *supra*). Domains II and III are involved in receptor recognition and binding, and are therefore considered determinants of toxin specificity.

Because of the devastation that insects can confer, there is a continual need to discover new forms of *Bacillus thuningiensis* delta-endotoxins.

### BRIEF SUMMARY OF THE INVENTION

Compositions and methods for conferring pesticide resistance to bacteria, plants, plant cells, tissues and seeds are provided. Compositions include nucleic acid molecules encoding sequences for delta-endotoxin polypeptides, vectors comprising those nucleic acid molecules, and host cells comprising the vectors. Compositions also include the polypeptide sequences of the endotoxin, and antibodies to those polypeptides. The nucleotide sequences can be used in DNA constructs or expression cassettes for transformation and expression in organisms, including microorganisms and plants. The nucleotide or amino acid sequences may be synthetic sequences that have been designed for expression in an organism including, but not limited to, a microorganism or a plant. Compositions also comprise transformed bacteria, plants, plant cells, tissues, and seeds.

In particular, isolated nucleic acid molecules corresponding to delta-endotoxin nucleic acid sequences are provided. Additionally, amino acid sequences corresponding to the polynucleotides are encompassed. In particular, the present invention provides for an isolated nucleic acid molecule comprising a nucleotide sequence encoding the amino acid sequence shown in SEQ ID NO: 2, 4, or 6, a nucleotide sequence set forth in SEQ ID NO: 1, 3, or 5, or the delta-endotoxin nucleotide sequence deposited in a bacterial host as Accession Nos. NRRL B-30805, NRRL B-30809, and NRRL B-30808. Nucleotide sequences that are complementary to a nucleotide sequence of the invention, or that hybridize to a sequence of the invention are also encompassed.

Methods are provided for producing the polypeptides of the invention, and for using those polypeptides for controlling or killing a lepidopteran or coleopteran pest. Methods and kits for detecting the nucleic acids and polypeptides of the invention in a sample are also included.

The compositions and methods of the invention are useful for the production of organisms with pesticide resistance, specifically bacteria and plants. These organisms and compositions derived from them are desirable for agricultural purposes. The compositions of the invention are also useful for generating altered or improved delta-endotoxin proteins that have pesticidal activity, or for detecting the presence of delta-endotoxin proteins or nucleic acids in products or organisms.

### BRIEF DESCRIPTION OF FIGURES

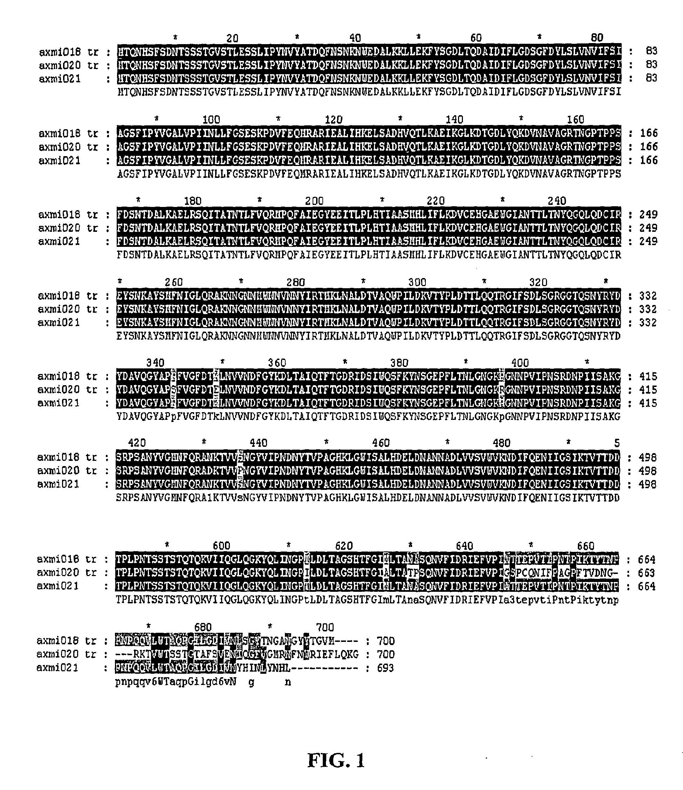

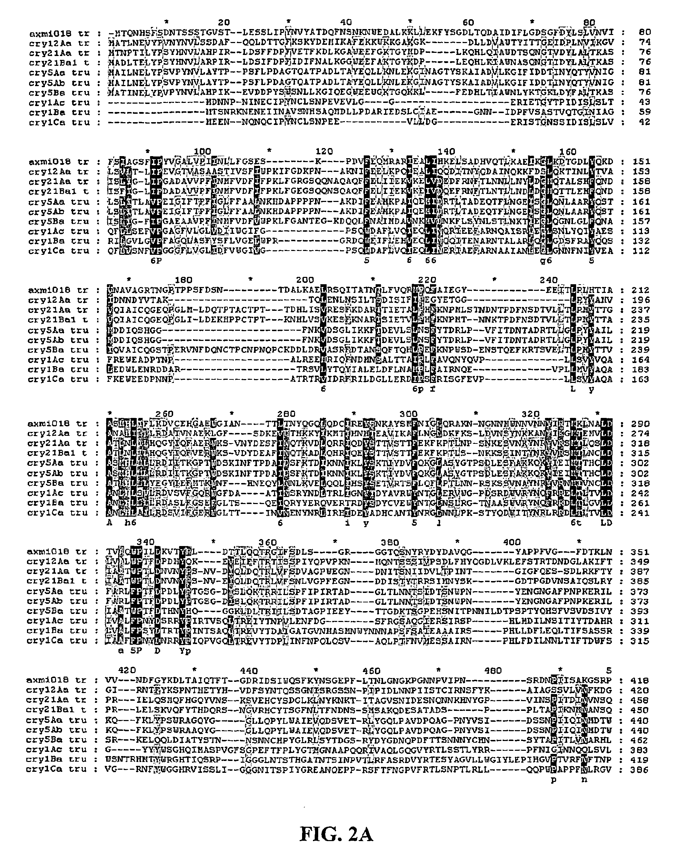

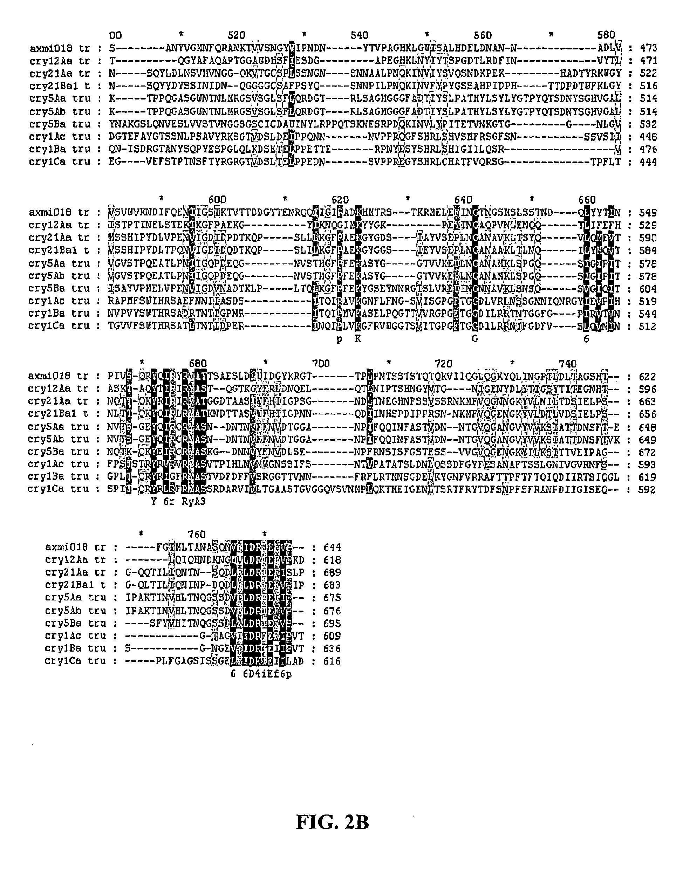

Figure 1 shows an alignment of AXMI-018 (SEQ ID NO: 2), AXMI-020 (SEQ ID NO: 4) and AXMI-21 (SEQ ID NO: 6). Toxins having C-terminal non-toxic domains were artificially truncated as shown. AXMI-018 and AXMI-020 have the C-terminal domain common to many crystal proteins, while AXMI-021 occurs naturally truncated. The alignment shows the most highly conserved amino acid residues highlighted in black, and highly conserved amino acid residues highlighted in gray. Figures 2A and 2B show an alignment of the truncated portion of AXMI-018 with cryl2Aa (SEQ ID NO:13), cry21Aa (SEQ ID NO:14), cry21Ba1 (SEQ ID NO:15), cry5Aa (SEQ ID NO:10), cry5Ab (SEQ ID NO:11), cry5Ba (SEQ ID NO:12), cry1Ac (SEQ ID NO:7), cry1Ba (SEQ ID NO:8), and cry1Ca (SEQ ID NO:9). The alignment shows the most highly conserved amino acid residues highlighted in black, and highly conserved amino acid residues highlighted in gray. Conserved group 1 in AXMI-018 is found from about amino acid residue 200 to about 222 of SEQ ID NO:2. Conserved group 2 is found from about amino acid residue 274 to about 312 of SEQ ID NO:2. Conserved group 3 is found from about amino acid residue 480 to about 533 of SEQ ID NO:2. Conserved group 4 is found from about amino acid residue 550 to about 559 of SEQ ID NO:4. Conserved group 5 is found from about amino acid residue 635 to about 644 of SEQ ID NO:2.

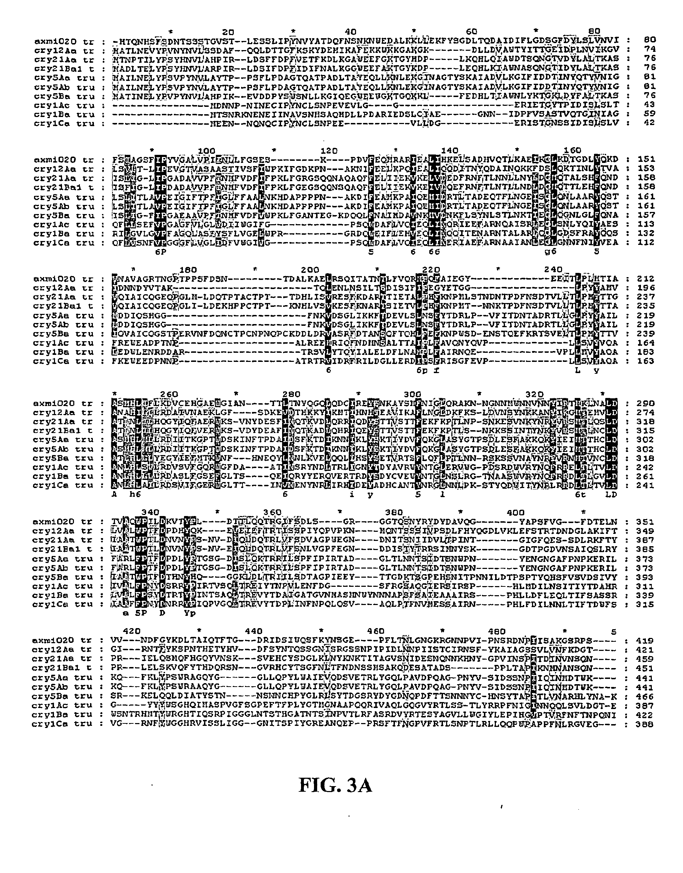

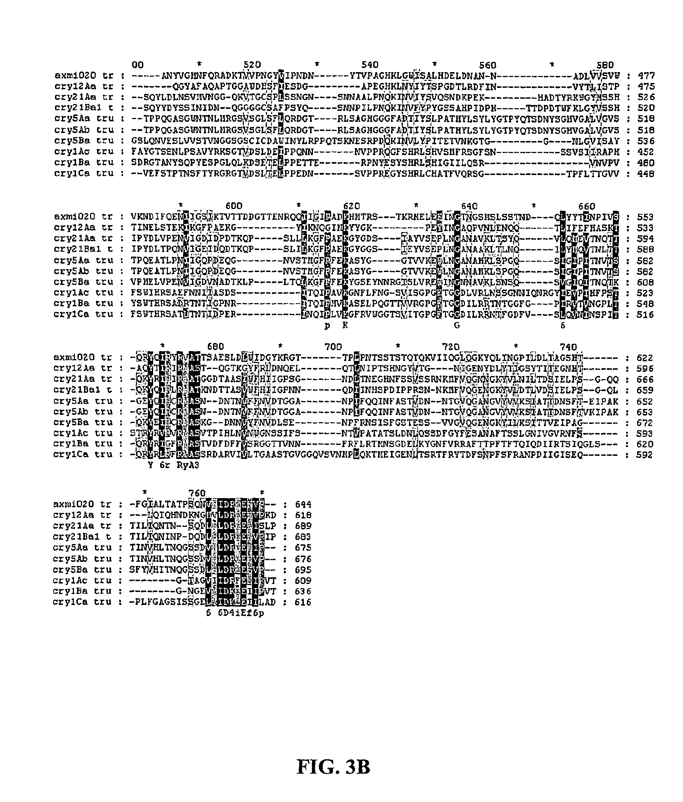

Figures 3A and 3B show an alignment of the truncated portion of AXMI-020 with cry12Aa (SEQ ID NO:13), cry21Aa (SEQ ID NO:14), cry21Ba1 (SEQ ID NO:15), cry5Aa (SEQ ID NO:10), cry5Ab (SEQ ID NO:11), cry5Ba (SEQ ID NO:12), cry1Ac (SEQ ID NO:7), cry1Ba (SEQ ID NO:8), and cry1Ca (SEQ ID NO:9). The alignment shows the most highly conserved amino acid residues highlighted in black, and highly conserved amino acid residues highlighted in gray. Conserved group 1 in AXMI-020 is found from about amino acid residue 200 to about 222 of SEQ ID NO:4. Conserved group 2 is found from about amino acid residue 274 to about 312 of SEQ ID NO:4. Conserved group 3 is found from about amino acid residue 480 to about 533 of SEQ ID NO:4. Conserved group 4 is found from about amino acid residue 550 to about 559 of SEQ ID NO:4. Conserved group 5 is found from about amino acid residue 635 to about 644 of SEQ ID NO:4.

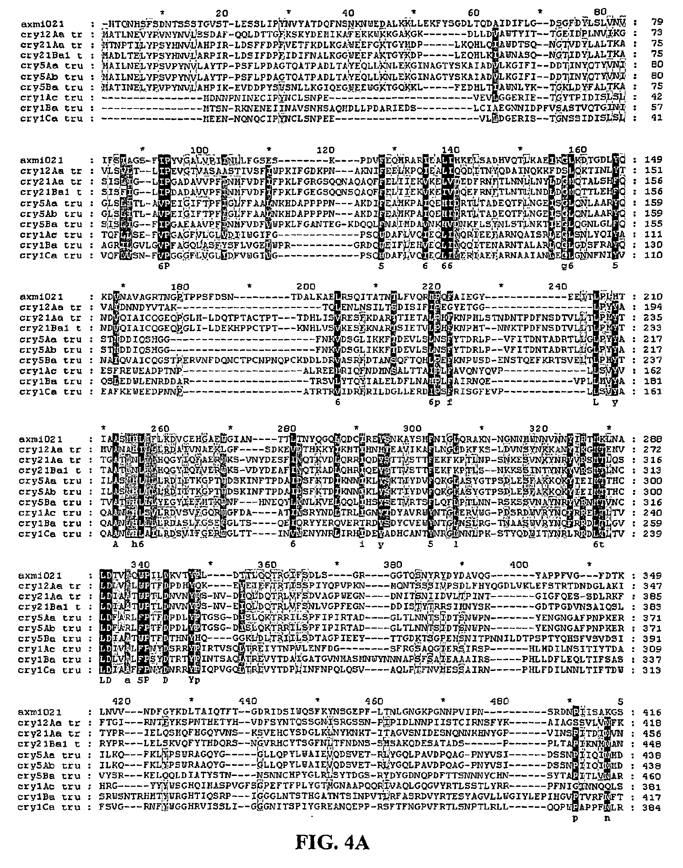

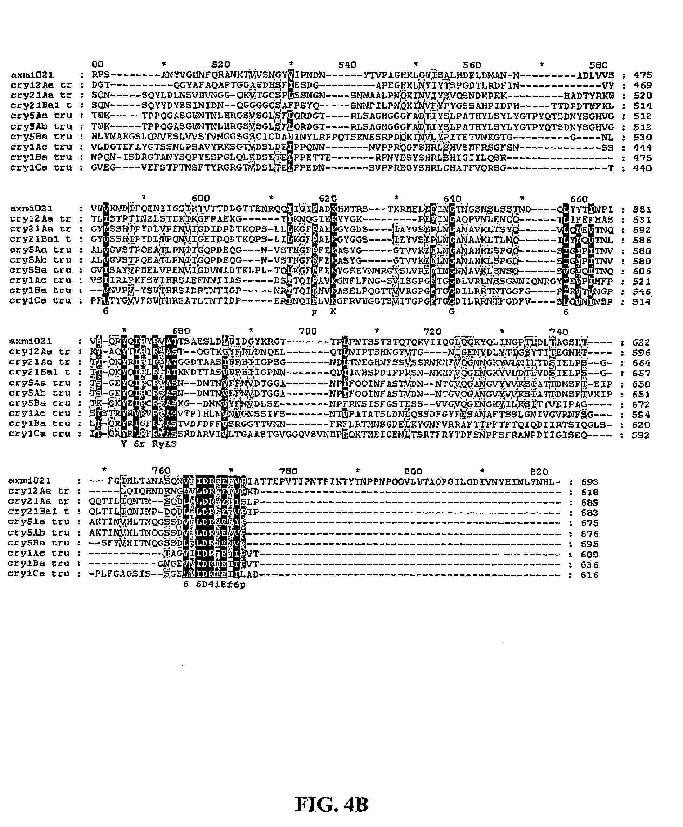

Figures 4A and 4B show an alignment of the truncated portion of AXMI-021 (SEQ ID NO:6) with cry12Aa (SEQ ID NO:13), cry21Aa (SEQ ID NO:14), cry21Ba1 (SEQ ID NO:15), cry5Aa (SEQ ID NO:10), cry5Ab (SEQ ID NO:11), cry5Ba (SEQ ID NO:12), cry1Ac (SEQ ID NO:7), cry1Ba (SEQ ID NO:8), and cry1Ca (SEQ ID NO:9). Toxins having C-terminal non-toxic domains were artificially truncated as shown. The alignment shows the most highly conserved amino acid residues highlighted in black, and highly conserved amino acid residues highlighted in gray. Conserved group 1 in AXMI-021 is found from about amino acid residue 200 to about 222 of SEQ ID NO:6. Conserved group 2 is found from about amino acid residue 274 to about 316 of SEQ ID NO:6. Conserved group 3 is found from about amino acid residue 491 to about 537 of SEQ ID NO:6. Conserved group 4 is found from about amino acid residue 554 to about 564 of SEQ ID NO:6. Conserved group 5 is found from about amino acid residue 635 to about 645 of SEQ ID NO:6.

### DETAILED DESCRIPTION OF THE INVENTION

The present invention is drawn to compositions and methods for regulating pest resistance in organisms, particularly plants or plant cells. The methods involve transforming organisms with a nucleotide sequence encoding a delta-endotoxin protein of the invention. In particular, the nucleotide sequences of the invention are useful for preparing plants and microorganisms that possess pesticidal activity. Thus, transformed bacteria, plants, plant cells, plant tissues and seeds are provided. Compositions are delta-endotoxin nucleic acids and proteins *ofBacillus thuringiensis.* The sequences find use in the construction of expression vectors for subsequent transformation into organisms of interest, as probes for the isolation of other delta-endotoxin genes, and for the generation of altered pesticidal proteins by methods known in the art, such as domain swapping or DNA shuffling. The proteins find use in controlling or killing lepidopteran or coleopteran pest populations and for producing compositions with pesticidal activity.

Plasmids containing the herbicide resistance nucleotide sequences of the invention were deposited in the permanent collection of the Agricultural Research Service Culture Collection, Northern Regional Research Laboratory (NRRL) on January 13, 2005, and assigned Accession Nos. NRRL B-30805, NRRL B-30809, and NRRL B-30808, for AXMI-018, AXMI-020, and AXMI-021, respectively. The plasmid assigned Accession No. NRRL B-30805 contains an insert having nucleotides 1-3605 of AXMI-018 (SEQ ID NO:1). These deposits will be maintained under the terms of the Budapest Treaty on the International Recognition of the Deposit of Microorganisms for the Purposes of Patent Procedure. These deposits were made merely as a convenience for those of skill in the art and are not an admission that a deposit is required under 35 U.S.C. §112.

By "delta-endotoxin" is intended a toxin from *Bacillus thuringiensis* that has toxic activity against one or more pests, including, but not limited to, members of the *Lepidoptera, Diptera,* and *Coleoptera* orders, or a protein that has homology to such a protein. In some cases, delta-endotoxin proteins have been isolated from other organisms, including *Clostridium bifermentans* and *Paenibacillus popilliae.* Delta-endotoxin proteins include amino acid sequences deduced from the full-length nucleotide sequences disclosed herein, and amino acid sequences that are shorter than the full-length sequences, either due to the use of an alternate downstream start site, or due to processing that produces a shorter protein having pesticidal activity. Processing may occur in the organism the protein is expressed in, or in the pest after ingestion of the protein. Delta-endotoxins include proteins identified as *cry1* through *cry43*, *cyt1* and *cyt2,* and Cyt-like toxin. There are currently over 250 known species of delta-endotoxins with a wide range of specificities and toxicities. For an expansive list see Crickmore et al. (1998), Microbiol. Mol. Biol. Rev. 62:807-813, and for regular updates see Crickmore et al. (2003) "Bacillus thuringiensis toxin nomenclature," at www.biols.susx.ac.uk/Home/Neil_Crickmore/Bt/index.

Provided herein are novel isolated nucleotide sequences that confer pesticidal activity. Also provided are the amino acid sequences of the delta-endotoxin proteins. The protein resulting from translation of this gene allows cells to control or kill pests that ingest it.

### Isolated Nucleic Acid Molecules, and Variants and Fragments Thereof

One aspect of the invention pertains to isolated nucleic acid molecules comprising nucleotide sequences encoding delta-endotoxin proteins and polypeptides or biologically active portions thereof, as well as nucleic acid molecules sufficient for use as hybridization probes to identify delta-endotoxin encoding nucleic acids. As used herein, the term "nucleic acid molecule" is intended to include DNA molecules (e.g., cDNA or genomic DNA) and RNA molecules (e.g., mRNA). The nucleic acid molecule can be single-stranded or double-stranded.

An "isolated" or "purified" nucleic acid molecule or protein, or biologically active portion thereof, is substantially free of other cellular material, or culture medium when produced by recombinant techniques, or substantially free of chemical precursors or other chemicals when chemically synthesized. In some embodiments, an "isolated" nucleic acid is free of sequences (such as, for example, protein encoding sequences) that naturally flank the nucleic acid (i.e., sequences located at the 5' and 3' ends of the nucleic acid) in the genomic DNA of the organism from which the nucleic acid is derived. For purposes of the invention, "isolated" when used to refer to nucleic acid molecules excludes isolated chromosomes. For example, in various embodiments, the isolated delta-endotoxin encoding nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. A delta-endotoxin protein that is substantially free of cellular material includes preparations of protein having less than about 30%, 20%, 10%, or 5% (by dry weight) of non-delta-endotoxin protein (also referred to herein as a "contaminating protein").

Nucleotide sequences encoding the proteins of the present invention include the sequence set forth in SEQ ID NO:1, 3, or 5, the delta endotoxin nucleotide sequences deposited in bacterial hosts as Accession Nos. NRRL B-30805, NRRL B-30809, and NRRL B-30808, and complements thereof. By "complement" is intended a nucleotide sequence that is sufficiently complementary to a given nucleotide sequence such that it can hybridize to the given nucleotide sequence to thereby form a stable duplex. The corresponding amino acid sequences for the delta-endotoxin proteins encoded by these nucleotide sequences are set forth in SEQ ID NO:2, 4, or 6.

Nucleic acid molecules that are fragments of these delta-endotoxin encoding nucleotide sequences are also disclosed. By "fragment" is intended a portion of the nucleotide sequence encoding a delta-endotoxin protein. A fragment of a nucleotide sequence may encode a biologically active portion of a delta-endotoxin protein, or it may be a fragment that can be used as a hybridization probe or PCR primer using methods disclosed below. Nucleic acid molecules that are fragments of a delta-endotoxin nucleotide sequence comprise at least about 50, 100, 200, 300, 400, 500, 600, 704, 800, 900, 1000, 1050, 1100, 1150, 1200, 1250, 1300, 1350, 1400, 1450, 1500, 1550, 1600, 1650, 1700, 1750, 1.800, 1850, 1900, 1950, 2000, 2050, 2100, 2150, 2200, 2250, 2300, 2350, 2400, 2450, 2500, 2550, 2600, 2650, 2700, 2750, 2800, 2850, 2900, 2950, 3000, 3050, 3100, 3150, 3200, 3250, 3300, 3350, 3400, 3450, 3500, 3550, 3600, 3650 contiguous nucleotides, or up to the number of nucleotides present in a full-length delta-endotoxin encoding nucleotide sequence disclosed herein (for example, 3675 nucleotides for SEQ ID NO: 1). By "contiguous" nucleotides is intended nucleotide residues that are immediately adjacent to one another. Fragments of the nucleotide sequences of the present invention will encode protein fragments that retain the biological activity of the delta-endotoxin protein and, hence, retain pesticidal activity. By "retains activity" is intended that the fragment will have at least about 30%, at least about 50%, at least about 70%, or at least about 80% of the pesticidal activity of the delta-endotoxin protein. Methods for measuring pesticidal activity are well known in the art. See, for example, Czapla and Lang (1990) JEcon. Entomol. 83:2480-2485; Andrews et al. (1988) Biochem. J. 252:199-206; Marrone et al. (1985) J. of Economic Entomology 78:290-293; and U.S. Patent No. 5,743,477.

A fragment of a delta-endotoxin encoding nucleotide sequence that encodes a biologically active portion of a protein of the invention will encode at least about 15, 25, 30, 50, 75, 100, 125, 150, 175, 200, 250, 300, 350, 400, 450, 500, 550, 600, 650, 700, 750, 800, 850, 900, 950, 1000, 1050, 1100, 1150, or 1200 contiguous amino acids, or up to the total number of amino acids present in a full-length delta-endotoxin protein of the invention (for example, 1224 amino acids for SEQ ID NO: 2).

In some embodiments, delta-endotoxin proteins of the present invention are encoded by a nucleotide sequence sufficiently identical to the nucleotide sequence of SEQ ID NO: 1, 3, or 5. By "sufficiently identical" is intended an amino acid or nucleotide sequence that has at least 80% or 85% sequence identity, about 90%, 91%, 92%, 93%, 94%, 95%, 96%, 97%, 98% or 99% sequence identity compared to a reference sequence using one of the alignment programs described herein using standard parameters. One of skill in the art will recognize that these values can be appropriately adjusted to determine corresponding identity of proteins encoded by two nucleotide sequences by taking into account codon degeneracy, amino acid similarity, reading frame positioning, and the like.

To determine the percent identity of two amino acid sequences or of two nucleic acids, the sequences are aligned for optimal comparison purposes. The percent identity between the two sequences is a function of the number of identical positions shared by the sequences (i.e., percent identity = number of identical positions/total number of positions (e.g., overlapping positions) x 100). In one embodiment, the two sequences are the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the BLASTN and BLASTX programs of Altschul et al. (1990) J. Mol. Biol. 215:403. BLAST nucleotide searches can be performed with the BLASTN program, score = 100, wordlength = 12, to obtain nucleotide sequences homologous to delta-endotoxin-like nucleic acid molecules of the invention. BLAST protein searches can be performed with the BLASTX program, score = 50, wordlength = 3, to obtain amino acid sequences homologous to delta-endotoxin protein molecules of the invention. To obtain gapped alignments for comparison purposes, Gapped BLAST (in BLAST 2.0) can be utilized as described in Altschul et al. (1997) Nucleic Acids Res. 25:3389. Alternatively, PSI-Blast can be used to perform an iterated search that detects distant relationships between molecules. *See* Altschul *et al.* (1997) *supra.* When utilizing BLAST, Gapped BLAST, and PSI-Blast programs, the default parameters of the respective programs (e.g., BLASTX and BLASTN) can be used. Alignment may also be performed manually by inspection.

Another non-limiting example of a mathematical algorithm utilized for the comparison of sequences is the ClustalW algorithm (Higgins et al. (1994) Nucleic Acids Res. 22:4673-4680). ClustalW compares sequences and aligns the entirety of the amino acid or DNA sequence, and thus can provide data about the sequence conservation of the entire amino acid sequence. The ClustalW algorithm is used in several commercially available DNA/amino acid analysis software packages, such as the ALIGNX module of the Vector NTI Program Suite (Invitrogen Corporation, Carlsbad, CA). After alignment of amino acid sequences with ClustalW, the percent amino acid identity can be assessed. A non-limiting example of a software program useful for analysis of ClustalW alignments is GeneDoc(TM). Genedoc(TM) (Karl Nicholas) allows assessment of amino acid (or DNA) similarity and identity between multiple proteins. Another non-limiting example of a mathematical algorithm utilized for the comparison of sequences is the algorithm of Myers and Miller (1988) CABIOS 4:11- 17. Such an algorithm is incorporated into the ALIGN program (version 2.0), which is part of the GCG Wisconsin Genetics Software Package, Version 10 (Accelrys, Inc., San Diego, CA). When utilizing the ALIGN program for comparing amino acid sequences, a PAM120 weight residue table, a gap length penalty of 12, and a gap penalty of 4 can be used.

Unless otherwise stated, GAP Version 10, which uses the algorithm of Needleman and Wunsch (1970) J. MoI. Biol. 48(3):443-453, will be used to determine sequence identity or similarity using the following parameters: % identity and % similarity for a nucleotide sequence using GAP Weight of 50 and Length Weight of 3, and the nwsgapdna.cmp scoring matrix; % identity or % similarity for an amino acid sequence using GAP weight of 8 and length weight of 2, and the BLOSUM62 scoring program. Equivalent programs may also be used. By "equivalent program" is intended any sequence comparison program that, for any two sequences in question, generates an alignment having identical nucleotide residue matches and an identical percent sequence identity when compared to the corresponding alignment generated by GAP Version 10. The invention also encompasses variant nucleic acid molecules selected from the group consisting of: (a) a nucleic acid molecule comprising a nucleotide sequence having at least 80% sequence identity to the nucleotide sequence of SEQ ID NO: 1, 3, or 5, or a complement thereof, wherein said nucleotide sequence encodes a polypeptide having pesticidal activity; and (b) a nucleic acid molecule which encodes a polypeptide comprising an amino acid sequence having at least 80% sequence identity to the amino acid sequence of SEQ ID NO: 2, 4, or 6, wherein said nucleotide sequence encodes a polypeptide having pesticidal activity. "Variants" of the delta-endotoxin encoding nucleotide sequences include those sequences that encode the delta-endotoxin proteins disclosed herein but that differ conservatively because of the degeneracy of the genetic code as well as those that are sufficiently identical as discussed above. Naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the delta-endotoxin proteins disclosed in the present invention as discussed below. Variant proteins encompassed by the present invention are biologically active, that is they continue to possess the desired biological activity of the native protein, that is, retaining pesticidal activity. By "retains activity" is intended that the variant will have at least about 30%, at least about 50%, at least about 70%, or at least about 80% of the pesticidal activity of the native protein. Methods for measuring pesticidal activity are well known in the art. See, for example, Czapla and Lang (1990) J. Econ. Entomol. 83: 2480-2485; Andrews et al, (1988) Biochem. J. 252:199-206; Marrone et al. (1985) J. of Economic Entomology 78:290-293; and U.S. Patent No. 5,743,477.

The skilled artisan will further appreciate that changes can be introduced by mutation into the nucleotide sequences of the invention thereby leading to changes in the amino acid sequence of the encoded delta-endotoxin proteins, without altering the biological activity of the proteins. Thus, variant isolated nucleic acid molecules can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present invention provided they are selected from the group consisting of: (a) a nucleic acid molecule comprising a nucleotide sequence having at least 80% sequence identity to the nucleotide sequence of SEQ ID NO: 1, 3, or 5, or a complement thereof, wherein said nucleotide sequence encodes a polypeptide having pesticidal activity; and (b) a nucleic acid molecule which encodes a polypeptide comprising an amino acid sequence having at least 80% sequence identity to the amino acid sequence of SEQ ID NO: 2, 4, or 6, wherein said nucleotide sequence encodes a polypeptide having pesticidal activity.

For example, conservative amino acid substitutions may be made at one or more predicted, nonessential amino acid residues. A "nonessential" amino acid residue is a residue that can be altered from the wild-type sequence of a delta-endotoxin protein without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Delta-endotoxins generally have five conserved sequence domains, and three conserved structural domains (see, for example, de Maagd et al. (2001) Trends Genetics 17:193-199). The first conserved structural domain consists of seven alpha helices and is involved in membrane insertion and pore formation. Domain II consists of three beta-sheets arranged in a Greek key configuration, and domain III consists of two antiparallel beta-sheets in "jelly-roll" formation (de Maagd *et al*., 2001, *supra*)*.* Domains II and III are involved in receptor recognition and binding, and are therefore considered determinants of toxin specificity.

Amino acid substitutions may be made in nonconserved regions that retain function. In general, such substitutions would not be made for conserved amino acid residues, or for amino acid residues residing within a conserved motif, where such residues are essential for protein activity. Examples of residues that are conserved and that may be essential for protein activity include, for example, residues that are identical between all proteins contained in the alignments of Figures 1, 2, 3, and 4. Examples of residues that are conserved but that may allow conservative amino acid substitutions and still retain activity include, for example, residues that have only conservative substitutions between all proteins contained in the alignments of Figures 1, 2, 3, and 4. However, one of skill in the art would understand that functional variants may have minor conserved or nonconserved alterations in the conserved residues.

Alternatively, variant nucleotide sequences can be made by introducing mutations randomly along all or part of the coding sequence, such as by saturation mutagenesis, and the resultant mutants can be screened for the ability to confer delta-endotoxin activity to identify mutants that retain activity. Following mutagenesis, the encoded protein can be expressed recombinantly, and the activity of the protein can be determined using standard assay techniques.

Using methods such as PCR, hybridization, and the like corresponding delta-endotoxin sequences can be identified, such sequences having substantial identity to the sequences of the invention. *See,* for example, Sambrook J., and Russell, D.W. (2001) Molecular Cloning: A Laboratory Manual. (Cold Spring Harbor Laboratory Press, Cold Spring Harbor, NY) and Innis, et al. (1990) PCR Protocols: A Guide to Methods and Applications (Academic Press, NY).

In a hybridization method, all or part of the delta-endotoxin nucleotide sequence can be used to screen cDNA or genomic libraries. Methods for construction of such cDNA and genomic libraries are generally known in the art and are disclosed in Sambrook and Russell, 2001, *supra.* The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known delta-endotoxin-encoding nucleotide sequence disclosed herein. Degenerate primers designed on the basis of conserved nucleotides or amino acid residues in the nucleotide sequence or encoded amino acid sequence can additionally be used. The probe typically comprises a region of nucleotide sequence that hybridizes under stringent conditions to at least about 12, about 25, at least about 50, 75, 100, 125, 150, 175, 200, 250, 300, 350, or 400 consecutive nucleotides of delta-endotoxin encoding nucleotide sequence(s) of the invention or a fragment or variant thereof. Methods for the preparation of probes for hybridization are generally known in the art and are disclosed in Sambrook and Russell, 2001, *supra*.

For example, an entire delta-endotoxin sequence disclosed herein, or one or more portions thereof, may be used as a probe capable of specifically hybridizing to corresponding delta-endotoxin-like sequences and messenger RNAs. To achieve specific hybridization under a variety of conditions, such probes include sequences that are unique and are at least about 10 nucleotides in length, or at least about 20 nucleotides in length. Such probes may be used to amplify corresponding delta-endotoxin sequences from a chosen organism by PCR. This technique may be used to isolate additional coding sequences from a desired organism or as a diagnostic assay to determine the presence of coding sequences in an organism. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Cold Spring Harbor, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length, or less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulfate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Optionally, wash buffers may comprise about 0.1% to about 1% SDS. Duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), the SSC concentration can be increased so that a higher temperature can be used. An extensive guide to the hybridization of nucleic acids is found in Tijssen (1993) Laboratory Techniques in Biochemistry and Molecular Biology— Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Cold Spring Harbor, NY).

### Isolated Proteins and Variants and Fragments Thereof

Delta-endotoxin proteins are also encompassed within the present invention. By "delta-endotoxin protein" is intended a protein having the amino acid sequence set forth in SEQ ID No: 2, 4, or 6. Fragments, biologically active portions, and variants thereof are also provided which have at least 80% sequence identity to the amino acid sequence of SEQ ID No: 2, 4 or 6 and which have pesticidal activity, and may be used to practice the methods of the present invention.

"Fragments" or "biologically active portions" include polypeptide fragments comprising amino acid sequences sufficiently identical to the amino acid sequence set forth in SEQ ID No: 2, 4, or 6, and that exhibit pesticidal activity. Such "fragments" or "biologically active portions" have at least 80% sequence identity to the amino acid sequence of SEQ ID No: 2, 4 or 6. Such biologically active portions can be prepared by recombinant techniques and evaluated for pesticidal activity. Methods for measuring pesticidal activity are well known in the art. See, for example, Czapla and Lang (1990) J. Econ. Entomol. 83:2480-2485; Andrews et al. (1988) Biochem. J. 252:199-206; Marrone et al. (1985) J. of Economic Entomology 78:290-293; and U.S. Patent No. 5,743,477. As used herein, a fragment comprises at least 8 contiguous amino acids of SEQ ID No: 2, 4, or 6. The invention encompasses other fragments, however, such as any fragment in the protein greater than about 600, 650, 700, 750, 800, 850, 900, 950, 1000, 1050, 1100, 1150, or 1200 amino acids.

By "variants" is intended proteins or polypeptides having an amino acid sequence that is at least 80%, 85%, 90%, 91%, 92%, 93%, 94%, 95%, 96%, 97%, 98% or 99% identical to the amino acid sequence of SEQ ID No: 2, 4, or 6. Such variants may also be encoded by a nucleic acid molecule that hybridizes to the nucleic acid molecule of SEQ ID No: 1, 3, or 5, or a complement thereof, under stringent conditions. Such variants will retain pesticidal activity. Variants include polypeptides that differ in amino acid sequence due to mutagenesis but which have at least 80% sequence identity to the amino acid sequence of SEQ ID No: 2, 4 or 6. Variant proteins encompassed by the present invention are biologically active, that is they continue to possess the desired biological activity of the native protein, that is, retaining pesticidal activity. Methods for measuring pesticidal activity are well known in the art. See, for example, Czapla and Lang (1990) J. Econ. Entomol. 83:2480-2485; Andrews et al. (1988) Biochem. J. 252:199-206; Marrone et al. (1985) J. of Economic Entomology 78:290-293; and U.S. Patent No. 5,743,477.

Bacterial genes, such as the AXMI-018, AXMI-020, and AXMI-021 genes of this invention, quite often possess multiple methionine initiation codons in proximity to the start of the open reading frame. Often, translation initiation at one or more of these start codons will lead to generation of a functional protein. These start codons can include ATG codons. However, bacteria such as *Bacillus sp.* also recognize the codon GTG as a start codon, and proteins that initiate translation at GTG codons contain a methionine at the first amino acid. Furthermore, it is not often determined a *priori* which of these codons are used naturally in the bacterium. Thus, it is understood that use of one of the alternate methionine codons may also lead to generation of delta-endotoxin proteins that encode pesticidal activity. These delta-endotoxin proteins are encompassed in the present invention and may be used in the methods of the present invention.

Antibodies to the polypeptides of the present invention, or to variants or fragments thereof, are also encompassed. Methods for producing antibodies are well known in the art (see, for example, Harlow and Lane (1988) Antibodies: A Laboratory Manual, Cold Spring Harbor Laboratory, Cold Spring Harbor, NY; U.S. Patent No. 4,196,265).

### Altered or Improved Variants

It is recognized that DNA sequences of a delta-endotoxin may be altered by various methods, and that these alterations may result in DNA sequences encoding proteins with amino acid sequences different than that encoded by a delta-endotoxin of the present invention. This protein may be altered in various ways including amino acid substitutions, deletions, truncations, and insertions. Methods for such manipulations are generally known in the art. For example, amino acid sequence variants of a delta-endotoxin protein can be prepared by mutations in the DNA. This may also be accomplished by one of several forms of mutagenesis and/or in directed evolution. In some aspects, the changes encoded in the amino acid sequence will not substantially affect the function of the protein. Such variants will possess the desired pesticidal activity. However, it is understood that the ability of a delta-endotoxin to confer pesticidal activity may be improved by the use of such techniques upon the compositions of this invention. For example, one may express a delta-endotoxin in host cells that exhibit high rates of base misincorporation during DNA replication, such as XL-1 Red (Stratagene, La Jolla, CA). After propagation in such strains, one can isolate the delta-endotoxin DNA (for example by preparing plasmid DNA, or by amplifying by PCR and cloning the resulting PCR fragment into a vector), culture the delta-endotoxin mutations in a non-mutagenic strain, and identify mutated delta-endotoxin genes with pesticidal activity, for example by performing an assay to test for pesticidal activity. Generally, the protein is mixed and used in feeding assays. See, for example Marrone et al. (1985) J. of Economic Entomology 78:290-293. Such assays can include contacting plants with one or more pests and determining the plant's ability to survive and/or cause the death of the pests. Examples of mutations that result in increased toxicity are found in Schnepf et al. (1998) Microbiol. Mol. Biol. Rev. 62:775-806.

Alternatively, alterations may be made to the protein sequence of many proteins at the amino or carboxy terminus without substantially affecting activity. These alterations can include insertions, deletions, or alterations introduced by modem molecular methods, such as PCR, including PCR amplifications that alter or extend the protein coding sequence by virtue of inclusion of amino acid encoding sequences in the oligonucleotides utilized in the PCR amplification. Alternatively, the protein sequences added can include entire protein-coding sequences, such as those used commonly in the art to generate protein fusions. Such fusion proteins are often used to (1) increase expression of a protein of interest (2) introduce a binding domain, enzymatic activity, or epitope to facilitate either protein purification, protein detection, or other experimental uses known in the art (3) target secretion or translation of a protein to a subcellular organelle, such as the periplasmic space of Gram-negative bacteria, or the endoplasmic reticulum of eukaryotic cells, the latter of which often results in glycosylation of the protein.

Variant nucleotide and amino acid sequences of the present invention also encompass sequences derived from mutagenic and recombinogenic procedures such as DNA shuffling. With such a procedure, one or more different delta-endotoxin protein coding regions can be used to create a new delta-endotoxin protein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between a delta-endotoxin gene of the invention and other known delta-endotoxin genes to obtain a new gene coding for a protein with an improved property of interest, such as an increased insecticidal activity. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad. Sci. USA 94:4504-4509; Crameri et al. (1998) Nature 391:288-291; and U.S. Patent Nos. 5,605,793 and 5,837,458.

Domain swapping or shuffling is another mechanism for generating altered delta-endotoxin proteins. Domains II and III may be swapped between delta-endotoxin proteins, resulting in hybrid or chimeric toxins with improved pesticidal activity or target spectrum. Methods for generating recombinant proteins and testing them for pesticidal activity are well known in the art (see, for example, Naimov et al. (2001) Appl. Environ. Microbiol. 67:5328-5330; de Maagd et al. (1996) Appl. Environ. Microbiol. 62:1537-1543; Ge et al. (1991) J. Biol. Chem. 266:17954-17958; Schnepf et al. (1990) J. Biol. Chem. 265:20923-20930; Rang et al. (1999) Appl. Environ. Microbiol. 65:2918-2925).

### Vectors

A delta-endotoxin sequence of the invention may be provided in an expression cassette for expression in a plant of interest. By "plant expression cassette" is intended a DNA construct that is capable of resulting in the expression of a protein from an open reading frame in a plant cell. Typically these casssettes contain a promoter and a coding sequence. Often, such constructs will also contain a 3' untranslated region. Such constructs may contain a "signal sequence" or "leader sequence" to facilitate co-translational or post-translational transport of the peptide to certain intracellular structures such as the chloroplast (or other plastid), endoplasmic reticulum, or Golgi apparatus.

By "signal sequence" is intended a sequence that is known or suspected to result in cotranslational or post-translational peptide transport across the cell membrane. In eukaryotes, this transport typically involves secretion into the Golgi apparatus, with some resulting glycosylation. By "leader sequence" is intended any sequence that, when translated, results in an amino acid sequence sufficient to trigger co-translational transport of the peptide chain to a sub-cellular organelle. Thus, this includes leader sequences targeting transport and/or glycosylation by passage into the endoplasmic reticulum, passage to vacuoles, plastids including chloroplasts, mitochondria, and the like.

By "plant transformation vector" is intended a DNA molecule that is necessary for efficient transformation of a plant cell. Such a molecule may consist of one or more plant expression cassettes, and may be organized into more than one "vector" DNA molecule. For example, binary vectors are plant transformation vectors that utilize two non-contiguous DNA vectors to encode all requisite cis- and trans-acting functions for transformation of plant cells (Hellens and Mullineaux (2000) Trends in Plant Science 5:446-451). "Vector" refers to a nucleic acid construct designed for transfer between different host cells. "Expression vector" refers to a vector that has the ability to incorporate, integrate and express heterologous DNA sequences or fragments in a foreign cell. The cassette will include 5' and 3' regulatory sequences operably linked to a sequence of the invention. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

"Promoter" refers to a nucleic acid sequence that functions to direct transcription of a downstream coding sequence. The promoter together with other transcriptional and translational regulatory nucleic acid sequences (also termed "control sequences") are necessary for the expression of a DNA sequence of interest.

Such an expression cassette is provided with a plurality of restriction sites for insertion of the delta-endotoxin sequence to be under the transcriptional regulation of the regulatory regions.

The expression cassette will include in the 5'-3' direction of transcription, a transcriptional and translational initiation region (i.e., a promoter), a DNA sequence of the invention, and a translational and transcriptional termination region (i.e., termination region) functional in plants. The promoter may be native or analogous, or foreign or heterologous, to the plant host and/or to the DNA sequence of the invention. Additionally, the promoter may be the natural sequence or alternatively a synthetic sequence. Where the promoter is "native" or "analogous" to the plant host, it is intended that the promoter is found in the native plant into which the promoter is introduced. Where the promoter is "foreign" or "heterologous" to the DNA sequence of the invention, it is intended that the promoter is not the native or naturally occurring promoter for the operably linked DNA sequence of the invention.

The termination region may be native with the transcriptional initiation region, may be native with the operably linked DNA sequence of interest, may be native with the plant host, or may be derived from another source (i.e., foreign or heterologous to the promoter, the DNA sequence of interest, the plant host, or any combination thereof). Convenient termination regions are available from the Ti-plasmid of *A*. *tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed host cell. That is, the genes can be synthesized using host cell-preferred codons for improved expression, or may be synthesized using codons at a host-preferred codon usage frequency. Generally, the GC content of the gene will be increased. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic Acids Res. 17:477-498.

In one embodiment, the delta-endotoxin is targeted to the chloroplast for expression. In this manner, where the delta-endotoxin is not directly inserted into the chloroplast, the expression cassette will additionally contain a nucleic acid encoding a transit peptide to direct the delta-endotoxin to the chloroplasts. Such transit peptides are known in the art. See, for example, Von Heijne et al. (1991) Plant Mol. Biol. Rep. 9:104-126; Clark et al. (1989) J. Biol. Chem. 264:17544-17550; Delta-Cioppa et al. (1987) Plant Physiol. 84:965-968; Romer et al. (1993) Biochem. Biophys. Res. Commun. 196:1414-1421; and Shah et al. (1986) Science 233:478-481.

The delta-endotoxin gene to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831.

### Plant Transformation

Methods of the invention involve introducing a nucleotide construct into a plant. By "introducing" is intended to present to the plant the nucleotide construct in such a manner that the construct gains access to the interior of at least one cell of the plant. The methods of the invention do not require that a particular method for introducing a nucleotide construct to a plant be used, only that the nucleotide construct gains access to the interior of at least one cell of the plant. Methods for introducing nucleotide constructs into plants are known in the art including, but not limited to, stable transformation methods, transient transformation methods, and virus-mediated methods.

By "plant" is intended whole plants, plant organs (e.g., leaves, stems, roots, etc.), seeds, plant cells, propagules, embryos and progeny of the same. Plant cells can be differentiated or undifferentiated (e.g. callus, suspension culture cells, protoplasts, leaf cells, root cells, phloem cells, pollen).

"Transgenic plants" or "transformed plants" or "stably transformed" plants or cells or tissues refers to plants that have incorporated or integrated exogenous nucleic acid sequences or DNA fragments into the plant cell. These nucleic acid sequences include those that are exogenous, or not present in the untransformed plant cell, as well as those that may be endogenous, or present in the untransformed plant cell. "Heterologous" generally refers to the nucleic acid sequences that are not endogenous to the cell or part of the native genome in which they are present, and have been added to the cell by infection, transfection, microinjection, electroporation, microprojection, or the like.

Transformation of plant cells can be accomplished by one of several techniques known in the art. The delta-endotoxin gene of the invention may be modified to obtain or enhance expression in plant cells. Typically a construct that expresses such a protein would contain a promoter to drive transcription of the gene, as well as a 3' untranslated region to allow transcription termination and polyadenylation. The organization of such constructs is well known in the art. In some instances, it may be useful to engineer the gene such that the resulting peptide is secreted, or otherwise targeted within the plant cell. For example, the gene can be engineered to contain a signal peptide to facilitate transfer of the peptide to the endoplasmic reticulum. It may also be preferable to engineer the plant expression cassette to contain an intron, such that mRNA processing of the intron is required for expression.

Typically this "plant expression cassette" will be inserted into a "plant transformation vector." This plant transformation vector may be comprised of one or more DNA vectors needed for achieving plant transformation. For example, it is a common practice in the art to utilize plant transformation vectors that are comprised of more than one contiguous DNA segment. These vectors are often referred to in the art as "binary vectors." Binary vectors as well as vectors with helper plasmids are most often used for *Agrobacterium-*mediated transformation, where the size and complexity of DNA segments needed to achieve efficient transformation is quite large, and it is advantageous to separate functions onto separate DNA molecules. Binary vectors typically contain a plasmid vector that contains the cis-acting sequences required for T-DNA transfer (such as left border and right border), a selectable marker that is engineered to be capable of expression in a plant cell, and a "gene of interest" (a gene engineered to be capable of expression in a plant cell for which generation of transgenic plants is desired). Also present on this plasmid vector are sequences required for bacterial replication. The cis-acting sequences are arranged in a fashion to allow efficient transfer into plant cells and expression therein. For example, the selectable marker gene and the delta-endotoxin are located between the left and right borders. Often a second plasmid vector contains the trans-acting factors that mediate T-DNA transfer from *Agrobacterium* to plant cells. This plasmid often contains the virulence functions (Vir genes) that allow infection of plant cells by *Agrobacterium,* and transfer of DNA by cleavage at border sequences and vir-mediated DNA transfer, as in understood in the art (Hellens and Mullineaux (2000) Trends in Plant Science 5 :446-451)*.* Several types of *Agrobacterium* strains (e.g. LBA4404, GV3101, EHA101, EHA105, etc.) can be used for plant transformation. The second plasmid vector is not necessary for transforming the plants by other methods such as microprojection, microinjection, electroporation, polyethylene glycol, etc.

In general, plant transformation methods involve transferring heterologous DNA into target plant cells (e.g. immature or mature embryos, suspension cultures, undifferentiated callus, protoplasts, etc.), followed by applying a maximum threshold level of appropriate selection (depending on the selectable marker gene) to recover the transformed plant cells from a group of untransformed cell mass. Explants are typically transferred to a fresh supply of the same medium and cultured routinely. Subsequently, the transformed cells are differentiated into shoots after placing on regeneration medium supplemented with a maximum threshold level of selecting agent. The shoots are then transferred to a selective rooting medium for recovering rooted shoot or plantlet. The transgenic plantlet then grows into a mature plant and produces fertile seeds (e.g. Hiei et al. (1994) The Plant Journal 6:271-282; Ishida et al. (1996) Nature Biotechnology 14:745-750). Explants are typically transferred to a fresh supply of the same medium and cultured routinely. A general description of the techniques and methods for generating transgenic plants are found in Ayres and Park (1994) Critical Reviews in Plant Science 13:219-239 and Bommineni and Jauhar (1997) Maydica 42:107-120. Since the transformed material contains many cells; both transformed and non-transformed cells are present in any piece of subjected target callus or tissue or group of cells. The ability to kill non-transformed cells and allow transformed cells to proliferate results in transformed plant cultures. Often, the ability to remove non-transformed cells is a limitation to rapid recovery of transformed plant cells and successful generation of transgenic plants.

Transformation protocols as well as protocols for introducing nucleotide sequences into plants may vary depending on the type of plant or plant cell, i.e., monocot or dicot, targeted for transformation. Generation of transgenic plants may be performed by one of several methods, including, but not limited to, microinjection, electroporation, direct gene transfer, introduction of heterologous DNA by *Agrobacterium* into plant cells (*Agrobacterimn-mediated* transformation), bombardment of plant cells with heterologous foreign DNA adhered to particles, ballistic particle acceleration, aerosol beam transformation (U.S. Published Application No. 20010026941; U.S. Patent No. 4,945,050; International Publication No. WO 91/00915; U.S. Published Application No. 2002015066), Lec1 transformation, and various other non-particle direct-mediated methods to transfer DNA.

Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

Following integration of heterologous foreign DNA into plant cells, one then applies a maximum threshold level of appropriate selection in the medium to kill the untransformed cells and separate and proliferate the putatively transformed cells that survive from this selection treatment by transferring regularly to a fresh medium. By continuous passage and challenge with appropriate selection, one identifies and proliferates the cells that are transformed with the plasmid vector. Molecular and biochemical methods can then be used to confirm the presence of the integrated heterologous gene(s) of interest into the genome of the transgenic plant.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a nucleotide construct of the invention, for example, an expression cassette of the invention, stably incorporated into their genome.

### Evaluation of Plant Transformation

Following introduction of heterologous foreign DNA into plant cells, the transformation or integration of the heterologous gene(s) in the plant genome is confirmed by various methods such as analysis of nucleic acids, proteins and metabolites associated with the integrated gene.

PCR analysis is a rapid method to screen transformed cells, tissue or shoots for the presence of incorporated gene(s) at the earlier stage before transplanting into the soil (Sambrook and Russell (2001) Molecular Clowning: A Laboratory Manual, Cold Spring Harbor Laboratory Press, Cold Spring Harbor, NY). PCR is carried out using oligonucleotide primers specific to the gene of interest or *Agrobacterium* vector background, etc.

Plant transformation may be confirmed by Southern blot analysis of genomic DNA (Sambrook and Russell, 2001, *supra*). In general, total DNA is extracted from the transformant, digested with appropriate restriction enzymes, fractionated in an agarose gel and transferred to a nitrocellulose or nylon membrane. The membrane or "blot" is subsequently probed with, for example, radiolabeled ³²P target DNA fragment to confirm the integration of introduced gene in the plant genome according to standard techniques (Sambrook and Russell, 2001, *supra*).

In Northern blot analysis, RNA is isolated from specific tissues of transformant, fractionated in a formaldehyde agarose gel, and blotted onto a nylon filter according to standard procedures that are routinely used in the art (Sambrook and Russell, 2001, *supra*)*.* Expression of RNA encoded by the delta-endotoxin is then tested by hybridizing the filter to a radioactive probe derived from a delta-endotoxin, by methods known in the art (Sambrook and Russell, 2001, *supra*)*.*

Western blot and biochemical assays and the like may be carried out on the transgenic plants to confirm the presence of protein encoded by the delta-endotoxin gene by standard procedures (Sambrook and Russell, 2001, *supra)* using antibodies that bind to one or more epitopes present on the delta-endotoxin protein.

### Pesticidal Activity in Plants

In another aspect of the invention, one may generate transgenic plants expressing a delta-endotoxin that has pesticidal activity. Methods described above by way of example may be utilized to generate transgenic plants, but the manner in which the transgenic plant cells are generated is not critical to this invention. Methods known or described in the art such as *Agrobacterium-*mediated transformation, biolistic transformation, and non-particle-mediated methods may be used at the discretion of the experimenter. Plants expressing a delta-endotoxin may be isolated by common methods described in the art, for example by transformation of callus, selection of transformed callus, and regeneration of fertile plants from such transgenic callus. In such process, one may use any gene as a selectable marker so long as its expression in plant cells confers ability to identify or select for transformed cells.

A number of markers have been developed for use with plant cells, such as resistance to chloramphenicol, the aminoglycoside G418, hygromycin, or the like. Other genes that encode a product involved in metabolism may also be used as selectable markers. For example, genes that provide resistance to plant herbicides such as glyphosate, bromoxynil, or imidazolinone may find particular use. Such genes have been reported (Stalker et al. (1985) J. Biol. Chem. 263:6310-6314 (bromoxynil resistance nitrilase gene); and Sathasivan et al. (1990) Nucl. Acids Res. 18:2188 (AHAS imidazolinone resistance gene).

Fertile plants expressing a delta-endotoxin may be tested for pesticidal activity, and the plants showing optimal activity selected for further breeding. Methods are available in the art to assay for pest activity. Generally, the protein is mixed and used in feeding assays. See, for example Marrone et al. (1985) J. of Economic Entomology 78:290-293.

The present invention may be used for transformation of any plant species, including, but not limited to, monocots and dicots. Examples of plants of interest include, but are not limited to, corn (maize), sorghum, wheat, sunflower, tomato, crucifers, peppers, potato, cotton, rice, soybean, sugarbeet, sugarcane, tobacco, barley, and oilseed rape, *Brassica* sp., alfalfa, rye, millet, safflower, peanuts, sweet potato, cassava, coffee, coconut, pineapple, citrus trees, cocoa, tea, banana, avocado, fig, guava, mango, olive, papaya, cashew, macadamia, almond, oats, vegetables, ornamentals, and conifers.

Vegetables include, but are not limited to, tomatoes, lettuce, green beans, lima beans, peas, and members of the genus *Curcumis* such as cucumber, cantaloupe, and musk melon. Ornamentals include, but are not limited to, azalea, hydrangea, hibiscus, roses, tulips, daffodils, petunias, carnation, poinsettia , and chrysanthemum. In some embodiments, plants of the present invention are crop plants (for example, maize, sorghum, wheat, sunflower, tomato, crucifers, peppers, potato, cotton, rice, soybean, sugarbeet, sugarcane, tobacco, barley, oilseed rape., etc.).

### Use in Pesticidal Control

General methods for employing strains comprising a nucleotide sequence of the present invention, or a variant thereof, in pesticide control or in engineering other organisms as pesticidal agents are known in the art. See, for example U.S. Patent No. 5,039,523 and EP 0480762A2.

The *Bacillus* strains containing a nucleotide sequence of the present invention, or a variant thereof, or the microorganisms that have been genetically altered to contain a pesticidal gene and protein may be used for protecting agricultural crops and products from pests. In one aspect of the invention, whole, i.e., unlysed, cells of a toxin (i.e., pesticide)-producing organism are treated with reagents that prolong the activity of the toxin produced in the cell when the cell is applied to the environment of target pest(s).

Alternatively, the pesticide is produced by introducing a delta-endotoxin gene into a cellular host. Expression of the delta-endotoxin gene results, directly or indirectly, in the intracellular production and maintenance of the pesticide. In one aspect of this invention, these cells are then treated under conditions that prolong the activity of the toxin produced in the cell when the cell is applied to the environment of target pest(s). The resulting product retains the toxicity of the toxin. These naturally encapsulated pesticides may then be formulated in accordance with conventional techniques for application to the environment hosting a target pest, e.g., soil, water, and foliage of plants. See, for example EPA 0192319, and the references cited therein. Alternatively, one may formulate the cells expressing a gene of this invention such as to allow application of the resulting material as a pesticide.

The active ingredients of the present invention are normally applied in the form of compositions and can be applied to the crop area or plant to be treated, simultaneously or in succession, with other compounds. These compounds can be fertilizers, weed killers, cryoprotectants, surfactants, detergents, pesticidal soaps, dormant oils, polymers, and/or time-release or biodegradable carrier formulations that permit long-term dosing of a target area following a single application of the formulation. They can also be selective herbicides, chemical insecticides, virucides, microbicides, amoebicides, pesticides, fungicides, bacteriocides, nematocides, molluscides or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers. Likewise the formulations may be prepared into edible "baits" or fashioned into pest "traps" to permit feeding or ingestion by a target pest of the pesticidal formulation.

Methods of applying an active ingredient of the present invention or an agrochemical composition of the present invention that contains at least one of the pesticidal proteins produced by the bacterial strains of the present invention include leaf application, seed coating and soil application. The number of applications and the rate of application depend on the intensity of infestation by the corresponding pest.

The composition may be formulated as a powder, dust, pellet, granule, spray, emulsion, colloid, solution, or such like, and may be prepared by such conventional means as desiccation, lyophilization, homogenation, extraction, filtration, centrifugation, sedimentation, or concentration of a culture of cells comprising the polypeptide. In all such compositions that contain at least one such pesticidal polypeptide, the polypeptide may be present in a concentration of from about 1% to about 99% by weight.

Lepidopteran or coleopteran pests may be killed or reduced in numbers in a given area by the methods of the invention, or may be prophylactically applied to an environmental area to prevent infestation by a susceptible pest. In some embodiments, the pest ingests, or is contacted with, a pesticidally-effective amount of the polypeptide. By "pesticidally-effective amount" is intended an amount of the pesticide that is able to bring about death to at least one pest, or to noticeably reduce pest growth, feeding, or normal physiological development. This amount will vary depending on such factors as, for example, the specific target pests to be controlled, the specific environment, location, plant, crop, or agricultural site to be treated, the environmental conditions, and the method, rate, concentration, stability, and quantity of application of the pesticidally-effective polypeptide composition. The formulations may also vary with respect to climatic conditions, environmental considerations, and/or frequency of application and/or severity of pest infestation.

The pesticide compositions described may be made by formulating either the bacterial cell, crystal and/or spore suspension, or isolated protein component with the desired agriculturally-acceptable carrier. The compositions may be formulated prior to administration in an appropriate means such as lyophilized, freeze-dried, desiccated, or in an aqueous carrier, medium or suitable diluent, such as saline or other buffer. The formulated compositions may be in the form of a dust or granular material, or a suspension in oil (vegetable or mineral), or water or oil/water emulsions, or as a wettable powder, or in combination with any other carrier material suitable for agricultural application. Suitable agricultural carriers can be solid or liquid and are well known in the art. The term "agriculturally-acceptable carrier" covers all adjuvants, inert components, dispersants, surfactants, tackifiers, binders, etc. that are ordinarily used in pesticide formulation technology; these are well known to those skilled in pesticide formulation. The formulations may be mixed with one or more solid or liquid adjuvants and prepared by various means, e.g., by homogeneously mixing, blending and/or grinding the pesticidal composition with suitable adjuvants using conventional formulation techniques. Suitable formulations and application methods are described in U.S. Patent No. 6,468,523.

"Pest" includes but is not limited to, insects, fungi, bacteria, nematodes, mites, ticks, and the like. Insect pests include insects selected from the orders *Coleoptera, Diptera, Hymenoptera, Lepidoptera, Mallophaga, Homoptera, Hemiptera, Orthroptera, Thysanoptera, Dermaptera, Isoptera, Anoplura, Siphonaptera, Trichoptera*, etc., particularly *Coleoptera*, *Lepidoptera*, and *Diptera.*

Insect pests of the invention for the major crops include: Maize: *Ostrinia nubilalis,* European corn borer; *Agrotis ipsilon,* black cutworm; *Helicoverpa zea,* corn earworm; *Spodoptera frugiperda,* fall armyworm; *Diatraea grandiosella,* southwestern corn borer; *Elasmopalpus lignosellus,* lesser cornstalk borer; *Diatraea saccharalis,* surgarcane borer; *Diabrotica virgifera,* western corn rootworm; *Diabrotica longicornis barberi,* northern corn rootworm; *Diabrotica undecimpunctata howardi,* southern corn rootworm; *Melanotus* spp., wireworms; *Cyclocephala borealis,* northern masked chafer (white grub); *Cyclocephala immaculata,* southern masked chafer (white grub); *Popillia japonica,* Japanese beetle; *Chaetocnema pulicaria,* corn flea beetle; *Sphenophorus maidis*, maize billbug; *Rhopalosiphum maidis*, corn leaf aphid; *Anuraphis maidiradicis,* corn root aphid; *Blissus leucopterus leucopterus,* chinch bug; *Melanoplus femurrubrum,* redlegged grasshopper; *Melanoplus sanguinipes,* migratory grasshopper; *Hylemya platura*, seedcom maggot; *Agromyza parvicornis,* corn blot leafminer; *Anaphothrips obscrurus,* grass thrips; *Solenopsis milesta,* thief ant; *Tetranychus urticae,* twospotted spider mite; Sorghum: *Chilo partellus,* sorghum borer; *Spodoptera frugiperda,* fall armyworm; *Helicoverpa* zea, corn earworm; *Elasmopalpus lignosellus,* lesser cornstalk borer_{;} *Feltia subterranea*, granulate cutworm; *Phyllophaga crinita*, white grub; *Eleodes, Conoderus,* and *Aeolus* spp., wireworms; *Oulema melanopus,* cereal leaf beetle; *Chaetocnema pulicaria,* corn flea beetle; *Sphenoplrorus maidis,* maize billbug; *Rhopalosiphum maidis;* corn leaf aphid; *Sipha flava,* yellow sugarcane aphid; *Blissus leucopterus leucopterus,* chinch bug; *Contarinia sorghicola,* sorghum midge; *Tetranychus cinnabarinus,* carmine spider mite; *Tetranychus urticae,* twospotted spider mite; Wheat: *Pseudaletia unipunctata,* army worm; *Spodoptera frugiperda,* fall armyworm; *Elasmopalpus lignosellus,* lesser cornstalk borer; *Agrotis orthogollia,* western cutworm; *Elasmopalpus lignosellus,* lesser cornstalk borer; *Oulema melanopus,* cereal leaf beetle; *Hypera punctata,* clover leaf weevil; *Diabrotica undecimpunctata howardi,* southern corn rootworm; Russian wheat aphid; *Schizaphis graminum,* greenbug; *Macrosiphum avenae,* English grain aphid; *Melanoplus femurrubrum,* redlegged grasshopper; *Melanoplus differentialis,* differential grasshopper; *Melanoplus sanguinipes,* migratory grasshopper; *Mayetiola destructor,* Hessian fly; *Sitodiplosis mosellana,* wheat midge; *Meromyza americana,* wheat stem maggot; *Hylemya coarctata,* wheat bulb fly; *Frankliniella fusca,* tobacco thrips; *Cephus cinctus,* wheat stem sawfly; *Aceria tulipae,* wheat curl mite; Sunflower: *Suleima helianthana,* sunflower bud moth; *Homoeosoma electellum,* sunflower moth; *zygogramma exclamationis,* sunflower beetle; *Bothyrus gibbosus,* carrot beetle; *Neolasioptera murtfeldtiana,* sunflower seed midge; Cotton: *Heliothis virescens,* cotton budworm; *Helicoverpa zea,* cotton bollworm; *Spodoptera exigua,* beet armyworm; *Pectinophora gossypiella,* pink bollworm; *Anthonomus grandis,* boll weevil; *Aphis gossypii,* cotton aphid; *Pseudatomoscelis seriatus,* cotton fleahopper; *Trialeurodes abutilonea,* bandedwinged whitefly; *Lygus lineolaris,* tarnished plant bug; *Melanoplus femurrubrum,* redlegged grasshopper; *Melanoplus differerztialis,* differential grasshopper; *Thrips tabaci_{;}* onion thrips; *Franklinkiella fusca,* tobacco thrips; *Tetranychus cinnabarinus,* carmine spider mite; *Tetranychus urticae,* twospotted spider mite; Rice: *Diatraea saccharalis,* sugarcane borer; *Spodoptera frugiperda,* fall armyworm; *Helicoverpa zea,* corn earworm; *Colaspis brunnea,* grape colaspis; *Lissorhoptrus oryzophilus,* rice water weevil; *Sitophilus oryzae,* rice weevil; *Nephotettix nigropictus,* rice leafhopper; *Blissus leucopterus leucopterus,* chinch bug; *Acrosternum hilare,* green stink bug; Soybean: *Pseudoplusia includens,* soybean looper; *Anticarsia gemmatalis,* velvetbean caterpillar; *Plathypena scabra,* green cloverworm; *Ostrinia nubilalis,* European corn borer; *Agrotis ipsilon,* black cutworm; *Spodoptera exigua,* beet armyworm; *Heliothis virescens,* cotton budworm; *Helicoverpa zea,* cotton bollworm; *Epilachna varivestis,* Mexican bean beetle; *Myzus persicae,* green peach aphid; *Empoasca fabae,* potato leafhopper; *Acrosternum hilare,* green stink bug; *Melanoplus femurrubrum,* redlegged grasshopper; *Melanoplus differentialis,* differential grasshopper; *Hylemya platura,* seedcorn maggot; *Sericothrips variabilis,* soybean thrips; *Thrips tabaci,* onion thrips; *Tetranychus turkestani,* strawberry spider mite; *Tetranychus urticae,* twospotted spider mite; Barley: *Ostrinia nubilalis,* European corn borer; *Agrotis ipsilon,* black cutworm; *Schizaphis graminum,* greenbug; *Blissus leucopterus leucopterus,* chinch bug; *Acrosternum hilare,* green stink bug; *Euschistus servus,* brown stink bug; *Delia platura,* seedcorn maggot; *Mayetiola destructor,* Hessian fly; *Petrobia latens,* brown wheat mite; Oil Seed Rape: *Brevicoryne brassicae,* cabbage aphid; *Phyllotreta cruciferae,* Flea beetle; *Mamestra configurata,* Bertha armyworm; *Plutella xylostella,* Diamond-back moth; *Delia* ssp., Root maggots.

Nematodes include *Caenorhabitis elegans* and parasitic nematodes such as root-knot, cyst, and lesion nematodes, including *Heterodera* spp., *Meloidogyne* spp., and *Globodera* spp.; particularly members of the cyst nematodes, including, but not limited to, *Heterodera glycines* (soybean cyst nematode); *Heterodera schachtii* (beet cyst nematode); *Heterodera avenae* (cereal cyst nematode); and *Globodera rostochiensis* and *Globodera pailida* (potato cyst nematodes). Lesion nematodes include *Pratylenchus* spp.

The following examples are offered by way of illustration and not by way of limitation.

### EXPERIMENTAL

### Example 1. Extraction of Plasmid DNA

A pure culture of strain ATX14875 was grown in large quantities of rich media. The culture was spun to harvest the cell pellet. The cell pellet was then prepared by treatment with SDS by methods known in the art, resulting in breakage of the cell wall and release of DNA. Proteins and large genomic DNA were then precipitated by a high salt concentration. The plasmid DNA was then precipitated by standard ethanol precipitation. The plasmid DNA was separated from any remaining chromosomal DNA by high-speed centrifugation through a cesium chloride gradient. The DNA was visualized in the gradient by UV light and the band of lower density (i.e. the lower band) was extracted using a syringe. This band contained the plasmid DNA from Strain ATX14875. The quality of the DNA was checked by visualization on an agarose gel.

### Example 2. Cloning of Genes

The purified plasmid DNA was sheared into 5-10 kb sized fragments and the 5' and 3' single stranded overhangs repaired using T4 DNA polymerase and Klenow fragment in the presence of all four dNTPs. Phosphates were then attached to the 5' ends by treatment with T4 polynucleotide kinase. The repaired DNA fragments were then ligated overnight into a standard high copy vector (i.e. pBluescript SK+), suitably prepared to accept the inserts as known in the art (for example by digestion with a restriction enzyme producing blunt ends).

The quality of the library was analyzed by digesting a subset of clones with a restriction enzyme known to have a cleavage site flanking the cloning site. A high percentage of clones were determined to contain inserts, with an average insert size of 5-6 kb.

### Example 3. High Throughput Sequencing of Library Plates

Once the shotgun library quality was checked and confirmed, colonies were grown in a rich broth in 2ml 96-well blocks overnight at 37°C at a shaking speed of 350 rpm. The blocks were spun to harvest the cells to the bottom of the block. The blocks were then prepared by standard alkaline lysis prep in a high throughput format.

The end sequences of clones from this library were then determined for a large number of clones from each block in the following way: The DNA sequence of each clone chosen for analysis was determined using the fluorescent dye terminator sequencing technique (Applied Biosystems, Foster City, CA) and standard primers flanking each side of the cloning site. Once the reactions had been carried out in the thermocycler, the DNA was precipitated using standard ethanol precipitation. The DNA was resuspended in water and loaded onto a capillary sequencing machine. Each library plate of DNA was sequenced from either end of the cloning site, yielding two reads per plate over each insert.

### Example 4. Assembly and Screening of Sequencing Data

DNA sequences obtained were compiled into an assembly project and aligned together to form contigs. This can be done efficiently using a computer program, such as Vector NTi, or alternatively by using the Pred/Phrap suite of DNA alignment and analysis programs. These contigs, along with any individual read that may not have been added to a contig, were compared to a compiled database of all classes of known pesticidal genes. Contigs or individual reads identified as having identity to a known endotoxin or pesticidal gene were analyzed further. Among the sequences obtained, clones pAX018, pAX020, and pAx021 contained DNA identified as having homology to known endotoxin genes. Therefore, these clones were selected for further sequencing.

### Example 5. Sequencing of pAX018, pAX020 and pAX021

Primers were designed to anneal to the clones of interest (pAX018, pAX020 and pAX021), in a manner such that DNA sequences generated from such primers will overlap existing DNA sequence of the clone(s). This process, known as "oligo walking", is well known in the art. This process was utilized to determine the entire DNA sequence of the region exhibiting homology to a known endotoxin gene. In the case of pAX021, this process was used to determine the DNA sequence of the entire clone, resulting in a single nucleotide sequence. The completed DNA sequence was then placed back into the original large assembly for further validation. This allowed incorporation of more DNA sequence reads into the contig, resulting in multiple reads of coverage over the entire region.

Analysis of the DNA sequence of each clone by methods known in the art identified an open reading frame with homology to known delta endotoxin genes. The open reading frames found in pAX018, pAX020 and pAX021 were designated as AXMI-018, AXMI-020 and AXMI-021, respectively. The DNA sequence of AXMI-018 is provided as SEQ ID NO:1, and the amino acid sequence of the predicted protein is designated SEQ ID NO:2. The DNA sequence of AXMI-020 is provided as SEQ ID NO:3 and its predicted protein sequence is provided in SEQ ID NO: 4. The DNA sequence of AXMI-021 is provided as SEQ ID NO:5, and the amino acid sequence of the predicted protein is provided in SEQ ID NO:6.

### Example 6. Homology Between AXMI-018, AXMI-020 and AXMI-021

The novel ORFs found in strain ATX14875 showed high homology to each other, with most changes observed near the toxic portion of the genes. AXMI-018 and AXMI-020 are full-length endotoxin genes, and contain a C-terminal non-toxic domain. AXMI-021 appears to be a naturally truncated endotoxin belonging to the same family. Figure 1 shows an alignment of the proteins, truncated to their predicted toxic portion. Table 1 shows the percent identity between the novel endotoxins at the amino acid level.

**Table 1. Amino acid identity between AXMI-018, AXMI-020 and AXMI-021**

| | AXMI-018 | AXMI-020 | AXMI-021 |
|---|---|---|---|
| AXMI-018 | - | 91% | 97% |
| AXMI-020 | 91% | - | 91% |
| AXMI-021 | 91% | 91% | - |

### Example 7. Homology of Novel Genes to Known Endotoxin Genes

Searches of DNA and protein databases with the DNA sequence and amino acid sequence of AXMI-018, AXMI-020, and AXMI-021 reveal that they are homologous to a set of known endotoxins.

Figures 2A and 2B show an alignment of AXMI-018 with several endotoxins. Blast searches identify cry12Aa1 (Accession No. L07027) as having the strongest block of homology. However, alignment of the entire AMXI-018 protein (SEQ ID NO:2) to a large set of endotoxin proteins shows that AXMI-018 is most homologous to cry21Ba1 (Accession No. AB088406), and shares 25% amino acid identity with this toxin (see Table 2). The second column of Table 2 shows the amino acid identities of the untrimmed, full-length proteins. The third column of Table 2 reflects the homology of AXMI-018 within the toxin domains. The endotoxin with the highest homology through the N-terminal active portion of the gene is cry5Ab1 (Accession No. L07026). The amino acid identity of the truncated cry5Ab1 to the truncated AXMI-018 is 18% (see Table 2).

**Table 2. Amino Acid Identity of AXMI-018 with Exemplary Endotoxin Classes**

| Endotoxin | Percent Amino Acid Identity to AXMI-018 | Percent Amino Acid Identity of truncated Toxins to AXMI-018 |
|---|---|---|
| *cry12Aa* | 22.2% | 16% |
| *cry21Aa* | 23.7% | 17% |
| *cry21Ba1* | 25% | 17% |
| *cry5Aa* | 21.4% | 17% |
| *cry5Ab* | 23% | 18% |
| *cry5Ba* | 20.8% | 17% |
| *cry1Ac* | 17.5% | 14% |
| *cry1Ba* | 19% | 14% |
| *cry1Ca* | 18.6% | 16% |

Figures 3A and 3B show an alignment of AXMI-020 with several endotoxins. Blast searches identify cry12Aa1 (Accession No. L07027) as having the strongest block of homology. However, aligning the AMXI-020 protein (SEQ ID NO:4) to a large set of endotoxin proteins shows that the most homologous protein throughout the full length gene is actually cry21Ba1 (Accession No. AB088406), at 25% amino acid identity (see Table 3). The second column of Table 3 shows the amino acid identities of the untrimmed, full-length proteins. The third column reflects the true identity of the active portion of the protein by aligning only the toxic domains. The endotoxin with highest homology through the N-terminal active portion of the gene is cry5Ab1 (Accession No. L07026). The amino acid identity of the truncated cry5Ab1 to the truncated AXMI-020 is 18% (see Table 3).

**Table 3. Amino Acid Identity of AXMI-020 with Exemplary Endotoxin Classes**

| Endotoxin | Percent Amino Acid Identity to AXMI-020 | Percent Amino Acid Identity of truncated Toxins to AXMI-020 |
|---|---|---|
| *cry12Aa* | 24.1% | 15% |
| *cry21Aa* | 24.2% | 17% |
| *cry21Ba1* | 25% | 17% |
| *cry5Aa* | 21.9% | 17% |
| *cry5Ab* | 23.1% | 18% |
| *cry5Ba* | 22.6% | 17% |
| *cry1Ac* | 18.4% | 14% |
| *cry1Ba* | 19.7% | 14% |
| *cry1Ca* | 18.8% | 16% |

Figures 4A and 4B show an alignment of AXMI-021 with several endotoxins. Alignment of AMXI-021 protein (SEQ ID NO:6) to a large set of endotoxin proteins shows that the most homologous protein is cry5Ab1 (Accession No. L07026). The overall amino acid identity of the artificially truncated cry5Ab1 to AXMI-021 is 17% (see Table 4). Inspection of the amino acid sequence of AXMI-021 suggests that it does not contain a C-terminal non-toxic domain as is present in several endotoxin families. By removing this C-terminal protein of the toxins from the alignment, the alignment reflects the amino acid identity present solely in the toxin domains (see Table 4, column three). This "trimmed" alignment is shown in Figure 4A and 4B.

**Table 4. Amino Acid Identity of AXMI-021 with Exemplary Endotoxin Classes**

| Endotoxin | Percent Amino Acid Identity to AXMI-021 | Percent Amino Acid Identity of truncated Toxins to AXMI-021 |
|---|---|---|
| *cry12Aa* | 10.4% | 15% |
| *cry21Aa* | 11.6% | 15% |
| *cry21Ba1* | 10.2% | 16% |
| *cry5Aa* | 9.1% | 16% |
| *cry5Ab* | 11% | 17% |
| *cry5Ba* | 10.9% | 14% |
| *cry1Ac* | 9.9% | 14% |
| *cry1Ba* | 10.2% | 14% |
| *cry1Ca* | 9.8% | 14% |

Searches of the pFAM database identify AXMI-018, AXMI-020, and AXMI-021 as having homology to the delta endotoxin, N-terminal domain family (PFAM Accession No. PF03945). An Endotoxin _N domain is found between amino acid residues 70 and 302 of each protein (SEQ ID NOS:2, 4, and 6). An Endotoxin_C domain is found between amino acid residues 507 and 646 of each protein (SEQ ID NOS:2, 4, and 6).

This family contains insecticidal toxins produced by *Bacillus* species of bacteria. The N terminus of the crystalized protein is cleaved after insect ingestion, resulting in an activated protein. The C terminal extension is cleaved in some protein members. This activated region of the delta endotoxin is composed of three structural domains. The N-terminal helical domain is involved in membrane insertion and pore formation. The second and third domains are involved in receptor binding.

### Example 8. Expression of AXMI-018 and AXMI-021 in Bacillus

The insecticidal genes AXMI-018 and AXMI-021 are amplified by PCR from pAX018 and pAX021, respectively. The PCR products are cloned into the *Bacillus* expression vector pAX916 by methods well known in the art. The *Bacillus* strain containing the vector with either AXMI-018, designated pAX920, or AXMI-021, designated pAX931, is grown in CYS media (10 g/l Bacto-casitone; 3 g/l yeast extract; 6 g/l KH2PO4; 14 g/l K2HPO4; 0.5 mM MgSO4; 0.05 mM MnCl2; 0.05 mM FeSO4), until sporulation is evident by microscopic examination. The resulting proteins are then tested for insecticidal activity in bioassays against important insect pests.

### CYS Media

To prepare CYS media: 10 g/l Bacto-casitone; 3 g/l yeast extract; 6 g/l KH₂PO₄; 14 g/l K₂HPO ₄; 0.5 mM MgSO₄; 0.05 mM MnCl₂; 0.05 mM FeSO₄. The CYS mix should be pH 7, if adjustment is necessary. NaOH or HCl are preferred. The media is then autoclaved and 100 ml of 10X filtered glucose is added after autoclaving. If the resultant solution is cloudy it can be stirred at room temperature to clear.

### Example 9. Assay for Pesticidal Activity

The ability of a pesticidal protein to act as a pesticide upon a pest is often assessed in a number of ways. One way well known in the art is to perform a feeding assay. In such a feeding assay, one exposes the pest to a sample containing either compounds to be tested (i.e, compositions of the present invention), or control samples (samples not containing the test compound). Often this is performed by placing the material to be tested, or a suitable dilution of such material, onto a material that the pest will ingest, such as an artificial diet. The material to be tested may be composed of a liquid, solid, or slurry. The material to be tested may be placed upon the surface and then allowed to dry. Alternatively, the material to be tested may be mixed with a molten artificial diet, then dispensed into the assay chamber. The assay chamber may be, for example, a cup, a dish, or a well of a microtiter plate.

Assays for sucking pests (for example aphids) may involve separating the test material from the insect by a partition, ideally a portion that can be pierced by the sucking mouth parts of the sucking insect, to allow ingestion of the test material. Often the test material is mixed with a feeding stimulant, such as sucrose, to promote ingestion of the test compound.

Other types of assays can include microinjection of the test material into the mouth, or gut of the pest, as well as development of transgenic plants, followed by test of the ability of the pest to feed upon the transgenic plant. Plant testing may involve isolation of the plant parts normally consumed, for example, small cages attached to a leaf, or isolation of entire plants in cages containing insects.

Other methods and approaches to assay pests are known in the art, and can be found, for example in Robertson, J. L. & H. K. Preisler (1992), Pesticide bioassays with arthropods, CRC, Boca Raton, FL. Alternatively, assays are commonly described in
the journals *Arthropod Management Tests* and *Journal of Economic Entomology* or by discussion with members of the Entomological Society of America (ESA).

### Example 10. C. elegans bioassay

The activity of a pesticidal protein(s) upon the nematode *Caenorhabitis elegans* (*C. elegans*) is a useful predictor of general nematicidal activity. *C*. *elegans* hermaphrodites are reared as known in the art, to generate populations of healthy animals for bioassay. General procedures for growth, harvesting, and genetic manipulation of *C. elegans* including growth media, etc., may be found in the art, for example, in Wood, ed. (1988) The Nematode Caenorhabditis elegans, Cold Spring Harbor Laboratory Press, Cold Spring Harbor, NY.

Sterile supernatants from organisms such as those expressing the polypeptides of the invention may be tested for activity upon *C. elegans.* Bioassays are performed in 96-well plates. For the test samples, five to ten nematodes are added to, for example, 80 µl of S medium (Woods, 1998, *supra)* and mixed with, for example, 20 µl of sterile supernatant, and 0.5 µl of concentrated HB101 (prepared as described in Woods, 1998, *supra)* and rifampicin (final concentration of 0.1 µg/µl). Assays are allowed to proceed at room temperature for 3 days, and the effects of the test compound on the C. *elegans* organisms are recorded.

### Example 11. Vectoring of AXMI-018, AXMI-020 and AXMI-021 for Plant Expression

The coding regions ofAXMI-018, AXMI-020, and AXMI-021 are operably connected with appropriate promoter and terminator sequences for expression in plants. Such sequences are well known in the art and may include the rice actin promoter or maize ubiquitin promoter for expression in monocots, the *Arabidopsis* UBQ3 promoter or CaMV 35S promoter for expression in dicots, and the nos or PinII terminators. Techniques for producing and confirming promoter - gene - terminator constructs also are well known in the art.

The plant expression cassettes described above are combined with an appropriate plant selectable marker to aid in the selection of transformed cells and tissues, and ligated into plant transformation vectors. These vectors may include binary vectors from *Agrobacterium*-mediated transformation or simple plasmid vectors for aerosol or biolistic transformation.

### Example 12. Transformation of Maize Cells with AXMI-018, AXMI-020 and AXMI-021

Maize ears are best collected 8-12 days after pollination. Embryos are isolated from the ears, and those embryos 0.8-1.5 mm in size are preferred for use in transformation. Embryos are plated scutellum side-up on a suitable incubation media, such as DN62A5S media (3.98 g/L N6 Salts; 1 mL/L (of 1000x Stock) N6 Vitamins; 800 mg/L L-Asparagine; 100 mg/L Myo-inositol; 1.4 g/L L-Proline; 100 mg/L Casamino acids; 50 g/L sucrose; 1 mL/L (of 1 mg/mL Stock) 2,4-D). However, media and salts other than DN62A5S are suitable and are known in the art. Embryos are incubated overnight at 25°C in the dark. However, it is not necessary *per se* to incubate the embryos overnight.

The resulting explants are transferred to mesh squares (30-40 per plate), transferred onto osmotic media for about 30-45 minutes, then transferred to a beaming plate (see, for example, PCT Publication No. WO/0138514 and U.S. Patent No. 5,240,842).

DNA constructs designed to express the pesticidal polypeptides of the invention in plant cells are accelerated into plant tissue using an aerosol beam accelerator, using conditions essentially as described in PCT Publication No. WO/0138514. After beaming, embryos are incubated for about 30 min on osmotic media, then placed onto incubation media overnight at 25°C in the dark. To avoid unduly damaging beamed explants, they are incubated for at least 24 hours prior to transfer to recovery media. Embryos are then spread onto recovery period media, for about 5 days at 25°C in the dark, then transferred to selection media. Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated by methods known in the art. The resulting shoots are allowed to root on rooting media, and the resulting plants are transferred to nursery pots and propagated as transgenic plants.

### Materials

### DN62A5S Media

| **Components** | **per liter** | **Source** |
|---|---|---|
| Chu's N6 Basal Salt Mixture (Prod. No. C 416) | 3.98 g/L | Phytotechnology Labs |
| Chu's N6 Vitamin Solution (Prod. No. C 149) | 1 mL/L (of 1000x Stock) | Phytotechnology Labs |
| L-Asparagine | 800 mg/L | Phytotechnology Labs |
| Myo-inositol | 100 mg/L | Sigma |
| L-Proline | 1.4 g/L | Phytotechnology Labs |
| Casamino acids | 100 mg/L | Fisher Scientific |
| Sucrose | 50 g/L | Phytotechnology Labs |
| 2,4-D (Prod. No. D-7299) | 1 mL/L (of 1 mg/mL Stock) | Sigma |

The pH of the solution is adjusted to pH 5.8 with 1N KOH/1N KCI, Gelrite (Sigma) up to 3g/L is added, and the mixture is autoclaved. After cooling to 50°C, 2 ml/L of a 5 mg/ml stock solution of Silver Nitrate (Phytotechnology Labs) is added. The recipe yields about 20 plates.

### Example 13. Transformation of AXMI-018, AXMI-020 and AXMI-021 into Plant Cells by Agobacterium-Mediated Transformation

Ears are best collected 8-12 days after pollination. Embryos are isolated from the ears, and those embryos 0.8-1.5 mm in size are preferred for use in transformation. Embryos are plated scutellum side-up on a suitable incubation medium, and incubated overnight at 25°C in the dark. However, it is not necessary *per se* to incubate the embryos overnight. Embryos are contacted with an *Agrobacterium* strain containing the appropriate vectors for Ti plasmid mediated transfer for about 5-10 min, and then plated onto co-cultivation media for about 3 days (25°C in the dark). After co-cultivation, explants are transferred to recovery period media for about five days (at 25°C in the dark). Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated as known in the art. The resulting shoots are allowed to root on rooting media, and the resulting plants are transferred to nursery pots and propagated as transgenic plants.

All publications and patent applications mentioned in the specification are indicative of the level of skill of those skilled in the art to which this invention pertains.

## Claims

1. A nucleic acid molecule selected from the group consisting of:
a) a nucleic acid molecule comprising the nucleotide sequence of SEQ ID NO: 1, 3, or 5, or a complement thereof;
b) a nucleic acid molecule comprising a nucleotide sequence having at least 80% sequence identity to the nucleotide sequence of SEQ ID NO: 1, 3, or 5, or a complement thereof, wherein said nucleotide sequence encodes a polypeptide having pesticidal activity;
c) a nucleic acid molecule which encodes a polypeptide comprising the amino acid sequence of SEQ ID NO: 2, 4, or 6;
d) a nucleic acid molecule which encodes a polypeptide comprising an amino acid sequence having at least 80% sequence identity to the amino acid sequence of SEQ ID NO: 2, 4, or 6, wherein said nucleotide sequence encodes a polypeptide having pesticidal activity; and,
e) the delta endotoxin nucleotide sequence of the DNA insert of the plasmid deposited as Accession No. NRRL B-30805, NRRL B-30809, NRRL B-30808, or a complement thereof;
optionally wherein said nucleotide sequence is a synthetic sequence that has been designed for expression in a plant.

2. The nucleic acid molecule of claim 1, wherein said nucleotide sequence is operably linked to a promoter capable of directing expression of the nucleotide sequence in a plant cell.

3. A vector comprising the nucleic acid molecule of claim 1 or 2, optionally further comprising a nucleic acid molecule encoding a heterologous polypeptide.

4. A plant or a bacterial host cell comprising the nucleic acid molecule of claim 2 or the vector of claim 3.

5. An isolated polypeptide with pesticidal activity, selected from the group consisting of:
a) a polypeptide comprising the amino acid sequence of SEQ ID NO: 2, 4, or 6;
b) a polypeptide comprising an amino acid sequence having at least 80% sequence identity to the amino acid sequence of SEQ ID NO: 2, 4, or 6, wherein said polypeptide has pesticidal activity;
c) a polypeptide that is encoded by the nucleotide sequence of SEQ ID NO: 1, 3, or 5;
d) a polypeptide that is encoded by a nucleotide sequence that is at least 80% identical to the nucleotide sequence of SEQ ID NO: 1, 3, or 5, wherein said polypeptide has pesticidal activity; and,
e) a polypeptide encoded by the delta endotoxin nucleotide sequence of the DNA insert of the plasmid deposited as Accession No. NRRL B-30805, NRRL B-30809, or NRRL B-30808.

6. The polypeptide of claim 5 further comprising heterologous amino acid sequences.

7. A composition comprising the polypeptide of claim 5, optionally wherein said composition comprises a powder, dust, pellet, granule, spray, emulsion, colloid, and solution and wherein said composition is prepared by desiccation, lyophilization, homogenization, extraction, filtration, centrifugation, sedimentation, or concentration of a culture of the bacterial host cell of claim 4.

8. The composition of claim 7, comprising from about 1% to about 99% by weight of said polypeptide.

9. A method for killing or controlling a lepidopteran, coleopteran, or nematode pest population comprising contacting said population with, or feeding to said pest, a pesticidally-effective amount of a polypeptide of claim 5.

10. A method for producing a polypeptide with pesticidal activity, comprising culturing the bacterial host cell of claim 4 under conditions in which a nucleic acid molecule encoding the polypeptide is expressed, said polypeptide being selected from the group consisting of:
a) a polypeptide comprising the amino acid sequence of SEQ ID NO: 2, 4, or 6;
b) a polypeptide encoded by the nucleic acid sequence of SEQ ID NO: 1, 3, or 5;
c) a polypeptide comprising an amino acid sequence having at least 80% sequence identity to a polypeptide with the amino acid sequence of SEQ ID NO: 2, 4, or 6, wherein said polypeptide has pesticidal activity;
d) a polypeptide encoded by a nucleic acid molecule comprising a nucleotide sequence having at least 80% sequence identity to the nucleic acid sequence of SEQ ID NO: 1, 3, or 5, wherein said polypeptide has pesticidal activity; and,
e) a polypeptide encoded by the delta endotoxin nucleotide sequence of the DNA insert of the plasmid deposited as Accession No. NRRL B-30805, NRRL B-30809, or NRRL B-30808.

11. A plant having stably incorporated into its genome a DNA construct comprising a nucleotide sequence that encodes a protein having pesticidal activity, wherein said nucleotide sequence is selected from the group consisting of:
a) the nucleotide sequence of SEQ ID NO: 1, 3, or 5;
b) a nucleotide sequence having at least 80% sequence identity to the nucleotide sequence of SEQ ID NO: 1, 3, or 5, wherein said nucleotide sequence encodes a polypeptide having pesticidal activity;
c) a nucleotide sequence encoding a polypeptide comprising the amino acid sequence of SEQ ID NO: 2, 4, or 6;
d) a nucleotide sequence encoding a polypeptide having at least 80% amino acid sequence identity to the amino acid sequence of SEQ ID NO: 2, 4, or 6, wherein said nucleotide sequence encodes a polypeptide having pesticidal activity; and,
e) the delta endotoxin nucleotide sequence of the DNA insert of the plasmid deposited as Accession No. NRRL B-30805, NRRL B-30809, or NRRL B-30808, or a complement thereof;
wherein said nucleotide sequence is operably linked to a promoter that drives expression of a coding sequence in a plant cell.

12. The plant of claim 11, wherein said plant is selected from the group consisting of maize, sorghum, wheat, cabbage, sunflower, tomato, crucifers, peppers, potato, cotton, rice, soybean, sugarbeet, sugarcane, tobacco, barley, and oilseed rape.

13. A Transformed seed of the plant of claim 12, wherein said seed comprises incorporated in the genome a construct as defined in claim 11.

14. A method for protecting a plant from a pest, comprising introducing into said plant or cell thereof at least one expression vector comprising a nucleotide sequence that encodes a pesticidal polypeptide, wherein said nucleotide sequence is selected from the group consisting of:
a) a nucleic acid molecule comprising the nucleotide sequence of SEQ ID NO: 1, 3, or 5;
b) a nucleic acid molecule comprising a nucleotide sequence having at least 80% sequence identity to the nucleotide sequence of SEQ ID NO: 1, 3, or 5, wherein said nucleotide sequence encodes a polypeptide having pesticidal activity;
c) a nucleic acid molecule that encodes a polypeptide comprising the amino acid sequence of SEQ ID NO: 2, 4, or 6;
d) a nucleic acid molecule that encodes a polypeptide having at least 80% sequence identity to the amino acid sequence of SEQ ID NO: 2, 4, or 6, wherein said nucleotide sequence encodes a polypeptide having pesticidal activity; and,
e) the delta endotoxin nucleotide sequence of the DNA insert of the plasmid deposited as Accession No. NRRL B-30805, NRRL B-30809, NRRL B-30808, or a complement thereof.

15. The method of claim 14, wherein said plant produces a pesticidal polypeptide having pesticidal activity against a lepidopteran, coleopteran, or nematode pest.

## Patentansprüche

1. Nukleinsäuremolekül, ausgewählt aus der Gruppe, bestehend aus:
a) einem Nukleinsäuremolekül, das die Nukleotidsequenz von SEQ ID NO:1, 3, oder 5 oder ein Komplement davon umfasst;
b) einem Nukleinsäuremolekül, umfassend eine Nukleotidsequenz mit wenigstens 80% Sequenzidentität zu der Nukleotidsequenz von SEQ ID NO:1, 3 oder 5 oder einem Komplement davon, wobei die Nukleotidsequenz ein Polypeptid mit Pestizidaktivität codiert;
c) einem Nukleinsäuremolekül, das ein Polypeptid codiert, das die Aminosäuresequenz von SEQ ID NO:2,4 oder 6 umfasst;
d) einem Nukleinsäuremolekül, das ein Polypeptid codiert, das eine Aminosäuresequenz umfasst, die wenigstens 80% Sequenzidentität zu der Aminosäuresequenz von SEQ ID NO:2, 4 oder 6 hat, wobei die Nukleotidsequenz ein Polypeptid mit Pestizidaktivität codiert, und
e) der Delta-Enotoxin-Nukleotidsequenz des DNA-Inserts des Plasmids, hinterlegt als Zugangsnummer NRRL B-30805, NRRL B-30809, NRRL B-30808, oder einem Komplement davon;
wobei die Nukleotidsequenz gegebenenfalls eine synthetische Sequenz ist, die zur Expression in einer Pflanze entwickelt wurde.

2. Nukleinsäuremolekül gemäß Anspruch 1, wobei die Nukleotidsequenz funktionsfähig mit einem Promotor verknüpft ist, der fähig ist, eine Expression der Nukleotidsequenz in einer Pflanzenzelle zu steuern.

3. Vektor, umfassend das Nukleinsäuremolekül gemäß Anspruch 1 oder 2, gegebenenfalls außerdem umfassend ein Nukleinsäuremolekül, das ein heterologes Polypeptid codiert.

4. Pflanze oder bakterielle Wirtszelle, die das Nukleinsäuremolekül gemäß Anspruch 2 oder den Vektor gemäß Anspruch 3 umfasst.

5. Isoliertes Polypeptid mit Pestizidaktivität, ausgewählt aus der Gruppe, bestehend aus:
a) einem Polypeptid, umfassend die Aminosäuresequenz von SEQ ID NO:2, 4 oder 6;
b) einem Polypeptid, umfassend eine Aminosäuresequenz, die wenigstens 80% Sequenzidentität zu der Aminosäuresequenz von SEQ ID NO: 2, 4 oder 6 hat, wobei das Polypeptid Pestizidaktivität hat;
c) einem Polypeptid, das durch die Nukleotidsequenz von SEQ ID NO:1, 3, oder 5 codiert wird;
d) einem Polypeptid, das durch eine Nukleotidsequenz codiert wird, die wenigstens 80% identisch zu der Nukleotidsequenz von SEQ ID NO:1, 3 oder 5 ist, wobei das Polypeptid Pestizidaktivität hat, und
e) einem Polypeptid, das durch die Delta-Endotoxin-Nukleotidsequenz des DNA-Inserts des Plasmids, hinterlegt als Zugangsnummer NRRL B-30805, NRRL B-30809 oder NRRL B-30808, codiert wird.

6. Polypeptid gemäß Anspruch 5, das außerdem heterologe Aminosäuresequenzen umfasst.

7. Zusammensetzung, umfassend das Polypeptid gemäß Anspruch 5, wobei gegebenenfalls die Zusammensetzung ein Pulver, ein Stäubemittel, ein Pellet, ein Granulat, ein Spray, eine Emulsion, ein Colloid und eine Lösung umfasst und wobei die Zusammensetzung durch Trocknung, Lyophilisierung, Homogenisierung, Extraktion, Filtration, Zentrifugation, Sedimentation oder Konzentrierung einer Kultur der bakteriellen Wirtszelle gemäß Anspruch 4 hergestellt wurde.

8. Zusammensetzung gemäß Anspruch 7, die etwa 1 Gewichts-% bis etwa 99 Gewichts-% des Polypeptids umfasst.

9. Verfahren zum Abtöten oder Bekämpfen einer Lepidopteren-, Coleopteren- oder Nematoden-Schädlingspopulation, das Inkontaktbringen der Population mit einer pestizidwirksamen Menge eines Polypeptids gemäß Anspruch 5 oder Füttern einer pestizidwirksamen Menge eines Polypeptids gemäß Anspruch 5 an den Schädling umfasst.

10. Verfahren zu Herstellung eines Polypeptids mit Pestizidaktivität, umfassend Kultivieren der Bakterienwirtszelle gemäß Anspruch 4 unter Bedingungen, unter denen ein Nukleinsäuremolekül, das das Polypeptid codiert, exprimiert wird, wobei das Polypeptid ausgewählt ist aus der Gruppe, bestehend aus:
a) einem Polypeptid, das die Aminosäuresequenz von SEQ ID NO:2, 4 oder 6 umfasst;
b) einem Polypeptid, das durch die Nukleinsäuresequenz von SEQ ID NO:1, 3 oder 5 codiert wird;
c) einem Polypeptid, das eine Aminosäuresequenz umfasst, die wenigstens 80% Sequenzidentität zu einem Polypeptid mit der Aminosäuresequenz von SEQ ID NO:2, 4 oder 6 hat, wobei das Polypeptid Pestizidaktivität hat;
d) einem Polypeptid, das durch ein Nukleinsäuremolekül codiert wird, das eine Nukleotidsequenz umfasst, die wenigstens 80% Sequenzidentität zu der Nukleinsäuresequenz von SEQ ID NO:1, 3 oder 5 hat, wobei das Polypeptid Pestizidaktivität hat, und
e) einem Polypeptid, das durch die Delta-Endotoxin-Nukleotidsequnz des DNA-Inserts des Plasmids, hinterlegt als Zugangsnummer NRRL B-30805, NRRL B-30809 oder NRRL B-30808, codiert wird.

11. Pflanze, die stabil in ihr Genom ein DNA-Konstrukt eingebaut hat, das eine Nukleotidsequenz umfasst, die ein Protein mit Pestizidaktivität codiert, wobei die Nukleotidsequenz ausgewählt ist aus der Gruppe, bestehend aus:
a) der Nukleotidsequenz von SEQ ID NO:1, 3 oder 5;
b) einer Nukleotidsequenz, die wenigstens 80% Sequenzidentität zu der Nukleotidsequenz von SEQ ID NO:1, 3 oder 5 hat, wobei die Nukleotidsequenz ein Polypeptid codiert, das Pestizidaktivität hat;
c) einer Nukleotidsequenz, die ein Polypeptid codiert, das die Aminosäuresequenz von SEQ ID NO:2, 4 oder 6 umfasst;
d) einer Nukleotidsequenz, die ein Polypeptid codiert, das wenigstens 80% Aminosäuresequenzidentität zu der Aminosäuresequenz von SEQ ID NO:2,4 oder 6 hat, wobei die Nukleotidsequenz ein Polypeptid codiert, das Pestizidaktivität hat, und
e) der Delta-Endotoxin-Nukleotidsequenz des DNA-Inserts des Plasmids, hinterlegt als Zugangsnummer NRRL B-30805, NRRL B-30809 oder NRRL B-30808, oder einem Komplement davon;
wobei die Nukleotidsequenz funktionsfähig mit einem Promotor verknüpft ist, der eine Expression einer codierenden Sequenz in einer Pflanzenzelle steuert.

12. Pflanze gemäß Anspruch 11, wobei die Pflanze aus der Gruppe, bestehend aus Mais, Hirse, Weizen, Kohl, Sonnenblume, Tomate, Cruciferen, Pfeffer bzw. Paprika, Baumwolle, Reis, Sojabohne, Zuckerrübe, Zuckerrohr, Tabak, Gerste und Ölsamenraps, ausgewählt ist.

13. Transformierter Samen der Pflanze gemäß Anspruch 12, wobei der Samen in das Genom ein Konstrukt, wie es in Anspruch 11 definiert ist, eingebaut umfasst.

14. Verfahren zum Schützen einer Pflanze vor einem Schädling, umfassend Einführen wenigstens eines Expressionsvektors, umfassend eine Nukleotidsequenz, die für ein pestizides Polypeptid codiert, in die Pflanze oder eine Zelle davon, wobei die Nukleotidsequenz ausgewählt ist aus der Gruppe, bestehend aus:
a) einem Nukleinsäuremolekül, das die Nukleotidsequenz von SEQ ID NO:1, 3 oder 5 umfasst;
b) einem Nukleinsäuremolekül, umfassend eine Nukleotidsequenz, die wenigstens 80% Sequenzidentität zu der Nukleotidsequenz von SEQ ID NO:1, 3 oder 5 hat, wobei die Nukleotidsequenz ein Polypeptid codiert, das Pestizidaktivität hat;
c) einem Nukleinsäuremolekül, das ein Polypeptid codiert, das die Aminosäuresequenz von SEQ ID NO:2, 4 oder 6 umfasst;
d) einem Nukleinsäuremolekül, das ein Polypeptid codiert, das wenigstens 80% Sequenzidentität zu der Aminosäuresequenz von SEQ ID NO:2, 4 oder 6 hat, wobei die Nukleotidsequenz ein Polypeptid codiert, das Pestizidaktivität hat, und
e) der Delta-Endotoxin-Nukleotidsequenz des DNA-Inserts des Plasmids, hinterlegt als Zugangsnummer NRRL B-30805, NRRL B-30809, NRRL B-30808 oder einem Komplement davon.

15. Verfahren gemäß Anspruch 14, wobei die Pflanze ein pestizides Polypeptid produziert, das Pestizidaktivität gegen einen Lepidopteren-, Coleopteren- oder Nematodenschädling hat.

## Revendications

1. Molécule d'acide nucléique choisie dans le groupe comprenant:
a) une molécule d'acide nucléique comprenant la séquence de nucléotides de Séquence n° 1, 3 ou 5, ou une séquence complémentaire de celle-ci;
b) une molécule d'acide nucléique comprenant une séquence de nucléotides présentant au moins 80% d'identité de séquence avec la séquence de nucléotides de Séquence n°1, 3 ou 5, ou une séquence complémentaire de celle-ci, ladite séquence de nucléotides codant un polypeptide possédant une activité antiparasitaire;
c) une molécule d'acide nucléique qui code un polypeptide comprenant la séquence d'acides aminés de Séquence n° 2, 4 ou 6;
d) une molécule d'acide nucléique qui code un polypeptide comprenant une séquence d'acides aminés présentant au moins 80% d'identité de séquence avec la séquence d'acides aminés de Séquence n° 2, 4 ou 6, ladite séquence de nucléotides codant un polypeptide possédant une activité antiparasitaire; et
e) la séquence de nucléotides de l'endotoxine delta de l'insert d'ADN du plasmide déposé sous le Numéro d'enregistrement NRRL B-30805, NRRL B-30809, NRRL B-30808 ou une séquence complémentaire de celle-ci;
ladite séquence de nucléotides étant facultativement une séquence synthétique qui a été conçue pour s'exprimer chez une plante.

2. Molécule d'acide nucléique de la revendication 1, ladite séquence de nucléotides étant liée opérationnellement à un promoteur capable de diriger l'expression de la séquence de nucléotides dans une cellule végétale.

3. Vecteur comprenant la molécule d'acide nucléique de la revendication 1 ou 2, comprenant facultativement en outre une molécule d'acide nucléique codant un polypeptide hétérologue.

4. Cellule hôte végétale ou bactérienne comprenant la molécule d'acide nucléique de la revendication 2 ou le vecteur de la revendication 3.

5. Polypeptide isolé présentant une activité antiparasitaire, choisi dans le groupe comprenant:
a) un polypeptide comprenant la séquence d'acides aminés de Séquence n° 2, 4 ou 6;
b) un polypeptide comprenant une séquence d'acides aminés présentant au moins 80% d'identité de séquence avec la séquence d'acides aminés de Séquence n° 2, 4 ou 6, ledit polypeptide possédant une activité antiparasitaire;
c) un polypeptide codé par la séquence de nucléotides de Séquence n° 1, 3 ou 5;
d) un polypeptide codé par la séquence de nucléotides qui est identique au moins à 80% à la séquence de nucléotides de Séquence n° 1, 3 ou 5, ledit polypeptide possédant une activité antiparasitaire; et
e) un polypeptide codé par la séquence de nucléotides de l'endotoxine delta de l'insert d'ADN du plasmide déposé sous le Numéro d'enregistrement NRRL B-30805, NRRL B-30809 ou NRRL B-30808.

6. Polypeptide de la revendication 5 comprenant en outre des séquences d'acides aminés hétérologues.

7. Composition comprenant le polypeptide de la revendication 5, ladite composition comprenant facultativement une poudre, poudre pour poudrage, des pastilles, des granules, un aérosol, une émulsion, un colloïde et une solution, et ladite composition étant préparée par dessiccation, lyophilisation, homogénéisation, extraction, filtration, centrifugation, sédimentation ou concentration d'une culture de la cellule hôte bactérienne de la revendication 4.

8. Composition de la revendication 7, comprenant d'environ 1% à environ 99% en poids dudit polypeptide.

9. Procédé d'éradication ou de contrôle d'une population de parasites lepidoptères, coleoptères ou nématodes, comprenant le fait de mettre en contact ladite population, ou de nourrir lesdits parasites, avec une quantité efficace du point de vue antiparasitaire d'un polypeptide de la revendication 5.

10. Procédé de production d'un polypeptide à activité antiparasitaire, comprenant le fait de cultiver la cellule hôte bactérienne de la revendication 4 en conditions dans lesquelles une molécule d'acide nucléique codant le polypeptide est exprimée, ledit polypeptide étant choisi dans le groupe comprenant:
a) un polypeptide comprenant la séquence d'acides aminés de Séquence n° 2, 4 ou 6;
b) un polypeptide codé par la séquence de nucléotides de Séquence n° 1, 3 ou 5;
c) un polypeptide comprenant une séquence d'acides aminés présentant au moins 80% d'identité de séquence avec un polypeptide portant la séquence d'acides aminés de Séquence n° 2, 4 ou 6, ledit polypeptide possédant une activité antiparasitaire;
d) un polypeptide codé par une molécule d'acide nucléique comprenant une séquence de nucléotides présentant au moins 80% d'identité de séquence avec la séquence d'acide nucléique de Séquence n° 1, 3 ou 5, ledit polypeptide possédant une activité antiparasitaire; et
e) un polypeptide codé par la séquence de nucléotides de l'endotoxine delta de l'insert d'ADN du plasmide déposé sous le Numéro d'enregistrement NRRL B-30805, NRRL B-30809 ou NRRL B-30808.

11. Plante présentant un assemblage d'ADN incorporé de manière stable dans son génome, comprenant une séquence de nucléotides qui code une protéine possédant une activité antiparasitaire, ladite séquence de nucléotides étant choisie dans le groupe comprenant:
a) la séquence de nucléotides de Séquence n° 1, 3 ou 5;
b) une séquence de nucléotides présentant au moins 80% d'identité de séquence avec la séquence de nucléotides de Séquence n°1, 3 ou 5, ladite séquence de nucléotides codant un polypeptide possédant une activité antiparasitaire;
c) une séquence de nucléotides qui code un polypeptide comprenant la séquence d'acides aminés de Séquence n° 2, 4 ou 6;
d) une séquence de nucléotides qui code un polypeptide présentant au moins 80% d'identité de séquence d'acides aminés avec la séquence d'acides aminés de Séquence n° 2, 4 ou 6, ladite séquence de nucléotides codant un polypeptide possédant une activité antiparasitaire; et
e) la séquence de nucléotides de l'endotoxine delta de l'insert d'ADN du plasmide déposé sous le Numéro d'enregistrement NRRL B-30805, NRRL B-30809 ou NRRL B-30808, ou une séquence complémentaire de celle-ci;
ladite séquence de nucléotides étant liée opérationnellement à un promoteur qui commande l'expression d'une séquence codante dans une cellule végétale.

12. Plante de la revendication 11, ladite plante étant choisie dans le groupe comprenant le maïs, le sorgho, le blé, le chou, le tournesol, la tomate, les crucifères, les poivres, la pomme de terre, le coton, le riz, le soja, la betterave à sucre, la canne à sucre, le tabac, l'orge et la navette oléagineuse.

13. Graine transformée de la plante de la revendication 12, ladite graine comprenant, incorporé dans son génome, un assemblage tel que défini en revendication 11.

14. Procédé de protection d'une plante contre un parasite, comprenant le fait d'introduire dans ladite plante, ou dans une cellule de ladite plante, au moins un vecteur d'expression comprenant une séquence de nucléotides qui code un polypeptide antiparasitaire, ladite séquence de nucléotides étant choisie dans le groupe comprenant:
a) une molécule d'acide nucléique comprenant la séquence de nucléotides de Séquence n° 1, 3 ou 5;
b) une molécule d'acide nucléique comprenant une séquence de nucléotides présentant au moins 80% d'identité de séquence avec une séquence de nucléotides de Séquence n° 1, 3 ou 5, ladite séquence de nucléotides codant un polypeptide possédant une activité antiparasitaire;
c) une molécule d'acide nucléique qui code un polypeptide comprenant la séquence d'acides aminés de Séquence n° 2, 4 ou 6;
d) une molécule d'acide nucléique qui code un polypeptide présentant au moins 80% d'identité de séquence avec la séquence d'acides aminés de Séquence n° 2, 4 ou 6, ladite séquence de nucléotides codant un polypeptide présentant une activité antiparasitaire; et
e) la séquence de nucléotides de l'endotoxine delta de l'insert d'ADN du plasmide déposé sous le Numéro d'enregistrement NRRL B-30805, NRRL B-30809, NRRL B-30808, ou une séquence complémentaire de celle-ci.

15. Procédé selon la revendication 14, ladite plante produisant un polypeptide antiparasitaire possédant une activité antiparasitaire contre un parasite lepidoptère, coleoptère ou nématode.
